# EUROPEAN PATENT APPLICATION

(11) **EP 3 970 741 A1**
(43) Date of publication of application: **23.03.2022**
(21) Application number: 21185987.1
(22) Date of filing: 17.05.2019
(51) Int. Cl.: A61K 39/275

(54) **STABILISED SUPEROXIDE DISMUTASE (SOD) HOMOLOG GENE AND USES THEREOF**

(30) Priority: 17.05.2018 GB 201808049
(62) Divisional of application: 19736488.8
(71) Applicant: University of Cape Town, Cape Town 7701 (ZA)
(72) Inventor: WILLIAMSON, Anna-Lise, Cape Town 7405 (ZA); MUNYANDUKI, Henry, Cape Town 7701 (ZA); DOUGLASS, Nicola Jennifer, Cape Town 7708 (ZA)
(74) Representative: HGF

(57) **Abstract**

The present invention relates to a stabilised SOD-homolog (SODis) gene of SEQ ID NO:2 and recombinant viruses comprising the gene. The invention also relates to a recombinant lumpy skin disease virus (LSDV) wherein a gene encoding a SOD-homolog polypeptide has been inactivated by deletion from the viral genome. The invention further relates to pharmaceutical compositions comprising the recombinant viruses and to the use of the recombinant viruses in methods of preventing a disease in a subject.

## Description

### BACKGROUND OF THE INVENTION

The present invention relates to a stabilised SOD-homolog (SODis) gene of SEQ ID NO:2 and recombinant viruses comprising the gene, having improved immunogenicity. The invention further relates to a recombinant lumpy skin disease virus (LSDV) having a deleted gene encoding a SOD-homolog polypeptide. The present invention also relates to pharmaceutical compositions comprising the recombinant viruses, as well as the use of said recombinant viruses in methods of preventing a disease in a subject.

Lumpy skin disease virus (LSDV) is a member of the *Capripoxvirus* genus of the *Poxviridae* family of viruses and is the causative agent of lumpy skin disease (LSD) in cattle. Lactating cattle have been found to be the most susceptible to disease. The rate of morbidity is often higher during an outbreak and can rise to 85%. LSD in cattle ranges from subclinical to an acute infection and usually lasts for 2-5 weeks. The acute infection is characterised by fever and localised or disseminated nodules developing on the skin, where lesions are often found in the upper respiratory tract and a secondary bacterial infection often occurs. Nodules have been reported to occur on the skeletal muscles and the mucosa of the oral and upper respiratory tract. Systemic effects include pyrexia, anorexia, dysgalactia and pneumonia. Cows may lose reproductive ability and milk producing capacity for several months. Fever, anorexia and abortions are also common. The drop in milk production in lactating cows along with temporary or permanent infertility in cows and bulls and skin lesions caused by the virus all impact on the economic effects of the disease. Eradication campaigns and imposed trade restriction on live animals and animal products add to the financial toll of an outbreak. In Africa economic losses due to LSD are comparable to that of foot and mouth disease. This has resulted in the World Organization for Animal Heath (OIE) recognising LSD as a notifiable agricultural disease.

LSDV is only able to naturally cause disease in cattle despite a high level of sequence identity shared with sheeppox virus (SPV) and goatpox virus (GPV). Some isolates of sheep and goatpox virus are able to infect both sheep and goats; however most isolates display host preference for either sheep or goats. These host range characteristics are reflected in the independent distribution of the different *Capripoxvirus* species. Wild game animals such as giraffes and impala have been shown to be permissive for wild-type LSDV where, during experimental infection, both animal species displayed pathology resembling that of LSDV infection in cattle.

There is thus a need for vaccines against Lumpy Skin Disease (LSD), which affects cattle in most African countries as well as the Middle East and Gulf Peninsula. It is spreading at an alarming rate and has recently entered Europe. Vaccines which are commercially available include a vaccine based on attenuated Neethling LSDV isolate, Herbivac^{®}LS (Herbivac) and MSD (Design Biologix product), all produced in South Africa. The Neethling LSDV isolate vaccine produced by Onderstepoort Biological Products is generally highly effective, but safety issues have been raised. The KS1-O180 strain is used in Ethiopia for immunization of small ruminants and cattle, but gives poor protection. The RM-65 attenuated sheep pox vaccine has been used at ten times the dose in cattle, but is less effective than vaccination with homologous strains.

In addition to the varying degrees of virulence of commercial vaccines there is also variability in susceptibility of different breeds of cattle to the LSDV vaccines. The European high-producing cattle breeds have been observed to be more susceptible to adverse vaccine reactions. It may therefore be desirable to have vaccines of differing potency for different breeds of cattle.

Herbivac has been found to be more immunogenic than Neethling, its parent strain. The DNA sequence of Herbivac was recently published and one significant difference was noted between Herbivac and Neethling, at ORF 134, a VACV B22R homolog. The inventors of the present application have also sequenced Herbivac and found a different single significant change from the published Neethling strain, at ORF 131, a gene potentially coding for a superoxide dismutase (SOD) homolog. *Leporipoxviruses* encode SOD homologs which act as decoy proteins. They are catalytically inert but bind the copper chaperone for SOD, thereby depleting the amount of copper available for use by the Cu, Zn SOD. This causes an intracellular increase in superoxide anion, which inhibits apoptosis.

In the present invention, a SOD-homolog ORF (SODis) was designed to resemble that of Herbivac, yet be more stable. The inventors have constructed recombinant LSDV viruses with and without the SOD-homolog gene. Assays to test the induction and inhibition of apoptosis indicated that the SOD-homolog causes inhibition of camptothecin induced apoptosis. The inventors have also shown that the presence or absence of the SOD-homolog influences immunogenicity of vaccine. In the present invention the SOD-homolog was modified to be more stable and less likely to mutate in the way that it has during its history of passaging, both in the generation of the Neethling vaccine as well as in the generation of Herbivac.

### SUMMARY OF THE INVENTION

The present invention relates to the SODis gene, constructs containing said gene or having a knocked-out SOD-homolog gene and vaccines comprising the gene or constructs for use in treating diseases.

According to a first aspect of the present invention there is provided for a stabilised SOD-homolog (SODis) gene of SEQ ID NO:2.

In a second aspect of the invention there is provided for a recombinant virus comprising the SODis gene of of SEQ ID NO:2.

According to a third aspect of the present invention there is provided for a recombinant lumpy skin disease virus (LSDV), wherein a gene encoding a SOD-homolog polypeptide has been inactivated by deletion from the viral genome. The SOD-homolog polypeptide may be selected from the group consisting of SEQ ID NO:1, SEQ ID NO:17, SEQ ID NO:18, and SEQ ID NO:19.

In a first embodiment of the invention, the recombinant virus or recombinant LSDV of the invention further comprises a marker gene selected from the group consisting of a guanine phosphoribosyltransferase gene, a green fluorescent protein gene, an enhanced green fluorescent protein gene and a red fluorescence protein gene. For example, the marker gene may be GPT, eGFP, or mCherry. It will be appreciated by a person of skill in the art that any selectable marker may be used in the recombinant virus or recombinant LSDV.

According to a fourth aspect of the present invention there is provided for a pharmaceutical composition, comprising the recombinant virus or recombinant LSDV of the invention and a pharmaceutically acceptable diluent or excipient.

In another embodiment of the invention, the pharmaceutical composition is capable of eliciting a protective immune response in a subject, particularly a ruminant, against a disease caused by a microorganism, particularly a virus from the viral genus *Capripoxviridae.*

According to one embodiment, the disease may be selected from the group consisting of lumpy skin disease, sheep pox, and goat pox.

In yet another embodiment, the ruminant is selected from the group consisting of cattle, sheep, goats, and wild ungulates.

The pharmaceutical composition of the present invention may be formulated for intramuscular, intradermal, intravenous or subcutaneous administration.

According to a further aspect of the present invention there is provided for a recombinant virus or recombinant LSDV as described, for use in a method of preventing a disease in a subject, particularly a ruminant. Preferably, the disease is caused by a microorganism, particularly a virus from the viral genus *Capripoxviridae.*

In another embodiment, the disease may be selected from the group consisting of lumpy skin disease, sheep pox, and goat pox.

In yet another embodiment, the ruminant is selected from the group consisting of cattle, sheep, goats, and wild ungulates.

### BRIEF DESCRIPTION OF THE FIGURES

Non-limiting embodiments of the invention will now be described by way of example only and with reference to the following figures:

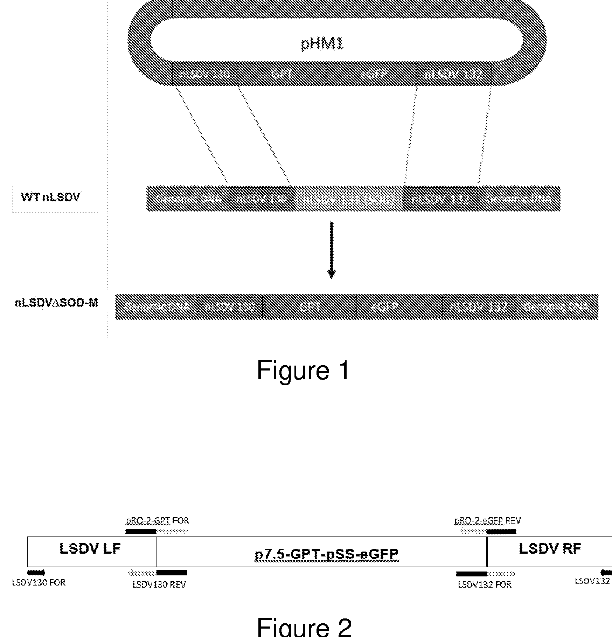

**Figure 1:** Schematic showing the deletion of SOD-homolog gene (ORF131) from nLSDV and replacement with GPT and eGFP marker gene to product nLSDVΔSOD-M. Within the infected and transfected cell homologous recombination between the plasmid and nLSDV genomic DNA at the left flank (ORF 130) and right flank (ORF 132) resulted in ORF 131 being replaced by the GPT and eGFP genes present in plasmid pHM1.
**Figure 2:** Diagram showing overlapping primers for assembly of fragments in the construction of pHM1.

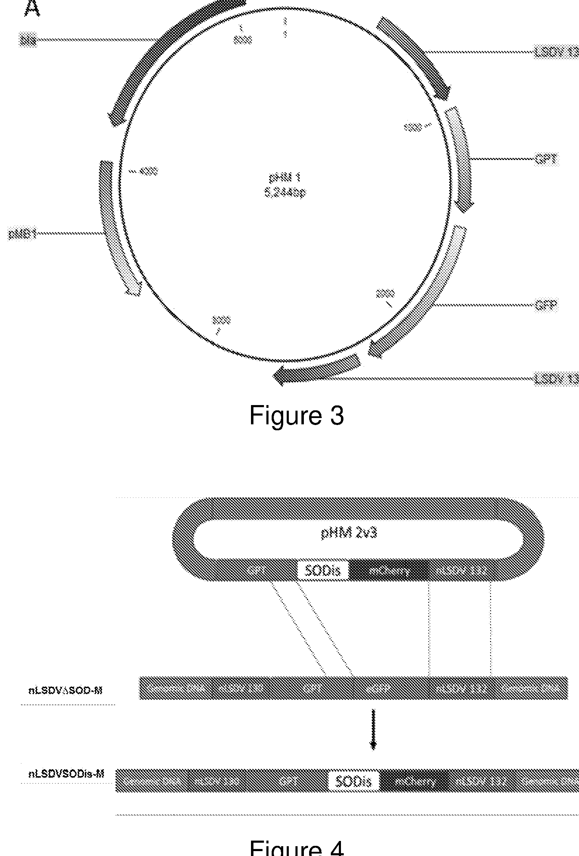

**Figure 3:** Transfer vector pHM1. LSDV 130 and 132 are open reading frames that flank the LSDV ORF131 (SOD). GPT - xanthine-guanine phosphoribosyltransferase, GFP - green fluorescence protein, bla - B-lactamase.
**Figure 4:** Schematic showing the insertion of a synthetic SOD-homolog gene (SODis) into nLSDVΔSOD-M to construct nLSDVSODis-M.

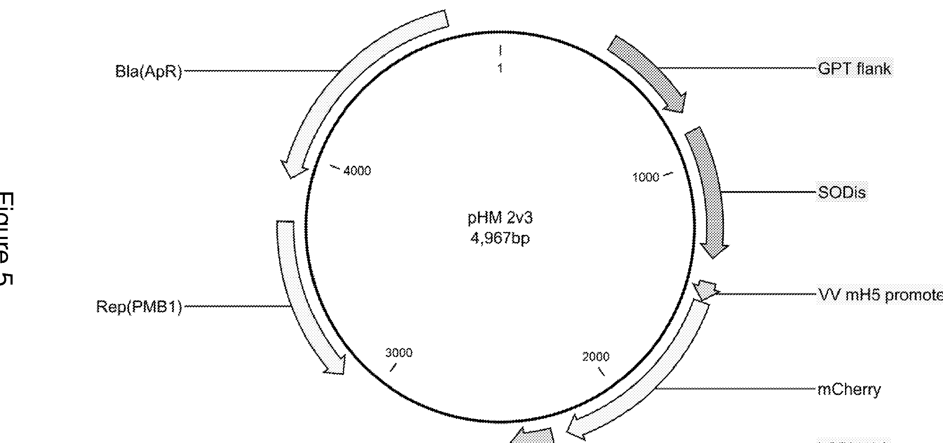

**Figure 5:** Plasmid map of transfer vector pHM2v3. GPT and LSDV132 served as flanking sequences for homologous recombination, such that the eGFP gene of nLSDVΔSOD-M can be replaced with the synthetic SOD-homolog gene (SODis) under its native promoter and mCherry under the control of the mH5 promoter.

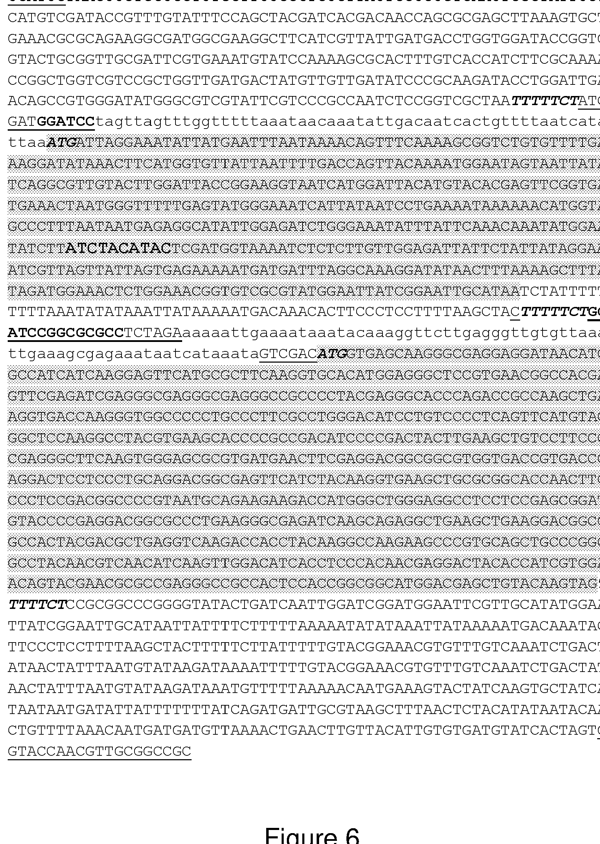

**Figure 6:** DNA sequence of pHM2v3 between the sites of insertion (Pvul and Notl). This sequence was synthesized and ligated into a pUC57 simple vector by Gene Script (USA). Restriction sites are underlined and the start codons of SODis and mCherry are italicized in bold. Also in bold italics are transcriptional stop signals. The native SOD-homolog gene promoter and the mH5 promoter upstream of the mCherry gene are depicted in lower case. Highlighted in bold and of larger font is the sequence that was altered to improve stability of the SOD-homolog gene by removing a series of AT repeats, yet retaining the translated amino acid sequence. An abbreviated form of the sequence is: Pvul-GPT-Clal-BamH1 - SODis ***-TTTTTCT*** - BamHl-Ascl- Xbal-PmH5 -Sall-mCherry - ***TTTTTCT-*** Sacll- Smal-LSDV 132- Kpnl- Acll-Notl.

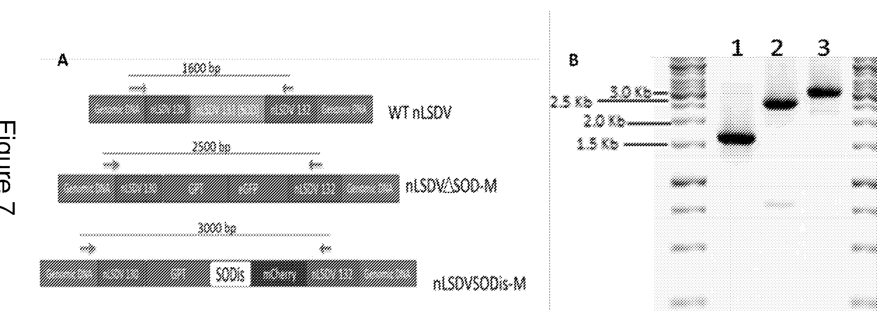

**Figure 7:** Confirmation of recombinant viruses nLSDVΔSOD-M and nLSDVSODis-M by PCR: Panel A - Schematic showing fragment sizes generated using a primer pair which bind in LSDV ORFs 129 and 132. Panel B - 1% Agarose gel electrophoresis of PCR products amplified from WT nLSDV (lane 1), nLSDVΔSOD-M (lane 2) and nLSDVSODis-M (lane 3).

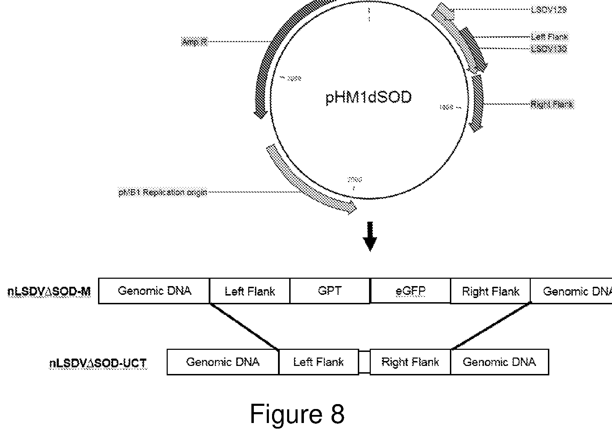

**Figure 8:** Transfer vector pHM1dSOD and strategy of construction of nLSDVΔSOD-UCT using nLSDVΔSOD-M as the parent virus. Homologous recombination can occur between the left (LSDV ORF 130) and right (LSDV ORF132) flanks, resulting in the removal of GPT and eGFP from the parent virus and the generation of a non-fluorescing recombinant lacking the LSDV SOD-homolog gene (ORF 131).

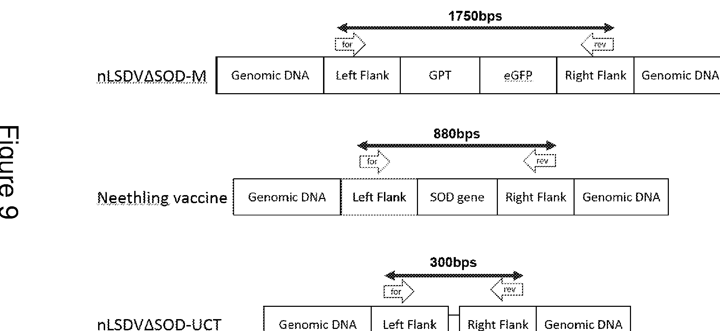

**Figure 9:** Confirmation of recombinant virus nLSDVΔSOD-UCT by PCR: Diagram to show where primers bind and sizes of PCR products amplified from the parent virus nLSDVΔSOD-M, the original parent virus Neethling and the recombinant nLSDVΔSOD-UCT.

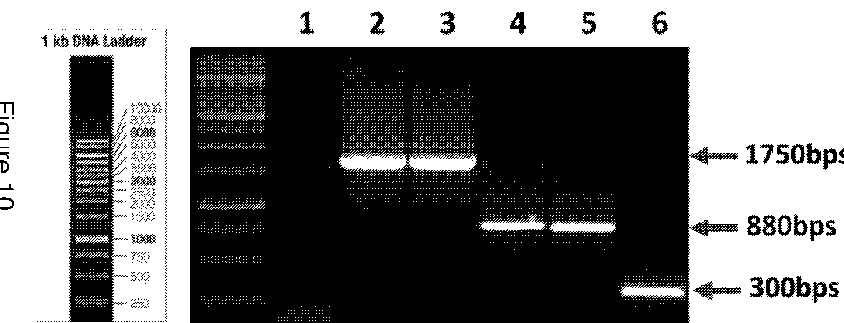

**Figure 10:** Confirmation of recombinant virus nLSDVΔSOD-UCT by PCR: Agarose gel electrophoresis of PCR products amplified from uninfected MDBK cells (lane 1), nLSDVΔSOD-M (lanes 2 and 3), nLSDV (lanes 4 and 5) and nLSDVΔSOD-UCT (lane 6). Primer sequences used were: LSDV 130 for: 5'-GGAGCACCATTTCCATATAC -3' (SEQ ID NO:24) and LSDV 132 rev: 5'-AGTAGCTTAAAAGGAGGGAAG -3' (SEQ ID NO:25).

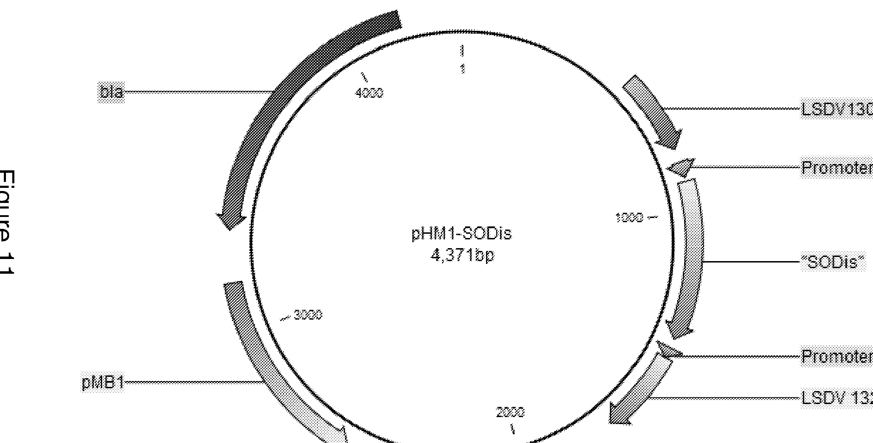

**Figure 11:** Transfer vector pHM1-SODis. This transfer vector has the novel SODis gene inserted between LSDV ORFs 130 and 132. It was used to remove the marker genes from nLSDVΔSOD-M in the construction of nLSDVSODis-UCT.

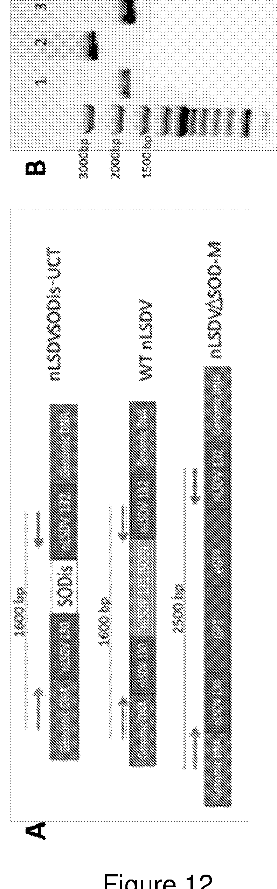

**Figure 12:** Confirmation of recombinant virus nLSDVSODis-UCT by PCR. Panel A - diagram to show sizes of PCR products amplified from the desired recombinant virus nLSDVDOSis-UCT, nLSDV and parent virus nLSDVΔSOD-M. Primers used were: forward primer 129F: 5'- GAGCCCTGTAATTCACTTTT - 3' (SEQ ID NO:26) and reverse primer 132R: 5' - ATCGATGGAAAAGATCCG - 3' (SEQ ID NO:27). Panel B - 1% agarose gel electrophoresis of PCR products amplified from nLSDVSODis-UCT (lane 1), the parent virus, nLSDVΔSOD-M (lane 2) and nLSDV (lane 3).

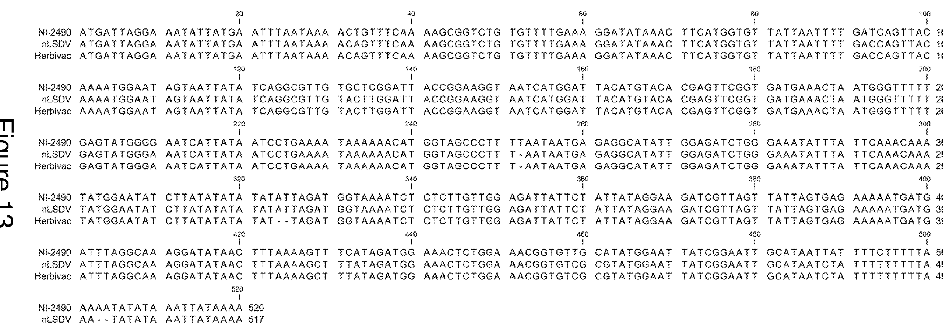

**Figure 13:** Nucleotide alignment of the SOD homologs from the virulent Neethling NI-2490, the OBP Neethling nLSDV and Herbivac. Herbivac and nLSDV share a single base pair deletion at position 252. Herbivac has a unique deletion of 2bp at positions 324-325, which restores the SOD-homolog gene ORF. It is noteworthy that the sequence from positions 314-325 comprises repeated AT binucleotides which explains how the 2bp deletion could have arisen.

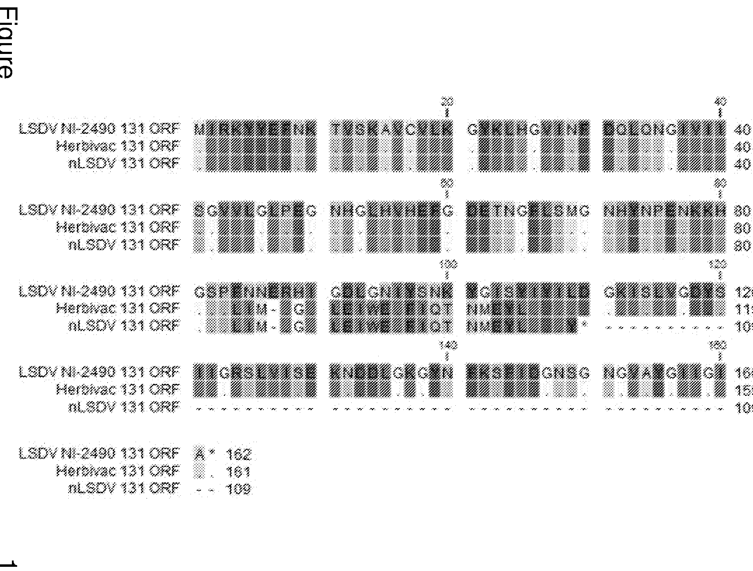

**Figure 14:** Amino acid alignment of the SOD homologs from the virulent Neethling NI-2490, Herbivac and the OBP Neethling nLSDV. The single base pair deletion common to nLSDV and Herbivac alters the amino acid sequence at position 84. The 2bp deletion unique to Herbivac restores the SOD-homolog gene ORF such that Herbivac resembles the virulent NI-2490 from position 106.

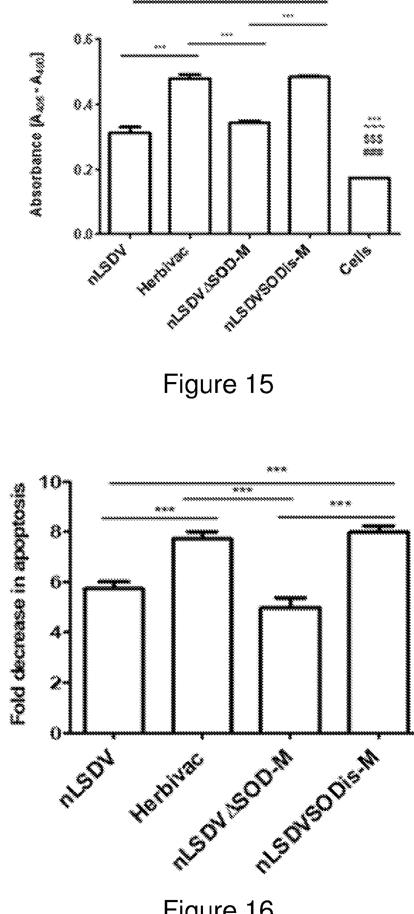

**Figure 15:** Induction of apoptosis. MDBK cells were infected with nLSDV, Herbivac, nLSDVΔSOD-M and nLSDVSODis-M at an MOI of 1. Uninfected cells were used as a control. Six hours post infection the cell medium was removed and the cells lysed. The Roche cell death detection ELISAPLUS kit was used to measure the number of nucleosomes released. The symbol *** represents a significance level of p<0.001. Bars denote mean and error bars SEM.
**Figure 16:** Inhibition of camptothecin induced apoptosis. MDBK cells were infected with nLSDV, Herbivac, nLSDVΔSOD-M and nLSDVSODis-M at an MOI of 1 in duplicate sets of wells. Uninfected cells were used as a control. Apoptosis was induced with 0.0001mg/ml camptothecin in one set of wells. The enrichment factor after induction of apoptosis was calculated and is expressed as fold decrease in apoptosis compared to uninfected cells.

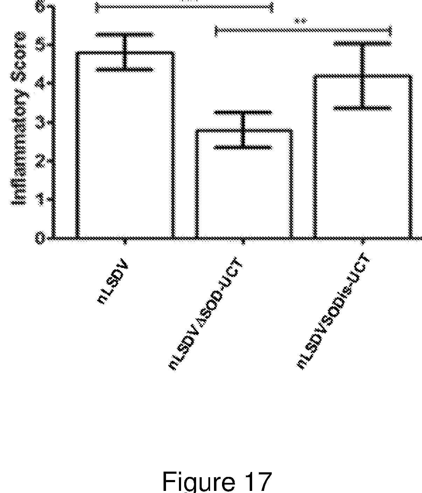

**Figure 17:** Inflammatory changes associated with the SOD-homolog gene. Seven day old fertilized chick embryo chorioallantoic membranes (CAMs) were infected for 5 days, washed, fixed with 4% buffered formalin, embedded, sectioned and stained with haematoxylin and eosin. For each virus-infected membrane five sections were evaluated under the light microscope and scored for inflammation (oedema, haemorrhage and infiltration with white blood cells). One-way ANOVA and Tukey post hoc testing was used to test for significance. The symbols ** and *** represent significance levels of p<0.01 and p<0.001 respectively. Bars denote mean and error bars denote standard deviation.

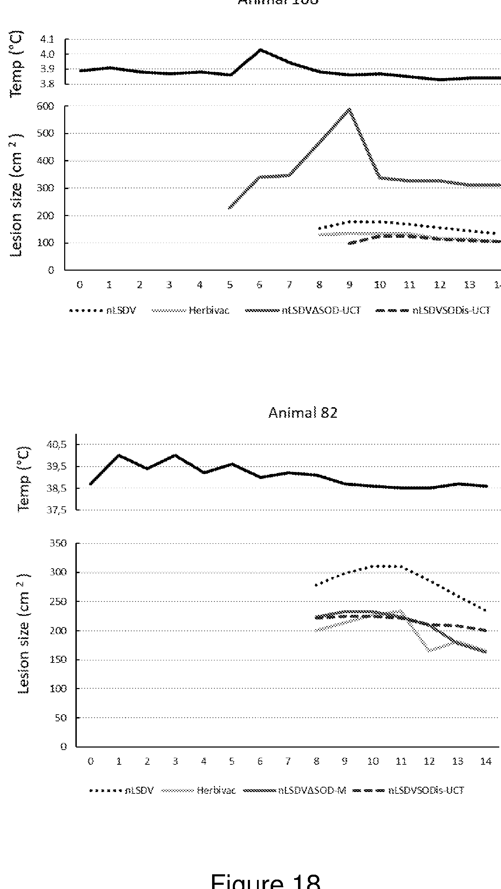

**Figure 18:** Comparison of lesions produced by different LSDV vaccines in cows. A group of five friesian calves, 6-12 months old, and LSDV seronegative, were each inoculated at different sites on the neck with a dose of 104 TCID50 of each of nLSDV, Herbivac, nLSDVΔSOD-UCT and nLSDVSODis-UCT. The animals were monitored daily for two weeks for temperature and lesion size. Two of the five animals (Animals 166 and 82) developed lesions. The surface area (cm²) of the lesions was measured and is graphically displayed. The black continuous line graph above represents rectal temperature. The neutralization titres at the end of the experiment were 1:64 for Animal 166 and 1:128 for Animal 82.

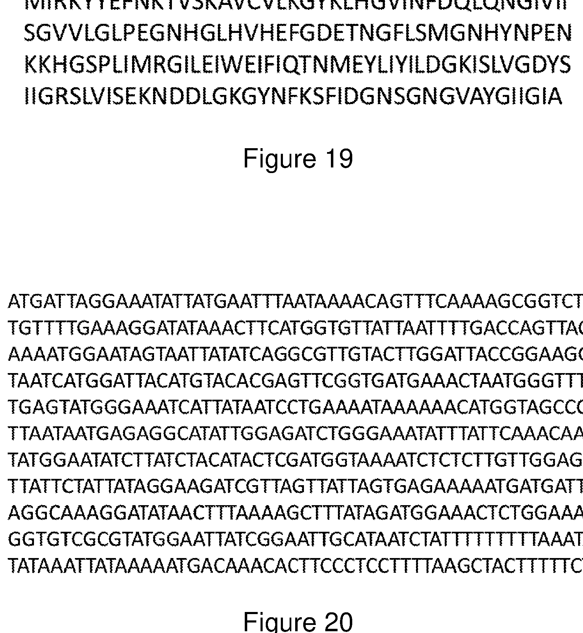

**Figure 19:** Amino acid sequence of the SOD-homolog polypeptide expressed from the nLSDVSODis-M and nLSDVSODis-UCT constructs (SEQ ID NO:1).
**Figure 20:** Nucleic acid sequence of the stabilised SOD-homolog (SODis) gene used in the nLSDVSODis-M and nLSDVSODis-UCT constructs (SEQ ID NO:2).

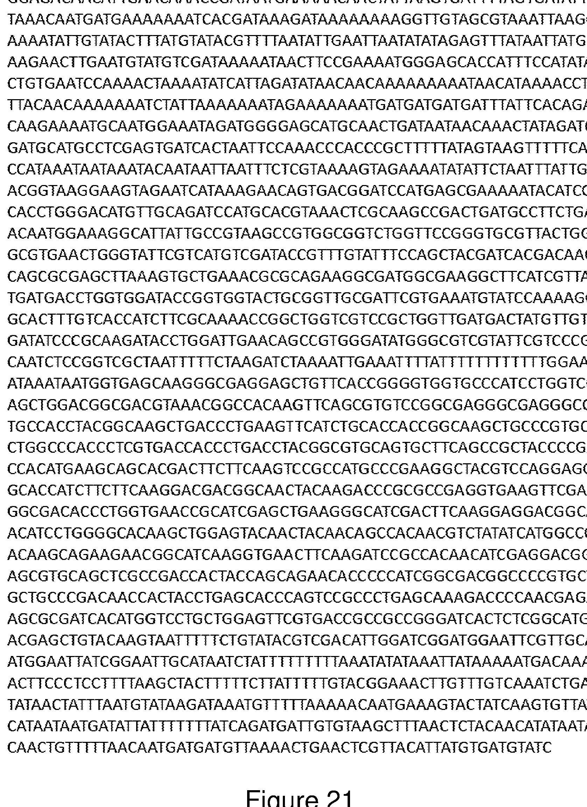

**Figure 21:** Nucleic acid sequence of the pHM1 insertion cassette used to obtain the nLSDVΔSOD-M construct (SEQ ID NO:20).

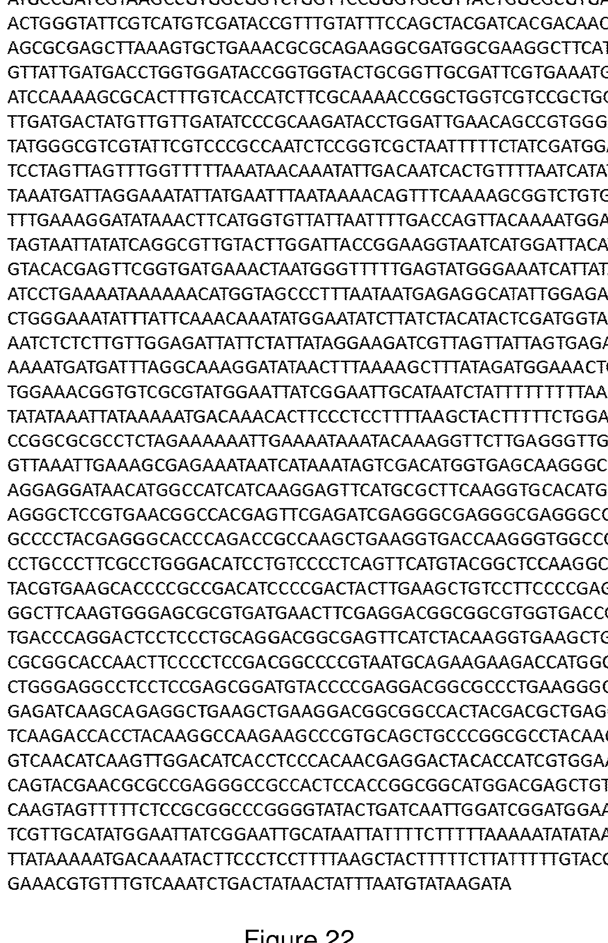

**Figure 22:** Nucleic acid sequence of the pHM2v3 insertion cassette used to obtain the nLSDVSODis-M construct (SEQ ID NO:21).

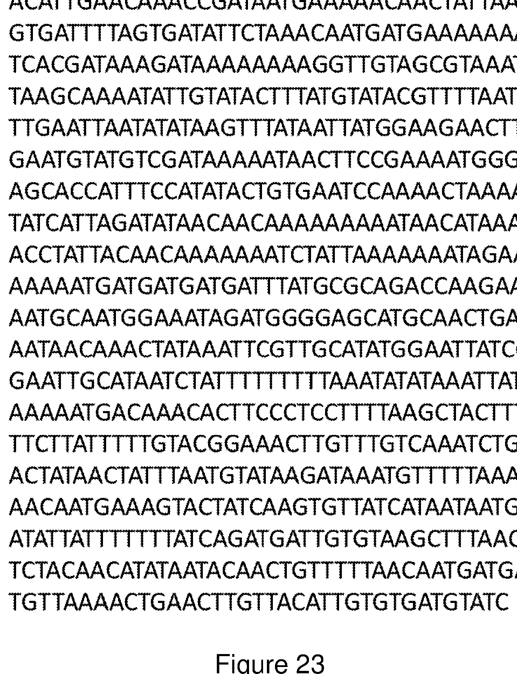

**Figure 23:** Nucleic acid sequence of the pHM1ΔSOD insertion cassette used to obtain the nLSDVΔSOD-UCT construct (SEQ ID NO:22).

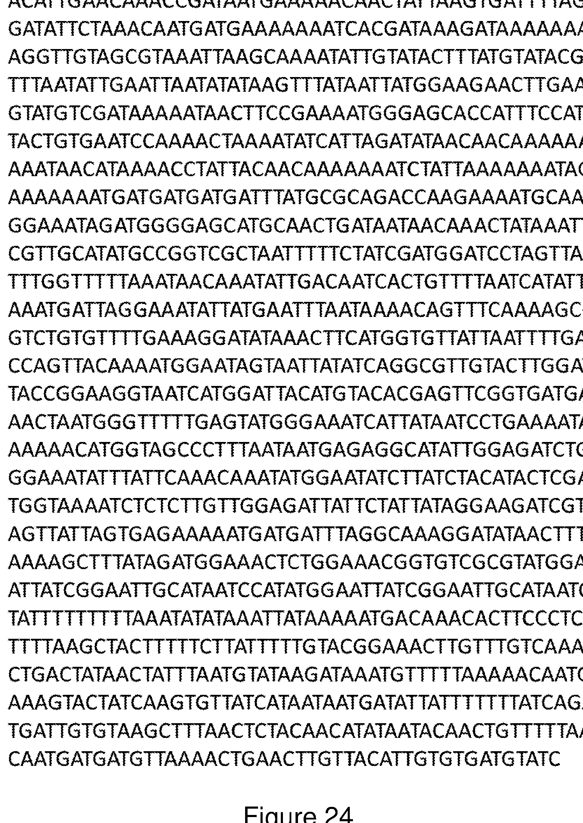

**Figure 24:** Nucleic acid sequence of the pHM1-SODis insertion cassette used to obtain the nLSDVSODis-UCT construct (SEQ ID NO:23).

### SEQUENCE LISTING

The nucleic acid and amino acid sequences listed in the accompanying sequence listing are shown using standard letter abbreviations for nucleotide bases, and the standard three letter abbreviations for amino acids. It will be understood by those of skill in the art that only one strand of each nucleic acid sequence is shown, but that the complementary strand is included by any reference to the displayed strand. In the accompanying sequence listing:
SEQ ID NO:1 - Amino acid sequence of the SOD-homolog expressed from the SODis gene.
SEQ ID NO:2 - Nucleic acid sequence of SODis gene.
SEQ ID NO:3 - Full genome sequence of Herbivac vaccine strain.
SEQ ID NO:4 - Full genome sequence of nLSDVΔSOD-UCT vaccine construct.
SEQ ID NO:5 - Full genome sequence of nLSDVΔSODis-UCT vaccine construct.
SEQ ID NO:6 - Nucleic acid sequence of LSDV 130 forward primer used to amplify flanking sequences of nLSDV ORF 130 from pZG1.
SEQ ID NO:7 - Nucleic acid sequence of LSDV 130 reverse primer used to amplify flanking sequences of nLSDV ORF 130 from pZG1.
SEQ ID NO:8 - Nucleic acid sequence of pRO-2-GPT forward primer used to amplify selection and marker genes from pRO-2.
SEQ ID NO:9 - Nucleic acid sequence of pRO-2-eGFP reverse primer used to amplify selection and marker genes from pRO-2.
SEQ ID NO:10 - Nucleic acid sequence of LSDV 132 forward primer used to amplify flanking sequences of nLSDV ORF 132 from pZG2.
SEQ ID NO:11 - Nucleic acid sequence of LSDV 132 reverse primer used to amplify flanking sequences of nLSDV ORF 132 from pZG2.
SEQ ID NO:12 - Nucleic acid sequence of pHM2v3 between the sites of insertion (Pvul and Notl).
SEQ ID NO:13 - Nucleic acid sequence encoding NI-2490 SOD-homolog.
SEQ ID NO:14 - Nucleic acid sequence encoding nLSDV SOD-homolog.
SEQ ID NO:15 - Nucleotide sequence encoding nLSDV SOD-homolog of truncated ORF
SEQ ID NO:16 - Nucleic acid sequence encoding Herbivac SOD-homolog.
SEQ ID NO:17 -Amino acid sequence of NI-2490 SOD-homolog.
SEQ ID NO:18 - Amino acid sequence of nLSDV SOD-homolog.
SEQ ID NO:19 - Amino acid sequence of Herbivac SOD-homolog.
SEQ ID NO:20 - Nucleic acid sequence of the pHM1 insertion cassette used to make the nLSDVΔSOD-M construct.
SEQ ID NO:21 - Nucleic acid sequence of the pHM2v3 insertion cassette used to obtain the nLSDVSODis-M construct.
SEQ ID NO:22 - Nucleic acid sequence of the pHM1ΔSOD insertion cassette used to obtain the nLSDVΔSOD-UCT construct.
SEQ ID NO:23 - Nucleic acid sequence of the pHM1-SODis insertion cassette used to obtain the nLSDVSODis-UCT construct.

### DETAILED DESCRIPTION OF THE INVENTION

The present invention will now be described more fully hereinafter with reference to the accompanying drawings, in which some, but not all embodiments of the invention are shown.

The invention as described should not be limited to the specific embodiments disclosed and modifications and other embodiments are intended to be included within the scope of the invention. Although specific terms are employed herein, they are used in a generic and descriptive sense only and not for purposes of limitation.

As used throughout this specification and in the claims which follow, the singular forms "a", "an" and "the" include the plural form, unless the context clearly indicates otherwise.

The terminology and phraseology used herein is for the purpose of description and should not be regarded as limiting. The use of the terms "comprising", "containing", "having" and "including" and variations thereof used herein, are meant to encompass the items listed thereafter and equivalents thereof as well as additional items.

The present invention relates to modified viral vaccine backbones. In particular, the inventors of the present invention have identified a gene (superoxide dismutase homolog (SOD)) which may influence immunogenicity of live virus vectors, in particular, poxviruses, which include Lumpy Skin Disease Virus (LSDV), other *capripoxviruses, orthopoxviruses, avipoxviruses,* as well as other viral vectors.

The Neethling vaccine strain of LSDV (nLSDV) was modified so as to delete the gene encoding the SOD-homolog completely and produce the vaccine nLSDVΔSOD-UCT (SEQ ID NO:4). In another embodiment, nLSDVSODis-UCT (SEQ ID NO:5) was produced, wherein the Neethling vaccine strain of LSDV SOD-homolog gene was replaced by a modified SOD-homolog gene, termed SODis, which has been modified so as to improve its stability. Two additional recombinants have been generated which contain marker genes. nLSDVΔSOD-M has the gene encoding the SOD-homolog deleted and replaced by the *E.coli* guanine phosphoribosyltransferase (GPT) gene and the enhanced Green Fluorescent Protein gene (eGFP). nLSDVSODis-M has the gene encoding the SOD-homolog replaced by the synthetic novel SODis gene, as well as GPT and mCherry (red fluorescence protein).

The SODis was constructed by the inventors by altering the nucleotide sequence of the Neethling vaccine strain SOD-homolog gene such that the repeat sequences of adenosine and thymine were removed, yet the amino acid sequence was identical to that of the Herbivac SOD homolog gene. Whole genome sequencing of the Herbivac vaccine indicated a mixed viral population where some viruses contained a restored SOD-homolog gene and some a truncated one. It is believed the mixed populated is due to the instability of the native SOD-homolog gene. When whole genome sequencing was performed on a population of nLSDVSODis-UCT all viruses contained the SODis gene and no truncated SOD-homolog sequences could be detected within the viral population.

The deletion or modification of the SOD-homolog gene in nLSDV has been engineered to improve immunogenicity of the vaccine or vaccine vector. The modified SOD homolog gene may be useful in other viral vectors to improve immunogenicity of the vaccine.

An improved LSDV vaccine would be of economic importance in the cattle industry. In addition the viral vector backbone could be used in the development of vaccines against other diseases, both veterinary and human. The SODis gene may be used in other viral vector backbones to improve immunogenicity.

Histological examination of infected chicken chorioallantoic membranes show that the presence and expression of the SODis gene increases mesodermal proliferation and immune cell infiltration at the site of infection. The expression of the SODis gene to produce a functional SOD-homolog also functions by inhibiting apoptosis.

Histologically, LSDV with the SOD-homolog gene deleted (nLSDVΔSOD-M and nLSDVΔSOD-UCT) and LSDV containing the modified SOD-homolog gene (nLSDVSODis-M and nLSDVSODis-UCT) differed from the Neethling LSDV. Herbivac resulted in increased immune cell infiltration at the site of infection compared to the parent Neethling vaccine strain and membranes infected with nLSDVSODis-M and nLSDVSODis-UCT showed marked proliferation of fibroblasts compared to nLSDVΔSOD-M and nLSDVΔSOD-UCT infected membranes.

It appears that inclusion of the SODis gene improves the LSDV vaccine in general. However, all vaccines including the SODis gene and those having the SOD-homolog gene deleted appear to induce different responses *in vivo,* as reflected by histological examination of chick CAMs and preliminary necrosis experiments.

It is known that cattle breeds differ in their responses to vaccination. More inbred European cattle are more sensitive to vaccination and are more likely to have adverse reactions to vaccination compared to the "hardier" animals found in more rural settlements, such as those in Africa. It is possible that the presence of the SODis gene and expression of the full-length SOD-homolog or the deletion of the SOD gene would not only alter the immunogenicity but also the virulence of the vaccine. A vaccine with increased virulence would not be desirable for use in more sensitive inbred cattle, but, if more immunogenic, may be better for use in Africa, where the cattle are hardier. A less virulent vaccine would be more desirable in places like Europe, where the cattle are inbred. The Neethling strain of LSDV has a partial SOD-homolog gene. It would appear that this truncated protein product has some effect on virus-host interaction, making the recombinant virus (in which the SOD-homolog gene is deleted) different from its parent strain. The differences between Neethling, LSDV having a deleted SOD-homolog gene and LSDV with the modified SODis gene will be further tested in cattle.

The modified viral vaccine backbones, vaccine constructs and compositions of the present invention are useful as improved vaccines against LSDV or as vector backbones for additional vaccines, either multivalent veterinary vaccines, or vectors for vaccines such as, but not restricted to, HIV-1.

The constructs according to the invention include, without limitation, a lumpy skin disease virus vaccine construct, wherein the gene or a functional part of the gene encoding a SOD-homolog has been deleted or inactivated in the viral genome. Alternatively, the constructs according to the present invention include a lumpy skin disease virus vaccine construct wherein the gene encoding the SOD-homolog has been stabilised.

The present invention targets the gene encoding a SOD-homolog of lumpy skin disease virus which is situated in open reading frame 131 (ORF131) of the LSDV Neethling strain. SEQ ID NO:1 provides a representative sequence of the SOD-homolog encoded by the stabilised SOD-homolog gene, termed SODis, having a nucleotide sequence of SEQ ID NO:2. SEQ ID NO:4 provides a representative sequence of the viral vaccine backbone of the present invention wherein the gene or a functional part of the gene encoding a SOD-homolog has been deleted from the viral genome. SEQ ID NO:5 provides a representative sequence of the viral vaccine backbone comprising a stabilised gene encoding the SOD-homolog.

By "lumpy skin disease" or "LSD" is meant an infectious disease in cattle caused by a virus of the family *Poxviridae.* The disease is characterized by fever, enlarged superficial lymph nodes and multiple nodules approximately 2-5 cm in diameter on the skin and mucous. Infected subjects also may develop edematous swelling in the limbs and exhibit lameness. LSD is caused by "lumpy skin disease virus" or "LSDV". LSD has important economic implications since affected animals tend to have permanent damage to their skin, lowering the commercial value of their hide, as well as chronic debility, reduced milk production, poor growth, infertility, abortion, and sometimes death.

Onset of fever, which may exceed 41°C, occurs almost one week after infection by the virus and persists for about one week. Around 1 week post-infection, all of the superficial lymph nodes become enlarged. Nodules appear seven to nineteen days after viral inoculation. The nodular lesions involve the dermis and the epidermis, but may extend to the underlying subcutis or even to the muscle. These lesions occur all over the body (but particularly on the head, neck, udder, scrotum, vulva and perineum).

LSDV is a double stranded deoxyribonucleic acid (dsDNA) virus. It is a member of the *capripoxvirus* genus of *Poxviridae* with an average virion size of 320 nm by 260 nm. Lumpy skin disease virus has a 151 kbp genome, consisting of a central coding region which is bounded by identical 2.4 kbp inverted terminal repeats and contains 156 genes. There are 146 conserved genes when comparing LSDV with *chordopoxviruses* of other genera. Within the central genomic region, LSDV genes share a high degree of collinearity and amino acid identity with the genes of other mammalian poxviruses. Examples of viruses with similar amino acid identity include *suipoxvirus, yatapoxvirus,* and *leporipoxvirus.*

It will be appreciated by those of skill in the art that the viral vaccine backbones, constructs and compositions of the present invention, in particular the viral vaccine backbone either comprising a stabilised SODis gene or having the SOD-homolog gene deleted may be useful in viral vaccines for microorganisms other than for LSDV, including viral vaccines for Human Immunodeficiency Virus-1 (HIV-1), Bovine Leukaemia Virus (BVL), East Coast Fever (ECF), rabies, Rift Valley Fever Virus (RVFV).

By "condition associated with poxvirus infection" is meant any condition, disease or disorder that has been correlated with the presence of an existing poxvirus infection, it includes secondary effects, such as reductions in milk production, temporary or permanent infertility in cows and bulls, abortions and damage to hides, weight loss and wool brake.

A "protein," "peptide" or "polypeptide" is any chain of two or more amino acids, including naturally occurring or non-naturally occurring amino acids or amino acid analogues, irrespective of post-translational modification (e.g., glycosylation or phosphorylation).

The terms "nucleic acid" or "nucleic acid molecule" encompass both ribonucelotides (RNA) and deoxyribonucleotides (DNA), including cDNA, genomic DNA, and synthetic DNA. The nucleic acid may be double-stranded or single-stranded. Where the nucleic acid is single-stranded, the nucleic acid may be the sense strand or the antisense strand. A nucleic acid molecule may be any chain of two or more covalently bonded nucleotides, including naturally occurring or non-naturally occurring nucleotides, or nucleotide analogs or derivatives. By "RNA" is meant a sequence of two or more covalently bonded, naturally occurring or modified ribonucleotides. The term "DNA" refers to a sequence of two or more covalently bonded, naturally occurring or modified deoxyribonucleotides.

The term "complementary" refers to two nucleic acids molecules, e.g., DNA or RNA, which are capable of forming Watson-Crick base pairs to produce a region of double-strandedness between the two nucleic acid molecules. It will be appreciated by those of skill in the art that each nucleotide in a nucleic acid molecule need not form a matched Watson-Crick base pair with a nucleotide in an opposing complementary strand to form a duplex. One nucleic acid molecule is thus "complementary" to a second nucleic acid molecule if it hybridizes, under conditions of high stringency, with the second nucleic acid molecule. A nucleic acid molecule according to the invention includes both complementary molecules.

In some embodiments, a viral vaccine backbone of the invention may include, without limitation, SODis (SEQ ID NO:1) encoded by the SODis gene (SEQ ID NO:2), the vaccine constructs of the invention may include, without limitation nLSDVΔSOD-UCT (SEQ ID NO:4), nLSDVSODis-UCT (SEQ ID NO:5), nLSDVΔSOD-M, or nLSDVSODis-M.

As used herein a "substantially identical" sequence is an amino acid or nucleotide sequence that differs from a reference sequence only by one or more conservative substitutions, or by one or more non-conservative substitutions, deletions, or insertions located at positions of the sequence that do not destroy or substantially reduce the antigenicity of one or more of the expressed polypeptides or of the polypeptides encoded by the nucleic acid molecules. Alignment for purposes of determining percent sequence identity can be achieved in various ways that are within the knowledge of those with skill in the art. These include using, for instance, computer software such as ALIGN, Megalign (DNASTAR), CLUSTALW or BLAST software. Those skilled in the art can readily determine appropriate parameters for measuring alignment, including any algorithms needed to achieve maximal alignment over the full length of the sequences being compared. In one embodiment of the invention there is provided for a polypeptide or polynucleotide sequence that has at least about 80% sequence identity, at least about 90% sequence identity, or even greater sequence identity, such as about 95%, about 96%, about 97%, about 98% or about 99% sequence identity to the sequences described herein.

Alternatively, or additionally, two nucleic acid sequences may be "substantially identical" if they hybridize under high stringency conditions. The "stringency" of a hybridisation reaction is readily determinable by one of ordinary skill in the art, and generally is an empirical calculation which depends upon probe length, washing temperature, and salt concentration. In general, longer probes required higher temperatures for proper annealing, while shorter probes require lower temperatures. Hybridisation generally depends on the ability of denatured DNA to re-anneal when complementary strands are present in an environment below their melting temperature. A typical example of such "stringent" hybridisation conditions would be hybridisation carried out for 18 hours at 65°C with gentle shaking, a first wash for 12 min at 65°C in Wash Buffer A (0.5% SDS; 2XSSC), and a second wash for 10 min at 65°C in Wash Buffer B (0.1% SDS; 0.5% SSC).

In some embodiments, the nucleic acid molecules of the invention may be operably linked to other sequences. By "operably linked" is meant that the nucleic acid molecules encoding the SODis gene of the invention and regulatory sequences are connected in such a way as to permit expression of the proteins when the appropriate molecules are bound to the regulatory sequences. Similarly, the nucleic acid molecules encoding the marker genes of the viral vaccine constructs of the present invention may also be operably linked to regulatory sequences. Such operably linked sequences may be contained in vectors or expression constructs which can be transformed or transfected into host cells for expression. It will be appreciated that any vector or vectors can be used for the purposes of expressing the SOD-homolog encoded by the SODis gene of the invention.

The term "recombinant" means that something has been recombined. When used with reference to a nucleic acid construct the term refers to a molecule that comprises nucleic acid sequences that are joined together or produced by means of molecular biological techniques. Recombinant nucleic acid constructs may include a nucleotide sequence which is ligated to, or is manipulated to become ligated to, a nucleic acid sequence to which it is not ligated in nature, or to which it is ligated at a different location in nature. Accordingly, a recombinant nucleic acid construct indicates that the nucleic acid molecule has been manipulated using genetic engineering, i.e. by human intervention. Recombinant nucleic acid constructs may be introduced into a host cell by infection or transfection. Such recombinant nucleic acid constructs may include sequences derived from the same host cell species or from different host cell species.

The term "homologous recombination" refers to a mechanism of genetic recombination in which two nucleic acid strands comprising similar nucleotide sequences exchange genetic material. Cells use homologous recombination during meiosis, where it serves to rearrange DNA to create an entirely unique set of haploid chromosomes, but also for the repair of damaged DNA, in particular for the repair of double strand breaks. The mechanism of homologous recombination is well known to the skilled person in the art.

The term "vector" refers to a means by which polynucleotides or gene sequences can be introduced into a cell. There are various types of vectors known in the art including plasmids, viruses, bacteriophages and cosmids. Generally, polynucleotides or gene sequences are introduced into a vector by means of a cassette. The term "cassette" refers to a polynucleotide or gene sequence that is expressed from a vector, for example, the SODis sequence encoding the SOD-homolog polypeptides of the invention. A cassette generally comprises a gene sequence inserted into a vector, which in some embodiments, provides regulatory sequences for expressing the polynucleotide or gene sequences. In other embodiments, the vector provides the regulatory sequences for the expression of the SOD-homolog polypeptide. In further embodiments, the vector provides some regulatory sequences and the nucleotide or gene sequence provides other regulatory sequences. "Regulatory sequences" include but are not limited to promoters, transcription termination sequences, enhancers, splice acceptors, donor sequences, introns, ribosome binding sequences, poly(A) addition sequences, and/or origins of replication.

As used herein the term "homolog" refers to a gene or polypeptide related to another gene or polypeptide by descent from a common ancestral nucleic acid or amino acid sequence. The term homolog may apply to the relationship between genes or polypeptides separated by an event of speciation or to the relationship between genes or polypeptides separated by an event of genetic duplication.

As used herein a "pharmaceutically acceptable carrier" or "excipient" includes any and all antibacterial and antifungal agents, coatings, dispersion media, solvents, isotonic and absorption delaying agents, and the like that are physiologically compatible. A "pharmaceutically acceptable carrier" may include a solid or liquid filler, diluent or encapsulating substance which may be safely used for the administration of the viral vaccine backbones, constructs and/or compositions to a subject. The pharmaceutically acceptable carrier can be suitable for intramuscular, intradermal, intravenous, intraperitoneal, subcutaneous, oral or sublingual administration. Pharmaceutically acceptable carriers include sterile aqueous solutions, dispersions and sterile powders for the preparation of sterile solutions. The use of media and agents for the preparation of pharmaceutically active substances is well known in the art. Where any conventional media or agent is incompatible with the viral vaccine backbone and/or construct, use thereof in the pharmaceutical compositions of the invention is not contemplated. Supplementary active compounds can also be incorporated into the compositions.

Suitable formulations or compositions to administer the viral vaccine backbones, constructs and/or compositions to subjects suffering from viral infection, particularly poxvirus infection, or subjects which are presymptomatic for a condition associated with viral infection fall within the scope of the invention. Any appropriate route of administration may be employed, such as, parenteral, intravenous, subcutaneous, intramuscular, intracranial, intraorbital, ophthalmic, intraventricular, intracapsular, intraspinal, intrathecal, intracistemal, intraperitoneal, intranasal, aerosol, topical, or oral administration.

As used herein the term "subject" includes mammals. Preferably the subjects are ruminants, including both wild and domestic ruminants. Most preferably, the subjects are cattle, sheep and/or goats. In an alternative embodiment, the subject is a human.

For vaccine formulations and pharmaceutical compositions, an effective amount of the viral vaccine backbones, constructs and/or compositions of the invention can be provided, either alone or in combination with other compounds (for example nucleic acid molecules, small molecules, peptides, or peptide analogues), with immunological adjuvants (for example, aluminium hydroxide dimethyldioctadecyl-ammonium hydroxide or Freund's incomplete adjuvant). The viral vaccine backbones, constructs and/or compositions of the invention may also be linked with a pharmaceutically acceptable carrier and/or other molecules, such as bovine serum albumin or keyhole limpet haemocyanin in order to enhance immunogenicity. The viral vaccine backbones, constructs and compositions of the invention can also be provided in the presence of a liposome, an adjuvant, or any carrier, such as a and in a form suitable for administration to mammals, for example, humans, cattle, sheep, etc.

In some embodiments, the viral vaccine backbones, constructs and/or compositions according to the invention may be provided in a kit, optionally with a carrier and/or an adjuvant, together with instructions for use.

An "effective amount" of a viral vaccine backbone, construct(s) and/or composition(s) according to the invention includes a therapeutically effective amount, immunologically effective amount, or a prophylactically effective amount. A "therapeutically effective amount" refers to an amount effective, at dosages and for periods of time necessary, to achieve the desired therapeutic result, such as treatment of an infection or a condition associated with such infection. The outcome of the treatment may for example be measured by a decrease in viraemia, inhibition of viral gene expression, delay in development of a pathology associated with poxvirus infection, stimulation of the immune system, or any other method of determining a therapeutic benefit. A therapeutically effective amount of a viral vaccine backbone, construct and/or composition may vary according to factors such as the disease state, age, sex, and weight of the individual, and the ability of the viral vaccine backbone, construct and/or composition to elicit a desired response in the individual. Dosage regimens may be adjusted to provide the optimum therapeutic response. A therapeutically effective amount is also one in which any toxic or detrimental effects of the viral vaccine backbone, construct and/or composition are outweighed by the therapeutically beneficial effects.

The dosage of any of the viral vaccine backbones, constructs and/or compositions of the present invention will vary depending on the symptoms, age and body weight of the subject, the nature and severity of the disorder to be treated or prevented, the route of administration, and the form of the composition. Any of the compositions of the invention may be administered in a single dose or in multiple doses. The dosages of the compositions of the invention may be readily determined by techniques known to those of skill in the art or as taught herein.

By "immunogenically effective amount" is meant an amount effective, at dosages and for periods of time necessary, to achieve a desired immune response. The desired immune response is preferably a "protective immune response" and may include stimulation or elicitation of an immune response, for instance a T-cell response, particularly in order to prevent a disease caused by a virus.

A "prophylactically effective amount" refers to an amount effective, at dosages and for periods of time necessary, to achieve a desired prophylactic result, such as prevention of onset of a condition associated with viral infection, particularly poxvirus infection. Typically, a prophylactic dose is used in subjects prior to or at an earlier stage of disease, so that a prophylactically effective amount may be less than a therapeutically effective amount.

Dosage values may vary and be adjusted over time according to the individual need and the judgment of the person administering or supervising the administration of the lumpy skin disease virus knock-out mutants or compositions of the invention. Dosage ranges set forth herein are exemplary only and do not limit the dosage ranges that may be selected. The amount of viral vaccine backbone or construct in the composition may vary according to factors such as the disease state, age, sex, and weight of the individual. Dosage regimens may be adjusted to provide the optimum therapeutic response. For example, a single dose may be administered, or multiple doses may be administered over time. It may be advantageous to formulate the compositions in dosage unit forms for ease of administration and uniformity of dosage.

The term "preventing", when used in relation to an infectious disease, or other medical disease or condition, is well understood in the art, and includes administration of a composition which reduces the frequency of or delays the onset of symptoms of a condition in a subject relative to a subject which does not receive the composition. Prevention of a disease includes, for example, reducing the number of diagnoses of the infection in a treated population versus an untreated control population, and/or delaying the onset of symptoms of the infection in a treated population versus an untreated control population.

The term "prophylactic or therapeutic" treatment is well known to those of skill in the art and includes administration to a subject of one or more of the compositions of the invention. If the composition is administered prior to clinical manifestation of the unwanted condition (e.g., disease or other unwanted state of the subject) then the treatment is prophylactic, i.e., it protects the host against developing the unwanted condition, whereas if it is administered after manifestation of the unwanted condition, the treatment is therapeutic (i.e., it is intended to diminish, ameliorate, or stabilise the existing unwanted condition or side effects thereof).

Toxicity and therapeutic efficacy of compositions of the invention may be determined by standard pharmaceutical procedures in cell culture or using experimental animals, such as by determining the LD₅₀ and the ED₅₀. Data obtained from the cell cultures and/or animal studies may be used to formulate a dosage range for use in a subject. The dosage of any composition of the invention lies preferably within a range of circulating concentrations that include the ED₅₀ but which has little or no toxicity. The dosage may vary within this range depending upon the dosage form employed and the route of administration utilised. For compositions of the present invention, the therapeutically effective dose may be estimated initially from cell culture assays.

The invention also relates in part to a method of prophylactically treating a subject, comprising administering to the subject in need thereof a prophylactically effective amount of the viral vaccine backbones, constructs and/or compositions of the present invention.

The following examples are offered by way of illustration and not by way of limitation.

### EXAMPLE 1

### Construction of recombinant viruses

All recombinant LSDV viruses were made in a similar fashion. Primary lamb testes (LT) cells were infected with a parent LSDV virus (see sections below for specific parent virus used) at an MOI of approximately 0.05 in a 12-well plate which was approximately 70-80% confluent with 10⁶ cells per well. After a two hour adsorption period the virus was removed and cells were transfected with 3 or 6µg of the transfer vector using X-tremeGENE^{™} Hp transfection reagent (Roche, Switzerland) (see different transfer vectors in each section below). Three days later the cells were lysed by three cycles of freezing and thawing, or by the addition of 1mM Tris, and the lysate used to infect MDBK cells. Individual foci, either fluorescent or not, depending on the recombinant being isolated, were picked and passaged repeatedly. Once the virus population comprised the desired fluorescing or colourless foci, the virus was diluted and single foci were selected in 96 well plates. Single foci were expanded and confirmed to be correct by PCR.

### Construction of nLSDVΔSOD-M

Figure 1 shows how homologous recombination takes place between the transfer vector pHM1 and the nLSDV genome (Accession no: Genbank: AF409138) to generate nLSDVΔSOD-M. The transfer vector was assembled by overlapping PCR (Figure 2) using primers listed in table 1. The flanking sequences were PCR amplified from pZG1 and pZG2 respectively (plasmids which contain the LSDV130 and LSDV132 flanks respectively cloned into pGEM^{®}-T easy). The GPT and eGFP genes were PCR amplified from plasmid pZG4 (plasmid containing GPT and eGFP amplified from pRO-2 and cloned into pGEM^{®}-T easy). Three PCR reactions were performed using the KAPA Hifi Polymerase to amplify the left flank, right flank and GPT-eGFP fragments. The cycling conditions comprised an initial denaturation step at 95 °C for 5 minutes, 25 cycles of: denaturation at 95 °C for 30 seconds, annealing at 55°C for 30 seconds for the left and right flank, and at 60°C for the marker genes, and extension at 70°C for 30 seconds for the left and right flank and 2 minutes for the marker genes. The final extension time was 7 minutes. After amplification, the PCR products were gel purified, quantified and used in an assembly PCR reaction. Equimolar amounts of DNA were added into the same tube for the overlapping PCR. The primers used for the assembly reaction were LSDV 130 forward and LSDV 132 reverse. The conditions for the overlapping PCR included an initial denaturation step at 95 °C for 5 minutes, 25 cycles of: denaturation at 95 °C for 30 seconds, annealing at 55°C for 30 seconds and extension at 70°C for 3 minutes. A final extension time of 7 minutes was used. The product from the overlapping assembly PCR was cloned into pJET (ThermoScientific) to produce pHM 1 (Figure 3).

For the construction of nLSDVΔSOD-M primary LT cells were infected with the Neethling vaccine strain of LSDV (OBP, SA) at an MOI of 0.04 in a 12-well plate and transfected with 3 µg of pHM1. The virus was passaged in MDBK cells in the presence of 2.5 µg/ml mycophenolic acid (MPA), supplemented with hypoxanthine and xanthine (Sigma-Aldrich, USA) seven times, followed by serial dilution in a 96-well plate. A single green fluorescent focus was expanded and confirmed to be the desired recombinant nLSDVΔSOD-M by PCR (Figure 7).

**TABLE 1: Primers used to amplify flanking sequences of LSDV 130 and LSDV 132 from pZG1 and pZG2 respectively, and the selection and marker genes from pRO-2.**

| **Name** | **Primer** | **SEQ ID NO** |
|---|---|---|
| LSDV 130 FOR | GGTACCGGAGACAACATTGAACAAACCGATAATG | SEQ ID NO:6 |
| LSDV 130 REV | GGAATTAGTGATCACTCGAGGCATGCATCGATCTATA GTTTGTTATTATCAGTTGCATG | SEQ ID NO:7 |
| pRO-2-GPT FOR | CATGCAACTGATAATAACAAACTATAGATCGATGCAT GCCTCGAGTGATCACTAATTCC | SEQ ID NO:8 |
| pRO-2-eGFP REV | GCAATTCCGATAATTCCATATGCAACGAATTCCATCC GATCCAATGTCGACGTATACAG | SEQ ID NO:9 |
| LSDV 132 FOR | GTATACGTCGACATTGGATCGGATGGAATTCGTTGC ATATGGAATTATCGGAATTGC | SEQ ID NO:10 |
| LSDV 132 REV | ACTAGTGATACATCACACAATGTAACAAGTTCAG | SEQ ID NO:11 |

### Construction of nLSDVSODis-M

nLSDVSODis-M was constructed using nLSDVΔSOD-M as the parent virus and pHM2v3 as the transfer vector (Figure 4). The eGFP gene was replaced by mCherry, which encodes a red fluorescing protein, and a synthetic SOD-homolog gene, which was designed to have improved stability by removal of repeated nucleotide sequences. Specifically, a series of AT repeats (from position 313 to position 322 of SEQ ID NO:14) was replaced with ATCTACATAC (see position 313 to position 322 of SEQ ID NO:2) (Figure 5 and Figure 6). pHM2v3 was ordered from GenScript.

For the construction of nLSDVSODis-M, primary LT cells were infected with nLSDVΔSOD-M and and transfected with 3 µg of pHM2v3. Forty-eight hours later cells were lysed in 1mM Tris and the lysate passaged in MDBK cells with no selection. Red fluorescing foci were picked over three successive passages. On the fourth passage, in a 96 well plate, a single red focus was isolated and confirmed upon further passage to be the desired recombinant nLSDVSODis-M (Figure 7).

### Construction of nLSDVΔSOD-UCT

nLSDVΔSOD-UCT was constructed so as to remove the marker genes and make a recombinant virus which resembles nLSDV in all ways except for the complete deletion of the SOD-homolog gene in nLSDVΔSOD-UCT when compared to nLSDV. nLSDVΔSOD-M was the parent virus used to infect cells which were transfected with transfer vector pHM1dSOD (Figure 8).

Primary lamb testes cells were seeded into a 6-well plate at a total of 5×10⁵ cells per well. Cells were infected with nLSDVΔSOD-M at an MOI of 0.05 within two hours of seeding. 24 hours post seeding and infecting, cells were transfected with 3 µl XtremeGeneHP + 6 µg pHM1-sel-pVV. Six days post transfection a lysate was prepared by two rounds of freeze-thawing (37 °C / -80 °C). Virus from this lysate was passaged in MDBK cells and single non-fluorescing foci were picked. This was done five times before a serial dilution was made in a 96-well plate and a single non-fluorescing focus was isolated for large scale preparation of the virus (Figure 9 and Figure 10).

### Construction of nLSDVSODis-UCT

nLSDVSODis-UCT is a recombinant virus based on the Neethling vaccine strain of LSDV (OBP, SA), with the SOD-homolog gene completely replaced by the novel SODis gene (SEQ ID NO:2), designed to be more stable than other poxvirus SOD-homolog genes by deleting a series of AT repeated nucleotides as described in the construction of nLSDVSODis-M. The SODis sequence (SEQ ID NO:2) is shown in Figure 6 and was used in the construction of nLSDVSODis-M. No marker genes are present in the recombinant virus nLSDVSODis-UCT.

For the construction of nLSDVSODis-UCT LT cells were infected with nLSDVΔSOD-M (expressing eGFP) at an MOI of 0.0001 and transfected with 6 µg of transfer vector pHM1-SODis (Figure 11). Cells were lysed by three cycles of freezing and thawing 48 hours post infection and transfection. The lysate was passaged in MDBK cells and three rounds of picking of non-fluorescing foci were performed. A single non-fluorescing focus was isolated on a 96-well plate and expanded in MDBK cells. PCR analysis confirmed the recombinant to be nLSDVSODis-UCT (Figure 12).

### EXAMPLE 2

### Genomic Sequencing of Herbivac, nLSDVΔSOD-UCT and nLSDVSODis-UCT

Full genomic sequencing has been performed on three LSDV genomes. Herbivac was provided by Deltamune^{®} (batch 008), which had been freeze-dried after growth in primary LT cells. nLSDVΔSOD-UCT and nLSDVSODis-UCT were constructed and grown in MDBK cells at UCT as described in Example 1. Virus was purified by centrifugation through a dextran (10 %) sucrose (36 %) gradient, DNA was extracted and sent to the Central Analytical Facility in Stellenbosch for whole genome sequencing using the Ion Torrent method. The genomic sequences for Herbivac, nLSDVΔSOD-UCT and nLSDVSODis-UCT are provided as SEQ ID NOs: 3, 4 and 5, respectively. Differences between the sequences are listed in Table 2.

Initially, Herbivac was sequenced with the aim of determining what difference in the genome could account for the improved immunogenicity over the Neethling vaccine, observed by Deltamune^{®}. One hundred percent of the genome was sequenced with 400x coverage. When assembled against the South African Neethling strain LSDV LW (Kara et al., 2003) only two single nucleotide changes (SNPs) and one deletion of 2bp were found. The SNPs are located in LSDV genes LW056 (hypothetical protein) and LW116 (RNA Polymerase subunit) (Figure 13). Both these SNPs result in conservative amino acid changes which are unlikely to affect the function of the expressed genes. The 2bp deletion is located in ORF131, encoding a SOD-homolog. In the South African Neethling strain LSDV LWthis SOD-homolog ORF (SEQ ID NO:15) is truncated relative to the more virulent LSDV Neethling 2490 strain (SEQ ID NO:13). The South African Neethling strain LSDV LW putative SOD-homolog protein is only 108 aa in length (SEQ ID NO:18) as opposed to 160 aa for the Herbivac SOD-homolog (SEQ ID NO:19) (Figure 14). The 2bp deletion in Herbivac (SEQ ID NO:16) relative to the South African Neethling strain LSDV LW (SEQ ID NO:15) appears to have created a "functional" SOD-homolog gene and this gene is now more similar to that of SOD-homolog from the more virulent Neethling 2490 strain which was sequenced by Tulman in 2001 (SEQ ID NO:13).

Based on this sequence analysis, a novel SOD-homolog gene was designed so as to remove the repeated AT binucleotides which render the gene unstable (SEQ ID NO:2). The novel gene is designated SODis (Superoxide dismutase homolog with improved stability). Figure 6 shows the SODis sequence within the transfer vector pHM2v3. The altered sequence can be seen in enlarged bold lettering.

nLSDV was engineered to remove the SOD-homolog completely (nLSDVΔSOD-UCT) and to replace the SOD-homolog with SODis (SEQ ID NO:2) (nLSDVSODis-UCT). In the process of making these potential LSDV vaccines/vaccine vectors, recombinant viruses were made containing marker genes (nLSDVΔSOD-M and nLSDVSODis-M). Whole genomic sequences have been determined for nLSDVΔSOD-UCT (SEQ ID NO:4) and nLSDVSODis-UCT (SEQ ID NO:5). The only significant differences noted between these sequences, Herbivac (batch 008) and the published NI-2490 Neethling vaccine sequence are in the open reading frame encoding the SOD-homolog. Table 2 lists the differences found between the genomic sequences.

**Table 2. Differences between LSDV genomic sequences determined (Herbivac, nLSDVΔSOD-UCT and nLSDVSODis-UCT) and the published sequences for the virulent NI-2490 LSDV and nLSDV LW vaccine strain.**

| **ORF** | **Variant locus** | **NI-2490 AF409137** | **nLSDV (LW) AF409138** | **Herbivac** | **nLSDVΔSOD- UCT** | **nLSDVSODis- UCT** | **Amino acid change** |
|---|---|---|---|---|---|---|---|
| 49 | 44568 | G | G | G | A | A | glu-lys |
| 56 | 49846 | T | C | T | T | T | thr-met |
| 71 | 61951 | G | G | G | A | A | no aa change |
| 116 | 108119 | C | T | C | C | C | ala-val |
| 130 | 118992 | G | A | A | G | G | thr-ala |
| 130 | 119015 | G | G | G | A/G mix | A/G mix | TAA-TAG stop |
| 131 | 119386 | TTA | TTA | T | SOD ORF deleted | SODis inserted | SOD gene differences |

### EXAMPLE 3

### Induction and inhibition of apoptosis by the SOD-homolog

The effect of the SOD-homolog on apoptosis was investigated. A monolayer of MDBK cells, in a 96-well plate, was infected, at an MOI of 1, with nLSDV, Herbivac, nLSDVΔSOD-M or nLSDVSODis-M. 24 hours post infection, apoptotic cell death was measured in the cell lysate using the Cell death detection ELISAPLUS assay (Roche Switzerland). All four LSDVs induced apoptosis in MDBK cells compared to uninifected cells. Herbivac and LSDVSODis-M showed a significant (p>0.001) increase in induction of apoptosis as compared to nLSDV and LSDVΔSOD-M (Figures 15).

The SOD-homolog of leporipoxviruses has been shown to inhibit apoptosis. It was hypothesized that the SODis gene would inhibit apoptosis in LSDV-infected cells. MDBK cells were infected with nLSDV, Herbivac, nLSDVΔSOD-M and nLSDVSODis-M and assayed for apoptosis in cells which were untreated and cells which were treated with camptothecin at 0.0001 mg/ml. Assays were performed 24 hours post infection using the Cell death detection ELISAPLUS assay (Roche, Switzerland) and results expressed as inhibition of camptothecin-induced apoptosis (Figure 16). Herbivac and LSDVSODis-M showed greater inhibition of apoptosis compared to nLSDV and LSDVΔSOD-M (p>0.001).

These experiments indicate that the full-length SOD homolog causes both activation of the extrinsic apoptotic pathway (figure 15) as well as inhibition of the intrinsic apoptotic pathway (Figure 16).

### EXAMPLE 4

### Inflammatory changes associated with the SOD-homolog gene on chick chorioallantoic membranes (CAMs)

The CAMs of seven day old fertilized chick embryos were either inoculated with PBS (negative control), or infected with nLSDV, nLSDVΔSOD-UCT or nLSDVSODis-UCT Five days post infection the membranes were harvested, washed, fixed with 4% buffered formalin, embedded, sectioned and stained with haematoxylin and eosin. For each virus-infected membrane five sections were evaluated under the light microscope and scored on a scale from 0 to 5 for inflammation (oedema, haemorrhage and infiltration with white blood cells) compared to the negative control (Figure 17). nLSDVΔSOD-UCT showed a significant (p>0.01) reduction in inflammatory changes compared to both nLSDV and nLSDVSODis-UCT.

### EXAMPLE 5

### Comparison of lesions produced by different LSDV vaccines in cows

A group of five Friesian calves, 6-12 months old, and LSDV seronegative, were each inoculated at different sites on the neck with a dose of 104 TCID50 of each of nLSDV, Herbivac, nLSDVΔSOD-UCT and nLSDVSODis-UCT. The animals were monitored daily for two weeks for temperature and lesion size. None of the animals developed serious complications, indicating the two vaccines nLSDVΔSOD-UCT and nLSDVSODis-UCT are safe for further field trials. Two of the five animals (Animals 166 and 82) developed lesions. Animal 166 developed a larger lesion to nLSDVΔSOD-UCT compared to the other vaccines. In both of the animals which formed lesions nLSDVSODis-UCT gave lesions smaller than those of nLSDV. This pilot experiment suggests that the SOD-homolog may contribute towards the restriction of LSDV infection in the bovine host, possibly by inducing greater immune cell infiltration to the site of infection.

### REFERENCES

Kara, P.D., Afonso, C.L., Wallace, D.B., Kutish, G.F., Abolnik,C., Lu, Z., Vreede, F.T., Taljaard, L.C., Zsak, A., Viljoen, G.J., Rock, D.L. 2003. Comparative sequence analysis of the South African vaccine strain and two virulent field isolates of lumpy skin disease virus. Arch. Virol. 148: 1335-1356.
Tulman, ER, Afonso, CL, Zsak, L, Kutish, GF & Rock, DL, 2001. Genome of Lumpy Skin Disease Virus. J Virol. 2001 Aug;75(15):7122-30

The following paragraphs are not claims, but represent preferred aspects and embodiments of the invention.
1. A stabilised SOD-homolog (SODis) gene of SEQ ID NO:2.
2. A recombinant virus comprising the SODis gene of paragraph 1.
3. The recombinant virus of paragraph 2, further comprising a marker gene selected from the group consisting of a guanine phosphoribosyltransferase gene, a green fluorescent protein gene, an enhanced green fluorescent protein gene and a red fluorescence protein gene.
4. A pharmaceutical composition, comprising:
   i. a recombinant virus of paragraph 2; and
   ii. a pharmaceutically acceptable diluent or excipient.
5. The pharmaceutical composition of paragraph 4, wherein the pharmaceutical composition is capable of eliciting a protective immune response in a subject against a disease caused by a microorganism.
6. The pharmaceutical composition of paragraph 5, wherein the microorganism is a virus from the viral genus *Capripoxviridae.*
7. The pharmaceutical composition of paragraph 5 or 6, wherein the disease is selected from the group consisting of lumpy skin disease, sheep pox, and goat pox.
8. The pharmaceutical composition of any one of paragraphs 5 to 7, wherein the subject is a ruminant.
9. The pharmaceutical composition of paragraph 8, wherein the ruminant is selected from the group consisting of cattle, sheep, goats, and wild ungulates.
10. The pharmaceutical composition of any one of paragraphs 4 to 9, which is formulated for intramuscular, intradermal, intravenous or subcutaneous administration.
11. A recombinant virus for use in a method of preventing a disease in a subject, wherein the recombinant virus comprises a SODis gene of SEQ ID NO:2 in its viral genome.
12. The recombinant virus for use of paragraph 11, wherein the disease is caused by a microorganism.
13. The recombinant virus for use of paragraph 12, wherein the microorganism is a virus from the viral genus *Capripoxviridae.*
14. The recombinant virus for use of any one of paragraphs 11 to 13, wherein the disease is selected from lumpy skin disease, sheep pox and goat pox.
15. The recombinant virus for use of any one of paragraphs 11 to 14, wherein the subject is a ruminant.
16. The recombinant virus for use of paragraph 15, wherein the ruminant is selected from the group consisting of cattle, sheep, goats and wild ungulates.
17. A recombinant lumpy skin disease virus (LSDV), wherein a gene encoding a SOD-homolog polypeptide has been inactivated by deletion from the viral genome.
18. The recombinant LSDV of paragraph 17, wherein the SOD-homolog polypeptide is selected from the group consisting of SEQ ID NO:1, SEQ ID NO:17, SEQ ID NO:18, and SEQ ID NO:19.
19. The recombinant LSDV of paragraph 17 or 18, further comprising a marker gene selected from the group consisting of a guanine phosphoribosyltransferase gene, a green fluorescent protein gene, an enhanced green fluorescent protein gene and a red fluorescence protein gene.
20. A pharmaceutical composition, comprising:
   i. a recombinant LSDV wherein a gene encoding a SOD-homolog polypeptide has been inactivated by deletion from the viral genome; and
   ii. a pharmaceutically acceptable diluent or excipient.
21. The pharmaceutical composition of paragraph 20, wherein the pharmaceutical composition is capable of eliciting a protective immune response in a subject against a disease caused by a microorganism.
22. The pharmaceutical composition of paragraph 21, wherein the microorganism is a virus from the viral genus *Capripoxviridae.*
23. The pharmaceutical composition of paragraph 21 or 22, wherein the disease is selected from the group consisting of lumpy skin disease, sheep pox, and goat pox.
24. The pharmaceutical composition of any one of paragraphs 21 to 23, wherein the subject is a ruminant.
25. The pharmaceutical composition of paragraph 24, wherein the ruminant is selected from the group consisting of cattle, sheep, goats, and wild ungulates.
26. The pharmaceutical composition of any one of paragraphs 20 to 25, which is formulated for intramuscular, intradermal, intravenous or subcutaneous administration.
27. A recombinant LSDV wherein a gene encoding a SOD-homolog polypeptide has been inactivated by deletion from the viral genome for use in a method of preventing a disease in a subject.
28. The recombinant LSDV for use of paragraph 27, wherein the disease is caused by a microorganism.
29. The recombinant LSDV for use of paragraph 28, wherein the microorganism is a virus from the viral genus *Capripoxviridae.*
30. The recombinant LSDV for use of any one of paragraphs 27 to 29, wherein the disease is selected from lumpy skin disease, sheep pox and goat pox.
31. The recombinant LSDV for use of any one of paragraphs 27 to 30, wherein the subject is a ruminant.
32. The recombinant LSDV for use of paragraph 31, wherein the ruminant is selected from the group consisting of cattle, sheep, goats and wild ungulates.

## Claims

1. A recombinant lumpy skin disease virus (LSDV), wherein a gene encoding a SOD-homolog polypeptide has been inactivated by deletion from the viral genome.

2. The recombinant LSDV of claim 1, wherein the SOD-homolog polypeptide is selected from the group consisting of SEQ ID NO:1, SEQ ID NO:17, SEQ ID NO:18, and SEQ ID NO:19.

3. The recombinant LSDV of claim 1 or 2, further comprising a marker gene selected from the group consisting of a guanine phosphoribosyltransferase gene, a green fluorescent protein gene, an enhanced green fluorescent protein gene and a red fluorescence protein gene.

4. A pharmaceutical composition, comprising:
i. a recombinant LSDV wherein a gene encoding a SOD-homolog polypeptide has been inactivated by deletion from the viral genome; and
ii. a pharmaceutically acceptable diluent or excipient.

5. The pharmaceutical composition of claim 4, wherein the pharmaceutical composition is capable of eliciting a protective immune response in a subject against a disease caused by a microorganism.

6. The pharmaceutical composition of claim 5, wherein the microorganism is a virus from the viral genus *Capripoxviridae.*

7. The pharmaceutical composition of claim 5 or 6, wherein the disease is selected from the group consisting of lumpy skin disease, sheep pox, and goat pox.

8. The pharmaceutical composition of any one of claims 5 to 7, wherein the subject is a ruminant.

9. The pharmaceutical composition of claim 8, wherein the ruminant is selected from the group consisting of cattle, sheep, goats, and wild ungulates.

10. The pharmaceutical composition of any one of claims 4 to 9, which is formulated for intramuscular, intradermal, intravenous or subcutaneous administration.

11. A recombinant LSDV wherein a gene encoding a SOD-homolog polypeptide has been inactivated by deletion from the viral genome for use in a method of preventing a disease in a subject.

12. The recombinant LSDV for use of claim 11, wherein the disease is caused by a microorganism.

13. The recombinant LSDV for use of claim 12, wherein the microorganism is a virus from the viral genus *Capripoxviridae.*

14. The recombinant LSDV for use of any one of claims 11 to 13, wherein the disease is selected from lumpy skin disease, sheep pox and goat pox.

15. The recombinant LSDV for use of any one of claims 11 to 14, wherein the subject is a ruminant, preferably wherein the ruminant is selected from the group consisting of cattle, sheep, goats and wild ungulates.


SEQUENCE LISTING

<110>  University of Cape Town

<120>  Stabilised Superoxide Dismutase (SOD) Homolog Gene and Uses
       Thereof

<130>  PA166012/PCT

<150>  GB 1808049.9
<151>  2018-05-17

<160>  27

<170>  PatentIn version 3.5

<210>  1
<211>  160
<212>  PRT
<213>  Artificial sequence

<220>
<223>  SODis amino acid sequence

<400>  1

Met Ile Arg Lys Tyr Tyr Glu Phe Asn Lys Thr Val Ser Lys Ala Val
1               5                   10                  15

Cys Val Leu Lys Gly Tyr Lys Leu His Gly Val Ile Asn Phe Asp Gln
            20                  25                  30

Leu Gln Asn Gly Ile Val Ile Ile Ser Gly Val Val Leu Gly Leu Pro
        35                  40                  45

Glu Gly Asn His Gly Leu His Val His Glu Phe Gly Asp Glu Thr Asn
    50                  55                  60

Gly Phe Leu Ser Met Gly Asn His Tyr Asn Pro Glu Asn Lys Lys His
65                  70                  75                  80

Gly Ser Pro Leu Ile Met Arg Gly Ile Leu Glu Ile Trp Glu Ile Phe
                85                  90                  95

Ile Gln Thr Asn Met Glu Tyr Leu Ile Tyr Ile Leu Asp Gly Lys Ile
            100                 105                 110

Ser Leu Val Gly Asp Tyr Ser Ile Ile Gly Arg Ser Leu Val Ile Ser
        115                 120                 125

Glu Lys Asn Asp Asp Leu Gly Lys Gly Tyr Asn Phe Lys Ser Phe Ile
    130                 135                 140

Asp Gly Asn Ser Gly Asn Gly Val Ala Tyr Gly Ile Ile Gly Ile Ala

145                 150                 155                 160

<210>  2
<211>  552
<212>  DNA
<213>  Artificial sequence

<220>
<223>  SODis nucleotide sequence

<400>  2
atgattagga aatattatga atttaataaa acagtttcaa aagcggtctg tgttttgaaa      60

ggatataaac ttcatggtgt tattaatttt gaccagttac aaaatggaat agtaattata     120

tcaggcgttg tacttggatt accggaaggt aatcatggat tacatgtaca cgagttcggt     180

gatgaaacta atgggttttt gagtatggga aatcattata atcctgaaaa taaaaaacat     240

ggtagccctt taataatgag aggcatattg gagatctggg aaatatttat tcaaacaaat     300

atggaatatc ttatctacat actcgatggt aaaatctctc ttgttggaga ttattctatt     360

ataggaagat cgttagttat tagtgagaaa aatgatgatt taggcaaagg atataacttt     420

aaaagcttta tagatggaaa ctctggaaac ggtgtcgcgt atggaattat cggaattgca     480

taatctattt ttttttaaat atataaatta taaaaatgac aaacacttcc ctccttttaa     540

gctacttttt ct                                                         552

<210>  3
<211>  32767
<212>  DNA
<213>  Lumpy skin disease virus

<400>  3
aagccaatta aacctaatta agtggaagcc aattaagtgg aagccaatta aacctaatta      60

agtggaagcc aattaaacct aattaagtgg aagccaatta agtggaagcc aattaaacct     120

gtaaatggat acttttttca ttcaatcttt taagtctaat cattagtgct gatttcacta     180

gcgaaatatc gttgtcggaa acgaggtttc gaagcaatac caacactttc acagaagaac     240

aagttggaga tgattcacca ccccaatatt ctgctgctct tgctaaaatg ccaatcactg     300

cacatgattc cctaatgtaa ttttcttttt tcaacatgga attaatcata atttttgatt     360

gttcaaatcc aattttagaa tccaaaaaca tgtttttgac aaaagctgtt agatcatttc     420

caaatacaag tgaggcatcc tttttgaaag attcaaaaac taagaacacc tttccagcaa     480

cctccctgga ggaaaatgcc aatgatgacc aaaacaaata atcagagatg gcggttgtga     540

tatcatcatc atctgaaaag ttgtttcggt agacatagtt gccggaagac atcttgtaaa     600

atgaacggga aaaaagtctc ttttaacccg aacttgttat ttcctgtgct tatttttcaa     660

aattattcaa ccatattcat catcctttca actaatcctt ttctaggtgg ttcctttatt     720

gtgttttgat tacagtagta ttcgtagtgt tttttaaacg gattaggaat tttaataaaa 780

tcaaaaactc cattatcatt tctaatattt atatcgcttg tatctttttt atctaataca 840

gttaatggca gatcattatt tatttcttta ctttctatag gtttgtgatt agtgtttata 900

ttgtcttgga ttttttcatc cttatccaag acagaatcga acggatttag gtttccaaac 960

atgaaggaga taagcttttg cattggaaac attaataatg aatacaaact atatataatt 1020

aaattacata atctagctat ataaaaaata cacaacatac aaactttagc taagctatta 1080

atgagtagtt gacaaaatcc ttttattgca tatatcacaa atcctaaaat ctttagtata 1140

agaactaatg tccctcttac acatctccat atactaaaga ttattttatg aattaatgca 1200

aaaatccacc agactaaaaa aaacggaaaa ctcctgtttc ttattcataa tttatatatg 1260

tgaaaaagaa caaaataaat attatatcgt cttttgtata acaatcctat agttcattat 1320

gatgaaatat ttttttaata taattatgct gatgttcata atattttttt ctatcaaatt 1380

taccaacact gcacgttttt aaataattta cgtgttcgct aaattttaca gctgagcaat 1440

ctattttatt attaaaccca aaaatctttt ttcctgtaga tatatatttt ttttcacatt 1500

catttctaac tataacactt gaacttacat gttttacaca actaatatct gttgttataa 1560

caaattctaa atcataatct ttaaaactta cactctcaac gtatgatttc atttctgtag 1620

ttacttgatc acaagaaatt gacaatgata ttctcttagc acactgtggc agcgaataca 1680

gagttgttat attactggtt gatatttcca tatatacata tttacccaat gaatagactg 1740

cttcgttaac agtatctaat ataaagttgt tttgtgtttc ttctttggaa caatcatcat 1800

gtgcgaaaat tttaaatcca tttccctctg ttagaagtcc tccaggtcct tgaattttat 1860

aacagccagt tttaatcgct ctaaaattct ctgtttggat acataatcct acttcgattt 1920

cccatgtgtt attttcttct tctgtgcatc tctttattga tattccgctt acactactag 1980

taaaaatgaa tagttgcaaa aagaaaatga acaaagttaa tgatgacata ttggttggtt 2040

gttttgacgt attaccgaca caaaccacct cgatactact gaactttttt ttctattatt 2100

aactaaaaaa acttgaaaac aaacctttct ttttttcctc tttcttaaaa ggatttatat 2160

atgatagaaa actgaaactt tttttctttt gcctaaaggg gtttatatta aacgaaaaca 2220

gattttttgt cgatgaggta atcatgttat aaggattcat taaccctatc attgcccaaa 2280

aaaacttcaa aaataattat atagagtagt atttaccacc aacatgaaaa caaacacaaa 2340

aataatacta ttctgttatg ttatttttct tagtttgtat gtatttagtt gtgtagtagc 2400

atcggctaaa aaatgtgacg acgttagctt tgattacata ttaaaagatt tacgttcaga 2460

atttagcaaa ataaaaagtt ttgttcaaga caatgatcaa gaaaatatga tgttattaag 2520

tcaatcaatg ctggacaagt tgacgagtcg cataggatgt aaatctttat cggatatgat 2580

aaaattttat ttaaatgatg ttttaccaaa tgcagaaaaa atagaacaca tgaaaaataa 2640

aataacttca ataggagaaa aattaaaatc gttaaaagaa aaactcatat cgtgtgattt 2700

tttacattgt gaaaatcatg acgaaataaa aacagttaaa acaattttta acaaattaaa 2760

agataaaggt atttataagg ctatgggaga gtttgatatt ttcattaatt acttagaaaa 2820

gtacatagtt aaaaagtaat caaatctata cttataaaac tacaacatta tagtttcttt 2880

ttatgtcata gttaatatta tctttgttta ttcttaagtt acatgtataa ataccactat 2940

catcatgtgt tgtgtttttt ataaccaact taagttcgtt actttgtatc ctatcatcgt 3000

atagcacttt gttattattt ttataccaat ctagtgtctt atttttaaaa taactactaa 3060

taaggtcaat atccatacaa tgtactgtcg aattaagtcc ttcattgaat ctgtaagtaa 3120

ttgtctcttc tttatccaaa aaagttaatt ttatagacat ttcttcacaa atattatgtg 3180

aacgaaatct acaaatataa attccgtcat cgctatttat aacagggtac aaccataagt 3240

tgtttcctat aagttgtacc cttgcgtttt cattatttac ttctattttt ttaaaatttt 3300

tgtaatcgtt ataatttctt acaaacgata gagagttagt ttgaaaccat gttatgtcat 3360

ataatgttat atttttttta aaaaaaatag attttttata attatccaat gggcatttta 3420

aaaatgccgg atctttgaaa tttacattca ctggagttag aataccgcct ctatatttac 3480

aatattctga taaaaatgcg ctttttaaac aacatattaa tatgataaaa tttataactt 3540

tcattttgtt gttgtgttat ttattttaaa tagaaaaata gcatcggact acgttaaaat 3600

ttatataatg tatttcagaa ttacagttcc cagttaatac ataataattt attttatctt 3660

ttaaattatt atttacgtac ttaactaagt cttttaaaaa atcaaaatcg ttttttgatt 3720

ttggatacca tatttcatca catgacgaat ctaaggcttt ccatgctata ttttttccat 3780

cttcgttttt taagtaatca aaaataatat cattataact aaatgatatt tccatacaca 3840

tatcgtcaaa attttcaaat attaattttt ccgttattat atcatcatac ttgtttacca 3900

aaacacattt acctgatcta tttaaaatta atccaaatgt atctacttct gtggaataat 3960

tatttacggt aacaagacag taacaaaact tcctatttcc ttcgtcttcg taaaagttta 4020

tatgattatt ttccgtcaaa tagggtaatt taaatatggt ttcgtcatta tttgaattat 4080

attttataac tacatttatt aatgctatat ttttttctcc atcagttata atatcagaac 4140

tgtgatttaa tgttttatca aataggacat cgtttttagt agaaaatata gtattgggtt 4200

ttatatgtat atttgtatta cctcctttgc tcgtattatt taaatacagt actaaatgat 4260

aacaataaca gttttttgat ttgaaatgta taaaatctct gtgtgtgttg aaaaaatctc 4320

ctttttcgta ctttataagc gtaattgtat tatctaatat aatatcgcta attttttgtg 4380

gaaattcaaa agataataac agtttttttc ttataacttt aaaaatatca tctaaacctc 4440

ttattatata ctgtttacat tttctttcat tagtattaat aacttctata cctttttcat 4500

gatcaaatac tttagactct tcccatatta tgtttttgta tttaatcatg tccataatta 4560

tttttttaac gttattaaaa taatcatcat taaaggtttt tacttttatt ataccatcac 4620

taatcgtttc catcttcaaa aaatattatt tatatttaat attttttaaa aactaatgtt 4680

aaggggtttt tttttatgtt ttataaaaat agtatgttaa ttcaattcat tgacgatata 4740

ctcaaaaagc gataacacaa gattgtaaat tcctacaatt ttattaaatg atttttctcc 4800

tttgaattta tttttagtaa ctttctgaat tcgccgttta agcaaactta aatctgtttt 4860

ttgttttaca agaaatgtat caactttcct aggcgttaat tcgtcaatac atgctttact 4920

tgtttcattt gtccttattt gataataatc agtttcgtaa tttccaaaaa ccgtaacaca 4980

aactttttct tgagaaccaa attcaatagc acatcccttt ttatcacaaa attgttcatc 5040

aacttcatat ttaattgtat ttgaatcacc aaacgtaata cttaaaatat atttaaacac 5100

aatattacaa tacttatcgt tattatatat aggagaattt ttattattat ccttggctag 5160

tgggtgatta atcgttagta cagtattaac atcttgtcgt ttaataaaca cagatggagt 5220

agtaataaca acactttcac aaattggttt aaacgtaaaa acgttcgatt ctgtattatc 5280

gactgtaata aattttatcc aaaaatcgtc agtattatca tctataaaag gtgaaacatt 5340

acaatatttt tgaatagtat aattacatgc ttgtttccat tctccactgc tataagtcat 5400

aatcattacc ctatagttac tatcgctgac atcgttagtc cactcgattt tagaattaaa 5460

atcattagat gttagtttta tatctttagg aaaagaataa catagtttaa agagcaaaaa 5520

agcaaaaaat actactgcgc ccatttttta gtaagtattt agttatattt tatatttttt 5580

atggaaatct atcacagata tatttttcaa attcttttct cttaaaatga cttatttcgt 5640

caaaaaatat gtaaaagctt ggatacttac atgtatcatg tgaagaaatt attgatatta 5700

aacctattat atatttcatt gtggttatgt caatcacatt attttcattt ttttcataaa 5760

gcgaaaaaca gtaacgtgtt ttacttaaac gcaattttga tattatttta ttaatatatt 5820

caacaccgta gtgattaacg gccatagcat catatgttaa aaactcagca tatattttac 5880

ctattttttt tgtatttttc atttttcgta ccataaaata attttccatc atatcgttaa 5940

aatttacttt tttcatggaa ttaatatgct taaaactttt agattttata aatggataaa 6000

aatcagcata gtattcacca tcaattttta attcttttat aactttttta taatcatttt 6060

cattaagtac atattctgat ataactttta tcatagtaaa cgtaaattta tttgaatttt 6120

ttaatacaat cttcgttatg tcatccttcg tataatcata atttgaaaga taaaaaactt 6180

tatttaaaaa atattttacc tttttttcat tgatagaaaa aagaaaggtt atatacttag 6240

taaagatttt aaatttattt tttgtataca taatgcataa cgattaacta tttttaaaaa 6300

gtgtaatatt ttttttcatt tttatatata ctcataaacc tgatatgttg tgttatcact 6360

cttcgcagca agataataat cgtacaatgc tattagtata tgtactacag ttataaatcc 6420

cacaacacaa ggaagggaat atgccatcgc ggatattagt gcaataagat cacgattttc 6480

aatattttct aacatacttt tcaaattaaa cttaactaaa agtatggtta gtggtaaaag 6540

tagtaaacat attaaagaaa cgcaaatagc tggcacgtta caatacataa acgaacattt 6600

ccatcttaaa caactctttt tatttttttt aatattgtat tttccattac atattttaca 6660

ttttgtatta tgtgaaaaat taatccattc ttctaaacag tttttatgaa ctatcttaaa 6720

ttcattcttg caattacaaa agtttgtact tacattatat tcatctttac aaatccaaca 6780

atgagtgttg gtattatcac tcccttccat ttttataaaa tatcattatt tgttgttatt 6840

atttttttat tttttatcca atgctaatac taccagcact actagtgcta cgcaatcgta 6900

aaagcttttt agtaaattct ctactacaaa acgcataaat tagtggatta ataaaacaat 6960

gacatagaga cacaatttca gctacatgaa ctgcaaggtt gacaaatcgt aatgccgtac 7020

atccactaaa aacatttaac aaatacaacg atgaaacaaa tacagttaca ctaaatggga 7080

gtaaaaacaa tactgaacag ataacaatca aaaacaccat ctttatggct ttcttattct 7140

ttgtttgcga ggtttttaaa gtatttaaga ttttatagta acaatatagc aaaataatta 7200

gcggtataat cattccaaat atgtttattt caaaatttat aaataatttc caaatttttg 7260

cattatcgtt ataaaataca tgacaatacg ttattccata tacttttttt gtttcataaa 7320

ataacattat tggaaaggat tcaatagttg aaacaatcca aaccaccata ctaagtacaa 7380

ttccatatcg ttttgtcctt atcggcattg attttactgg gtgaactaca gctaggtatc 7440

tatcgatact catcaatgtt ataaatgaca tactattgta aaaaccaaca aagtaaaaca 7500

tagctttgaa tttacacaaa caatctccta aactccattg tttagcgata ctatcgtata 7560

aattaaaagg aaacaccaac acgaaaatta aatcagacaa tgtcagatta agcaaaaata 7620

tatcctgtat tgtttttatc ttatatttac gaagaacagt taacacaatt atatttccaa 7680

ataatccaag aaagaatata gtcgaatata aagtaatcag tccaaaactt gtagtatcca 7740

caccatcatc acaatgtggg atatcgacta tattcacttc ataatcatca taatcatcgc 7800

tataataagt tgtattatat gcggttgtat aattagatat tgttgtcgta ttttcataag 7860

ttgaaggcgt tgtaacatta ttttgatttg ttgaaattgt actgagaatt gtgctaataa 7920

ttgtagtagc tatagtggta atattactgc tactattata catggtggcg ctactaactg 7980

tgctaagagt ataattcatt tttgttggag ttgtgcttta cttaatcata cacttttatt 8040

ttattttttt tttttaataa aaacacagta ttattgtatc atcatttatt tttttaaaaa 8100

aactttttat tgtttgtcaa aaaaggtgag atttctaaat ttaattaaat ccttacaatt 8160

gtaacctgac gtattagttt catcaatatc aataccaaga ctagttagat aatcaaatat 8220

ctttttatca gaatgaaaca aaatataact atacaatata ttattattat caaaatcttt 8280

ttgcaaaaaa ttaactccac tatctattag taactttatt acatttatac gaacattaaa 8340

attacacatg tacatgtgta acaaattttt cccattttca tcttcttctg ttacagatga 8400

tccagaatca attaatattt taactatttc aggttcgaca ttcttgttaa aagaaagata 8460

atgagctagt gctgataaat tattatctct agttttaaaa tttatatcgg atccattttc 8520

tatgagaaat tttaatattt caacattaac tttatctttt tcgacacacg aaaacaatgg 8580

cgtttcatat aagtcgttac aatcattaac aaattttatc catttcttta ctccatttat 8640

atcatcttgt tcaacgtaat aaaataatgg attacaatat ctataaaata agtaatctgt 8700

aaagtcatta tcataatcac tacataattt ttccttttcc atagtaataa ttgatgatgg 8760

caaactagct tattaataaa aagttatatt tcatttttgt aaagtgtttt taaatttaca 8820

tatttaattt tttttgtgag atagttgtat tctaagttat gtaagttaca tgtgaaatta 8880

gcagatagat attttctttt aacttctttt ataattaatg caattttata agagtttcct 8940

acgtacattt ctctacctaa gtaaatattt ttacttctat cgtaatcaag tacattaact 9000

ccgttaatta accaaaatat tgaacttcta taaacacgtt gtatgttttt agattctcca 9060

atacattcca tcaaaaagtt tttacctaaa gctattgtac tatttaaaaa atttactgtt 9120

aaatttatat catctttaaa tcttttttta attacgttat tatgataata acgttttaat 9180

gccaagtttt ttggattaca ccgtgtagca gtagttatgg aacataaata tactccttca 9240

tcactcttag aaatatttcg tattagtaat ttttctttgc tttttttata tttaaaatta 9300

ttgtttattt tttccctgtc tttaaaccaa tttacttcat aagaacaatt aaacgtattt 9360

ataaagtttt ttgtatttat acacaacaac tcatagtttg taccaatgac agagtttata 9420

gtctcttttt tataaatatc taacataaca gatgaagtaa aataatatgt tgagttatgt 9480

acattacatg catatattcc agaatcattt tttgttatat tatttatcca caggctatgt 9540

ccaacagaat atactctatc ggtatttttg aactcatgaa aagatttttt tacatcccct 9600

gttttaacgt attcattgta attaactaaa aaccatttta ctgttgtatt ataatttaaa 9660

gtatcaacat atggacattt tactatttct gagttatttt caaacgtttt catcacggat 9720

tgtagataac caaagcttaa ttttgtaaaa gcaagaattt taacattgat aatcagtaaa 9780

tagaaaataa atgtcgttac tttttccatc ttatccaata agattttaat cttacaagat 9840

ttaaaaaaaa atattttttt ttttaatttt tagttgttat tataaacttt tacatcaata 9900

tagcccttta ttttgttcat ccttataatt tgcacattaa ttaatttatt aactaatgtt 9960

ttttttatat tattcatttg atccatgttt atattaacat aatctattac aagtgcttcc 10020

aaaccatagt tgattagttt tacatataca atgttatctt ttataaacac aataccatct 10080

gttacttcat taatgttagg taaaacgtaa caaaatgcca aatcgctatt cgatgacatt 10140

tttgctctca ctttctgaaa cttttttttc agaaataccg tttttgtgct cgcatgtatt 10200

atttaaacaa ttccatatat cgttcaatat taaagttttt ttaattggtt cctccaaatc 10260

cattagaaca catgttaagt tagtattttt aatttcttca gttacgtttt taattatcaa 10320

atcagatcta ggttctttgc cacattttaa aggttttttg ttaaaataca ttcgttcttt 10380

ataaatgtcg ccgttcataa attcaacaaa cgaattatta tttcctatca gccaatacac 10440

atatgcataa ttgctatgat gactatatcc tttacattgt agtaaaacgt tttcgccctc 10500

tttatgagga aagtaaataa ctaaatccct tttttttgca catttactat aaaccacata 10560

aaaggttgta aataatgtca gacaaatgac tactttcttg aaagagatca ttttttattt 10620

tttagaacta tattttatat ttttttttaa aaataatgaa acaaacttct taaatgacat 10680

ctaacgccct catatcctaa tttgcatctg caaaacatta gtggtttttt attgtaagat 10740

aaaataaaag ttgttttata acatgtacca ccatttaaac aaaaatttat acttttatca 10800

cacatcatta tatctttgtt taagatagtt atgtaatcgt ttttattttc tttagaaagc 10860

attaatataa ttttatagaa taacaaaacg gtaaaaaata ttttttttac ttccatcgta 10920

tcctaccaat tattttaagc aaataatatg taataataat acttcctatc attacgaaaa 10980

agttaattat tgaaaatata aaatcatttt gttcatcgat ttttttattt ttttcaacaa 11040

aacttataat gtctttttga taactagaaa atgtatttat tagctcattt agtgctaaat 11100

catcattaac aacttcattg ttttcacttt ttttcttttt tatgataatg gataacaacg 11160

atatagaagc taatattact gaaggtctaa aatcactatt tatagtttcg ataattatat 11220

tttttacatc atttatatca taacaagatt taacgttatt gtgtaaaata aaattacata 11280

tttcattaaa ctcagtttta taatcttttt ttattacctc acagcattcg cgtatcatta 11340

ttaaaattgc caactcatta ttatttaatg attctattct taagtctcgc aagtacacat 11400

tcaaaacttt ttctatgttg taattgctgg tatccatttt tacaaaaagt aacttatatt 11460

ttcctttatt ataaaaaact ataatattcc acttgaccca aatccattat tatctctatt 11520

tgtaccatct aaacatttaa cttctttaat ttttggatat tctacttttt caaaaataat 11580

ttgcgccact cgatcgcctc ttttaatatt aaatgtattt ttaccattat taataaatat 11640

aactccaatg ttccccctat aatcttcatc tataactcct cctcctatat ctatgcaata 11700

atataacgaa agaccagacc ttggcgctat tcgtccataa catttttttg gaatagataa 11760

actgatatcg gttttaacta aaattctttc ttttgattta acgatataat cataagcact 11820

atataaatca tatcctgcag aaaaatctgt tgatttggta ggcagtttag caaaagcaga 11880

caaacgtata cagttaacga cagacgaact atgatccatt ttgttatttt ttaaaattag 11940

gatatttttt ttttgttttt ttaaaaaaca ctatctatgt ctaatgctcc tataacgttc 12000

catccttttg ttttatcgtc aaatatttct atttcacgta tatatctttc ataatgaaaa 12060

cctccaacga tcataataca atcatcaaaa atacatattg atgaattaaa tcttggctga 12120

ttcagacacg actcaattct ccatttacaa gacataggat taaagctttc gaccatgtta 12180

aaaactttta tattattgat atatgaaatt ccgccaacaa catatattaa accatgatgg 12240

tatatagcgc atccgccata atgagaatat tttgtacact cacccaatga ccatttatta 12300

tatttcaaat tatacacttc tactgattta tcatattcag atattcctcc aataacatag 12360

atggtatcat tgacattaac aacacatgga ttgtatctag gatatagtaa atttggttct 12420

tctctccaca cactttctcc aaatgaccaa ctttcaactt tattaactat acaattatta 12480

tagattccac caataacata tattctatta ttaaaataag tggcacctgg gcattttctt 12540

ggataaatta atgaaggtaa gttttctctt tcaaaagttt ttacattgat tgcgaataca 12600

ctatttactg atcgtaagta tttatccaca ccccctaaaa agtaaatatt attaccaata 12660

gcaattgaag tacaaaactt aaaattttca tctattatgt tgcttaattt acttaagtta 12720

ctgggttgtt gaatactttc atttaatgtt ttgttttcat caatttcatt acaattgttt 12780

tcatacaata gttcattatc ataaacactt tcatctatta tatttatatc cattatatta 12840

tttgtaaaca gtatttttgt taagttatca ttataaacct taaacttaaa agactttgta 12900

aatttgtcac tattaataaa ttctttattt ttatagttac atcgtggtat gtgtttatca 12960

ctatttttgt taaaatcata aggttttttt ttaaatctgg acgaccacgc ttgtaacctt 13020

ttttttcctt tttctgttaa aaaggtaaat cttacagcgt catttactaa cttcatcgta 13080

ttaagcctat tagttttttt gacagttccc cacttaatta taaaattaag aacaaaatct 13140

tcggcatcga caactaaatc actacttttt aatatgggcc ttaattcatt aactcttaat 13200

ttagttaaat cgttgcttat atacataaac ctttttttaa tatatttatc tgcaacatcg 13260

tacacatcgt aacaaccata catataagat cttctaagat atcaacacaa ttactatcat 13320

caatgtaatt tatcataaaa tcagtacatg catccattat aaacttaatt tcgattacgt 13380

tagaaattga aaaaatgtca acaacatttt tgctattcaa tgtaatataa ccagtattaa 13440

tatactctaa cacatacgta aatggcttat atttaaaagg taacttaact tcgttactgt 13500

attttttaat agctttttta aagtaattag atgatatctc gaaaatatct ttccttgttt 13560

gtgatatacc tccaattgta aatattaaaa ttctatcata atcatttttt aataacatgt 13620

actttagttc ttctacatat tctttattga tatacctttt taaagtcata ttggttattc 13680

aagataatta acaagagttg ttatttattc aactttataa tattcaataa aaactaaaaa 13740

tcaacatcta acgaaaaaac attatcgtct ttgttagaaa taacgcccat tttttgatat 13800

tcactaactc ttttttcaaa gaagttagtt ttaccttcca gtgatatatt ttccataaaa 13860

tcaaatggat ttgttgacat aaacacttta tcacaaccta gttctaatag taatctatca 13920

gcaataaact caatatattg agacattaaa acacaattca tacctattag ttttacagga 13980

attatattgg ttaaaaattc tttttctata gacacagcct cgtttataat aatcgtaata 14040
atgtcattag aaggaggata ttttagatgt ttaaaaatta aacacgcaaa atcacaatga 14100
aggccctcat ctctactaat aagttcgttt gaaaatgtta atcctggcat caatcctcgt 14160
ttttttaacc aaaatattgc agcgaatgat ccggaaaaga atattccctc aactgcagca 14220
aacgctatta atcgttctcc aaacgatgct tttttgtttg atatccattt tctagcccat 14280
tcagattttt ttttaataaa atccattgtt tcaatagcat taaataaatg ttccttctct 14340
ttagtacttg aaatgtatgt atcaataagt aagctataca tttcagagtg tatgttttcc 14400
atagctatct gaaaaccata aaaacatcta gcttctgacc attgtacctc cgagtaaaat 14460
ctttcggcca aattttcatt aactatacca tcactagccg caaaaaatgc caatatgttt 14520
ttaataaagt attgctcttc attgttgagt tttttccaat cggctaaatc cttagaaaga 14580
tctacttcat caactgtcca aaaactagcg acagcttttt tatacattac ccatatttct 14640
ttatataata taggaaaaac tacaaatctt gaagatgttt ctttaagaat tggttccatt 14700
ttattttata ccataaaaat tatatttcat tattttattt agtcaacatt gacgcccaca 14760
cacttatcat cgatataata aacattttct aattcgtcaa aggatcttct tgtggttaat 14820
ttcatcattc ttacttttat tttattaaac aaatcgaata agtaaaagca gttaaccagt 14880
tgaaatagca aataaagact tattacgata acaattatta aaaccatcaa aattagtaca 14940
gttcctatgc taaaggcgtt attgttaggc attgctgttg taagattcat ctgatgcatt 15000
tgctaccact attgtaaaat ttatactttt ttttattttt tatagaaata catcatcatt 15060
tgaaacactg tcgtcataat ttatgtaata cattacttta tgaggtgtct ttctttttgt 15120
aaatttatac ttgtagttat aattatattt agtatcatta tcatcactac taacggtatc 15180
actatcacta ttactattac tatcattagc accattacca tcaccatcac catctaacaa 15240
tgtaacacgt tcaatagata ttgccgtttt catattgctt ttccttataa aactaacaca 15300
tttaatagcc ttttctttta tacttttttt taataatctg taaccaaata ctataccaca 15360
ataaattttt ttatccattt ttcctcactt actatcaata acgtataact tagtttattt 15420
tttttgtttt ttaaccttaa ataatatatt aatttattta gataaaaata tttatttatt 15480
tttttttgac gattttatac atccttcata gtataaaagt atatagttat agtagtatta 15540
tgtacttttt gcagtgtaaa ctacttatgt gcaatcaaaa gttgaaagta aacataagag 15600
tcacatttta tgtatcataa aacgaagtat ttgttgttat taccattgga taatcgttat 15660
gcaggtaggt ttctttttta tcattgtgag atgatggttt tttaatattt aaacaatatc 15720
catatttttt aatttcgtct ttagatgggg tagagttact actattgctg ttatatggct 15780
ttccatttac aacaactttg ttaacttttt ttagtttttc gctcatgttt tatttaataa 15840

gtatttattt atgttattag ctatgattta tgtttcattt ttaatgattt tttaaaaaaa 15900

attatacata taaaaatgaa ccgtatttgt acttaatgtt tatttttcgt tttatagaac 15960

ataaaccaag taaaaaaata ataaaaatta tcaatacaaa aaaaataaac caatgaacat 16020

cgttgatgaa tggtatccta ttatcgatat aattattaat tgtatatcta ttactcattg 16080

atttcattat tgtattcata acacaatttg cttcagaagt tccagaatta attatttgta 16140

ataaaatgtt ttttccattt ggtgcgatac actctccaat atcgaaagtt tgaatatcaa 16200

tcacattagt tacggcggcc gaagaagtac atttgcgtat tatttcgggt atataatttt 16260

tatcttgatt atccaaatca atcccaactt cattcgctaa ttcatacttc tcgttttgag 16320

atagagtatt agcaaactct ttaaatgttt ttatcaataa gtaaaaactt agttctttat 16380

catcaataca taaatttatt atttttaatt tacacccttt aagcgttccc tttatttcac 16440

taatgtgaat accacaagtt gtgttaactg gaacggagta taatgataaa ttttgtaaat 16500

agcgctctat gaataaatta tacaatgtat caacttgaaa gtggttttcc atttatccac 16560

gttgagaaaa tgtttttctt tgaaataatt ttttctaaaa aagaaatagg atgtaatatt 16620

gatattttta acctaaattt atcacacgta tttttattac aacatataat aaattcttct 16680

aatgtattat aaaattcttt atctgtttct atttctggat atgtttttaa aagtgtatga 16740

atgaaaaaat gaaaatcgta gtaccaatta tgttcaactt ttatattatt ttttattttt 16800

ttatttatta tgttcgcaac ctgtgaaaag tcaaaatcat ttaaacaagc ttttataggt 16860

tcattaaaaa tataagtatt ttcatttaat gatattttta ttgattcgtc cgaatcaaaa 16920

attaaaatat tatcaggttt taaatcaaca tgtaaaaagt tgttacagca tggtaattca 16980

tatattttaa catataaaag agccatctgt aaaaaaataa attttatata ctcaactaat 17040

gacttaaaac ctaattcaat agccattttt tcactaactt tatcagaaga gaattttgcc 17100

aaaggaaata taataatatt tcctctatcg tataaataat tagaccgttt ttcgtgttca 17160

aaaaaatgaa acatgtgtgt aaaatagttt attacattta cattactttg tataactata 17220

gggtaaaaat atgataatag ttttacaaat tttacatctc cttttttctc attaaacgat 17280

tttaaaaaat atttatgtga aaaattactt atacttaatt tttgatcatc cattgtttga 17340

ataagtaata atatcatata tagtacccgc ttatataagt tatataaaaa tgtaagttta 17400

aagtttacac ccattgccca cgcacacact attagttttt tttcatctcc ctttaagtta 17460

ttaaacaaga actttggtat tgtatattct gcagttgttt caataggact atactgttta 17520

ttaggttcat aaacaaattt tacaacatac ttatcaattt taaaaactat accatatcct 17580

ccagttgaaa tatgataaaa gtcatcatta attggataaa atctcttatc tttttgctga 17640

aaaaacgatg gattgatata ttcgttttca gcaattttat ttaatgtttc ttttgtaaac 17700

tttttaaagt atcttattaa cctaacatta ggtgaccaac tttggtaaat atctaattga 17760

gatattacat aatcaaaata aatatcatcg cctaatactg ttgtagattt tatatcttca 17820

tcgttatccc attggcattc taatgagtta tcatctattt ccatgattac catttattgt 17880

ataaaaagcg tttattataa tattatagtt aataataata ttttacttta tttattatat 17940

agtcgttaat caattgataa tgttttatat tataatctaa aagtttgtct atttttaaaa 18000

ataaatttgt gattttttcc caaagggttt catcatcgtc gatcttgtac ttttttttga 18060

aactatacaa aaagttatta tttttattta aaatagtatc ataaataaca aagttgttag 18120

gaatataaca attattttct ttttccatta ccttttttaa ctttataatt ttattaacta 18180

attcgattat ttttaatctt ctaattgaaa taatatttga cgttatgttt aatgtttctt 18240

cattattctt ttcataaaaa aatgcttttt ctgtaattga taattcttta ggagataaga 18300

ttgtcactaa ataaattgat tcgccaccta tttgtggtaa aaaattaacc ccattaagaa 18360

agccactcat aataaaatct ccttcttcta tatttatttt taaaaatgta gtatctggaa 18420

taaataatcc agatattcct ttattcttaa cacaggccgt aatgacaacg ctattactaa 18480

atgttgattc gtttaaaaaa aatgcatttc cgtgttttaa tttaaccaaa tacgatccta 18540

taactgtaaa aaactcttca ttgagtaaaa gtttataaaa aatatttaca tttatttctt 18600

tcaatgaata aaatcctact ttttttttac tcatatctaa gtcatttaaa aaaagattaa 18660

aagaaaaata taactcttca cttttacttt ttaaaaaatt accaactgca acattcccac 18720

cattaactga atataaatca tcataacatc tattatttaa aaagtacttc aaaatatatt 18780

cttctttttt tttattactt aaacacataa tagacattat cgtatttata aatcatatat 18840

catttcatat aatgcatttt tatctttttt aaaatcgtcc gatatatata tttctttata 18900

attatacatt aacacatagt ctcctaaaaa atgactatca attttttgta ttaaagaatg 18960

aatattgaat ctacagtaac taatagaaaa tgctttaaat aataataaat aattatttaa 19020

tgtagtatta ttttcataat gctctagtaa ttcgttatta tttactatat agtctacgat 19080

atcatcgctt aatatttctt ttatatgatc aatagttttc ataaaatcgt cgttatgaaa 19140

tgtaatactg ttttcccatt ctaacacgcc taactccttt attttatatc cgcctatttt 19200

tctcattaat tttatatcta ttgaggtcaa tggagtaaaa aatacagatg tttctaattc 19260

atcatcgatt ataggaatct ttacgttatt atttttactc actttacata atgctatata 19320

taaattgtta ttatttatat ctatacttct tttatagatg gggaaaccaa tagggtatcg 19380

ccctaaactt attatatcta atatggattt aactttaaat ataacaaatt tttttttagg 19440

agaataattg accgttttaa catttttaaa ccttattaac tgcataataa atcgtacagt 19500

gtcgtgttct agtggataat acataaaatt gtttacttta gatagtgatg tttgtattat 19560

tttagttaaa aaaatatctt ctattctatt aaaagaaaac atattaaaag gatatattaa 19620

attatttaaa acatcatatt gtttgttgat ggaatcatat aataccaaaa ctgactctaa 19680
tgccattttt tctaatgctt ttattttaca ttttgcttct tcttccgtga taaaatcttt 19740
tggtataatt atcttttttt tattattcca ctgtttaaaa taatcactta tttccatttt 19800
acacgaaaag ttagcgatat ctactaatat tagtttgaat ccgtcatctg atacaatata 19860
atcatttcct ataaaattcc atcctatgtt ttttggccac gacgctttaa gcagtaaaaa 19920
gtcaaaaacg ttgcttaaaa accctactaa aaatgctgtt ttaatattta ttagtattgt 19980
ttcctttaac cattttaaaa aacataatat taactctttt gagttaaaat ttataaatga 20040
tttaccaaaa cttgtcatca tataaattga tggacctaat ggagttatta ttgagtttat 20100
aattataggt attattgtaa ctacttcatc atatatgtta ttttttgacc ataacctttt 20160
cgatgcaata cttttaacat tataaggcaa tatgtctagt tccggtctat tatatttttt 20220
tttttgttta acccatatat aatgttttcc gatgcaccct attatcattt catcaattaa 20280
tgtataatca tcattacctt ttttactatt tttataatta acaaatctat aattagataa 20340
tccattatcg cacatccaat aatttatctc actcaatgaa atatttttat tttcaaaaac 20400
atgcaattta tgaacagatt tatttatttt atcgttttct aaagctcttc ttttgacaat 20460
atctaccaaa ggagatatag atgttttagg tgaaaaaaat gtttctgtgt cgacttcgtc 20520
aatgaataac ttgtgcatat gcatgtcaat aaaatatggt tctacatact ttgtaattcc 20580
aactatttct aaatgagata gttttgttac agatcgcact aaatctaaat tatttaggta 20640
tattataact ccctttccgc ttcctaatct cgctaatata atagagtgtt tatatgtttc 20700
taatgcatta ataatattaa attcattgtt ttgtttttta aacatgtttg attacagcac 20760
tgtatttttt ttgtctgacc aatctctttc aaatatattt gacaacatct taactaattc 20820
attatcgttg gtattaaaac taacaaacgc atagtgtaaa taatgtgttc cgtcaaaatt 20880
tgcaggagtt atatgtgcaa attcgttatc aataatcatc aacttggtat tattagtatc 20940
atgaaaaatt ttaacagtta aatcattgtt actacacaaa gattgtaaac ttttaaccga 21000
tgacataaca taaacgtcat tttatcccca agatccaacc aaaagcctaa cttttacacc 21060
tcgattgata gttgcactga tgatagcatt gtaaatatct ggccaaaatg ttgtttttaa 21120
atcttctctt ataacgggaa ccaacgataa caattccaaa tctattgatt tttttgctga 21180
attaattttg ctcaacacaa catccgcatc taaagttcta gaatatccta atatatgatc 21240
aggagaatct gacaaaaaca caccaccaat tggattatta atatggtatt ttgtactcac 21300
tggcaaacaa catgttatat atattcctct aaaaatactg ctgttgttat ttaaagcttt 21360
aaatgtttca aaccttcttt gtaaatctaa agctaatggc gcgtaagtag aatatatacc 21420
taaagtcttt atattggaaa tggatccacc agttaaagag gtgttaccta tatagcattt 21480
attataatca gaaacccaaa aacttcccaa taatactccc ggatcgttat ctcctcctat 21540

ttttactttg acatactcaa ttttatttga tattaattcc tcttcatcct tgttaccgct 21600

ttgatgatct actaaaattg taactttaat tccagatgct gcggcttctt ttaatttatt 21660

taaaatattt tttccatgtt cgctagtgcg aagattacaa caaaatgatg ctatatttat 21720

atttttttta gcttgtgaaa taatttcatc aaagcactca tgtgtgagca tatgttgtgt 21780

tgttatccct gaggttttag gcaatgtttc aacgagtcta catcctgctc cagatggagg 21840

ttttgaaaaa aataaggacc acatttaatg ctaaaaaaaa aatagttatt ttataacaat 21900

gtttgttttt cttcaatatg tttgtagtta ttatctttat ttatttcttt aaacataata 21960

actaaatcta aaattattga cactataccc actaatgcaa aaatagcacc aataaagacc 22020

aaaaaaaatt caaacatcat ttaatagaca tgttatttat ttaaattatt ctatttgaaa 22080

ctttgaagac caaaaatgtt ttattcttaa cccaattatt aaatatttta agtcaaaagt 22140

tggtacaaat atatttatat ttaaactatt atcatcttta ttaatgatac aatatttaca 22200

tattgttttg ttttttgttg cctcgtcaat acattttaaa acaaaacttg gtaataagta 22260

ttgacattga ggttgttgaa aatgaaatgg tgaatatagt aactcagtta atgtaaacct 22320

ataacctttt ataataacag aatctgatgt agctacggaa tttgtaggaa agtgttttac 22380

tgtatccatc ttgacaaagc atctatttcc aagtaagcaa ttatcatctt tattgatttt 22440

tattctgtcc atatttctaa atggacttaa tttaattatt tcatctaact tgttaacatt 22500

catggttgaa aatgttaatg atgttagatt tatcttaaaa aaaacgaatg ctttttcatt 22560

tttgatcgca tacacttcta cactgttata tttttaaagg atgtacatat atcgtttaca 22620

taatggatgt tatctaaatc ttttatttta tttacaaatt tattataata ataatttttt 22680

tcctttatat tttttttact ttttcttgga tgcaatgctt tccttaacca taacgaatca 22740

caattaattg catccattaa tattttaaaa gtttcaaagt tatcgtaaat aaatttcttt 22800

atgatatcat cactacatgt attcatatta attaaaacaa acaaatcttt gagctttttt 22860

cttgtattaa ttttgctatc aagattaata tgtctaaatg atggggttat cccatcaaac 22920

aataaattat ctttaacata atatatcgtt aaggatttat ttctttttct cattaaaaag 22980

ttatataaat gatcatttga aacagatagt tgttttttct ggtaaaatac caatatatca 23040

attttattat catgagatat acactcccat ctttttgaag atataacttt ttcatctata 23100

taccctacct cttttagctc aatttttata gatgaattta ttaaattgca atagtttcta 23160

catattttta tttgataatc tatagatgta tcaaataacg atattaaact tgttactata 23220

gctgcatttt ttaacatttt ttaaaaaaca aaggtgattt tttatacttt tttataatag 23280

tttttcattt atgttatagt aatataaatg gcagcatata attcacaata ttatgcaaac 23340

acaccatttt atataacgac aaaagaagga aaatatcttg tattaaaagc aattaaagtt 23400

tgcgatataa gaacagttga atgtgatggc gataaagcat catgtatttt aaaagtggaa 23460

aaatcaaatc aaacttgtga caggccatct tccccatgtg aacgagctgc aagagcttca 23520

tccccaaata gagggaataa ctctgtacca tttatgcgca caaatatgtt agaggacctt 23580

caagcgaaaa atcgtagcgt tatgtctaga atacttggtt aaattttaat gcgttataat 23640

atctttttct atatcaatta taccatgttt accacttttc ttatctctag gtataatatc 23700

ataaattggg tttttttcta ccccaattaa tgagtttacg atatctgtaa gtggttttat 23760

tatatctcct ttagttaaaa tttgataaat taatatatgt gcgcttaatc ctctggaact 23820

tatctcatga atatcggttt ttgatttcat tagtattgta tttgaaaaat aagtatataa 23880

aatattatta tttatcaaga atgcagaatt tgaaacattt tcaaaatcaa tagcatcgtc 23940

agcatttaaa tctaatatat cactagataa tgaactttca tctaaataaa ctaaacattt 24000

tttgaatggg aaatcgtaat tgctagtatc gaccgtgatg atacgtttat caatgtctat 24060

aactgaatac ataggcatgt tatttttttt tgtttggtta ctgataaagt ttataccata 24120

gttaactcta acatactcta ataatgtagc taatctaact gttagttttt caggattttt 24180

agctaaatct tctaatctgt ctatttgtga aaacattttt aacattgaca taagttgcaa 24240

tgttggatct actatatata tattatcaat taaaactttt ggtattgatt gcatggtatc 24300

ttgtcgtata ttaaaactat ctattatatg gctatcgttt tgatccttta acaccatata 24360

gttttttaag tatggtactt ttaataaaac aacatttaag ccagtaataa acttgattaa 24420

aaacgcaaga tctataagaa aagttctaga gtttgtttgt aatatatcta tatctccata 24480

tttaacatcc ggatttaata aatgtagaga atatgatcca taacagatac aatttttatt 24540

atgtcgtcgc aaatactctt ccattaaaga gtttacatta tcaactaacg atgaaacatt 24600

atgtctaccc atggttttat ccgtcatctg tgcaacgttc ataaaattta aagcatccct 24660

agctaaagat tgcatcgatg atgcattata gtaaatatta gaagtaaata aatcaggccc 24720

tttagttgtc aatatcccta atacatcagt atatgttgta actaatatac tttgtaattc 24780

tattatggta tataacttac caattgagta tgtgttctct tgtttagaaa agtaagtaag 24840

aattgatctt tttatatcag atggagaaga cttgatatca gaaaaaaatt tctttttatt 24900

cttttttatc aaagatataa atatttcttt attaaaaatg tttactcttt gtatgttttt 24960

atattgtgac ttcaacatgt gaaactcggt taaagatgga tctcttttta aatacaattt 25020

taaaacatct acaacaattt tatcattcat ttttgtttat tcaatatgat gaagatacaa 25080

actttttcac aaaaaagtag tgtatttttt aaataatttt tttttattat tacaaatcca 25140

ctatcgcata tatatttttt aaaaagattt aattctttac aaacgctaga tatgtcttca 25200

gtaagtgttt catttatttt cattcctgta aagaaattac ttataagtaa agatataaaa 25260

acattagttc ctctactaga atacgttgac cctactacta agtataaaaa aagaatttga 25320

ataaatgaat ttattataga gtggaaattg gatatgggtt catttagtga ataaatatta 25380

gttatataat tgcctaaaaa tttatattcg ataaaatcta caggcgatat cttaaaaata 25440

acgccgtgct ctatctttga agggtgtgaa tatttagatc cttttattat atcaattata 25500

ggaacccagg aaggaaaata tctatatggt aaatatacca aaccatatgt cgttaactta 25560

gcaattgtaa atacttgatt taaaatatct tcttctgttt ttaaaataat atcattttct 25620

ttatttattt gtatttttat tccagtgcta tcttcaatat aagacatatt tttaaaaacg 25680

gtcatatcga tttctggtat attactatat aatattaatg attttatttt attttgatca 25740

tatataaaca tactattttt atttggcaat tttttaaatg gtattaagtt ttttatatca 25800

ctaacctttt taaggataaa aataacatta ttttcgctgt gtgtgcagga tagataaaat 25860

aacgacggtt gaattttata gctaaaatat ttatttatat tattagatga aaatatattt 25920

agtaaataca tcgtagatgt tttatttaat ggataatcaa acattaactt gttcatttta 25980

tgagagcaaa atccatattt ttggacaaaa atatctatta gcttattatt tttattatac 26040

caatcagtac taaatgtgta atccgaaacc aagtttgcgt ttttttctaa taatacattt 26100

atatcaattg tatctagtag ctcaactaaa tcttcgtatc ccatttcaat acacactatc 26160

ataactaacg aactatgtat tggaatatct ttgattttaa ctttaatatt tgattcacaa 26220

actattttta tacataattc ttctgttaat atatttaatg gtattttatc tataatagta 26280

gaaagcaaat tgatataatt tttttctatt aagtaattga taatatacat tttattgtta 26340

taatcataat tatcaatata tttacagcat atttttaaaa aatcttttgt caattcatat 26400

tttgtaaaat ctattaatgg atctttaaaa aaaaaactat aatataatcc taattcatct 26460

tttttatcgt ataaaaattc tatagcatag gttttatatt gatcaacttc tccatttttt 26520

attttagtta tgataaattt ttttattctg tcatctttta attgttcaga cgttaacata 26580

tctaaaaatg ttaaaatatc tttatgaaat tttatagatt gtaaaacatc caagataagt 26640

tcaatagaaa ccacgcttaa aattccatca ttgtttggta ttattccata tttatataac 26700

atatccatta aaaatttact ttcaaaggat tgatgttgat ataaactatc acataaacta 26760

aagtcattaa taaaaatttt ttctgccaat gactcactaa acaaatagtt tatattttta 26820

atactttccg aatttatttt agttagtata ttttctactt cgatgttaga aaatctgtgt 26880

attagacaaa aaatatcatc atctgtaata accatatact gaatacattt cagtattatt 26940

cttttactac attttttaat aatatctttt ttatgaaaat ttatatgttc tttgtataac 27000

tcaatatttt tttgattata aactaaagct atcaaatcaa caaatggtac agtttttggt 27060

ttgaataaat atattttact aggaaattct tttcttatcc taaaatacca tttttgtggc 27120

aaattattgg ggttaattcc attactgagc attttcatag catcatctat atttactttt 27180

ttatatgatt ctgttaaatt atcacaatta tataaaataa atgcttcctt taacggaatt 27240

aaaaaattaa acatattatt gtaaatttaa aatttgataa tagatgtatt gattagaaaa 27300

tcaatagttc gtttcgtcga tttttgttta gcttcttttt ttgtattacc aatttctgga 27360

aaaaatttta taccgcttat aataacagag gccaaaaaca ttggttttct accatttcca 27420

caactagata tatttatgta ccaatctcta gatgtaaact gacaatattc gtttatcgca 27480

gaacaaggat ttttttcttt ccataaaaca attttatagt acggaattgt atcagaaaat 27540

gatttattta gttttttcgt atcgttattc tcattgtcat cgttatcgat gccatttttt 27600

ttaaaccatt taggtggatt cgatcttatc ataaaaataa aaccgtcatt atgtagttta 27660

tacagctgtt tgtttacatt tgatttttca atatttagtt ttctagatat ttctattgct 27720

gttataaact cactttctga tagattattt accatttttt tcacaagttc atacgaatct 27780

acttcatcac aagaatacat ttttattttt gttgtattta aaactaaaaa attttttttt 27840

tttcattttt atagtttatg ttatttttct acagctctaa actttggagg tttaaaattt 27900

ttcttgcagt ctctacaaga atgcataact aatggtggtt catctgctgc tcttgtttgt 27960

atcatgacgg gaatagtgtt tttacttttg cactcagggc atgggaggtt atacttttta 28020

tctaaaacat taaaatattc ttcgtggttt atatctctaa tattgtctat attgaattct 28080

acattttttt gaacacattt tattccaaaa agtatatatc ttaaaatttc tttctctgtt 28140

ccatttgtcg tacgtataag atcacatatg tcagaataat ctttatttgt ctctattaaa 28200

ggcttgtttc tatacgataa tttatttttt gaatcttttg agtattcaat acctatattg 28260

tttcttggtt caaattttgt ttcttctata tttgactttg tattaataat atttcttata 28320

taaaacttgg aagctttgtc tgttgcccat tttaatattt cttctacctt tgatttttct 28380

tttacatatt tataaattat atcttctatg tcttgatatg tgggggtagt attatcacta 28440

tatgaattag tattatcatc atccatattt gtactgaagt tgtttaattt caatatttac 28500

aaaaatgaat ttttagttct ttatttagat ttttccatta atggcaaaaa tggaaaatat 28560

tacaccacct aatcactttg aaaaagcttc tgtttatgca tgtaggttat ttaaacagtt 28620

ttataacgaa aaaaaatatg atttagatga atgtttttta aaagttagaa atagcatgtc 28680

tcgatactat tctatggttg gtgatgattt agtttggaac agagaaatat taagttactt 28740

atatgaatac atagcatata aaaataatgc aaataatcca aattttgttc tatcttcaaa 28800

aattgttaat ttaactaaga aaaatacaaa aaagataaaa aatgaaataa attctgttgt 28860

tcaaacttac aaattggata aacatataaa aaaagtaaat ataacacact atgattatat 28920

aaaaataaaa aaaacgtatg atacattcaa aaaaaatgtt gatattaaat caaatgatat 28980

atctgtaaca ctattaaaaa tttataaaca actaagctta attagtgatg tttcgataaa 29040

tacacgttct atgacaaaat ctttaatgta ttacatgttt cctgatctct ttaaaaagga 29100

taaacgacat gtattttaca gagtagacgc taaaaaagga tattgtgtat tatcatctaa 29160

aaagataaac ttaattaggg ttttattaga atataaattt gatataaaat acaaagagtg 29220

gggattaata aaaagatata ttaatactat ttgtaataaa ggtagtaaaa gtagcaaaat 29280

ctgtattgga aaatatccat atttaaagtt aaaacctgtt tctaaaatta aatttaatta 29340

tagatctaaa tacgtaagtg attttaagag aatagtgagt tctagttttt cattggttga 29400

tttgacagaa aaaataacga aaaaaacttt tagtaatatt tttagaaatg aaattagtca 29460

tctgtataag tttaatgcag aaaaaaacca tctcgcgcta gatgaataca aaaaaatgag 29520

aatgtgtaat aaaataatat ctgctataga ttatccaaat aaagatcaca tttatagtgc 29580

gataatagaa actgttaaca attacttaga taatcctccg aaaaagctag gatacaatat 29640

tgatacttgt ttgactatag caaaaaacaa ttaacaacta atcatggatt ttatacgaag 29700

aaagtattta atatatacaa tagaaaacaa agttgatttt ttaaaagatg aagtactaac 29760

aaagatatca aactttacat taaatcatat attagccatt aaatatttaa ttcttaactt 29820

tcctagagat gttatatcaa aagatgtatt atcgaaccca aatttttatg tttttttaca 29880

tatggtaaag tgtgatgaca tttatgagac tgttttaaaa cattcgttcg atattcctac 29940

gttatatata aaagcactta taaaaaatta tccgttattt aataacacaa tagaaagata 30000

taaaacttta gttagtgaac tttttttaga cgataaattt atcgaaatag taaaatattc 30060

gtctgaattt gataatatca tagctgttaa ttacgatctt ttactaaatc ctttattcca 30120

taataatgaa ccgattaaaa atatggaaat tatttatagt aaacttttta aaaaatctaa 30180

ttttaggaag gtaaaaaaaa tggaggttat cagattaatg atatgggcgt acctaagtaa 30240

acaagatact ggattagatt ttactgattt tgactctcaa gatatataca cactgtttca 30300

aaaaagtgat aaagttatta ttcatagtga tatgacagaa aagtttaaag agtatatttt 30360

cgggtcggaa aaaactagtt attggttatg gttaaatgaa tcaatattta atgataatgt 30420

ctacaatgaa gggtttgcag aattaatgta cgataaaatt cttagtttta tctattcaga 30480

gattaaacaa ggaagagtaa ataagaatat gctgaaatta gtctatatat ttgaaaatga 30540

tgaatatata cagtctattt tattacaaat aatttacggg atacctggtg atatcttatc 30600

aattatagat gtaaaggatg ataactggaa aaaatatttt ataggttttt ataaagaaaa 30660

ttttatcgat ggaaaaactt ttataagtgc aaaaacattc aactatgatt tatttaaagt 30720

agtggctaaa ataaatcctg agtactttga cccagacaaa ataatatcca tatttgatca 30780

taaacctgaa aaagttaaat attttgatac aatagatata aataatacat ttatatcaaa 30840

tataatttat gaaacgaatg aattaaatct ttcaacgatt gaagaattac aatcatgcca 30900

aatatataat gaagaaacag aatatttcat taaagaatat aatacttatc tttatttaaa 30960

agagaaggat ccatatattt tgtataatgg catgttaaca aatatatcaa aagttccatc 31020

aaataaaaag ttttcattgt ttagtaaaaa catattaaaa tattatatag atggaaaatt 31080

ggcaaatata ggtcttgtgt taccaaatta taaaggtgat atattggtaa aaatattatc 31140

acatttgaaa tgtgtagagg acgttactgt tttcattaaa ttttcagtat gtaaaaactc 31200

tagcatacta ccatcaataa taagaacaat tttagcaaac tttaatattt caataataat 31260

attatttcaa aagtttttaa gggaaaactt attttacgtg gaatcttttt tagaaaaaac 31320

aaatcattta actaataacg ataaaaagta tatactagaa ataataactc gtggaagatc 31380

ttaatttaaa atgatgggag acatttattc agagtcacaa aaaagaaacg acgatcaaaa 31440

aacatacttt acaagaaatt taagtccttt aatgaaaaat acatatttat accataatta 31500

ttcatatgga tggataccgg aaacagctat ttggagtagt agatttgcaa atttagacgt 31560

aacagagttt tacccaatta caataaattt aataaaaaaa tttgaattta tgctttcatt 31620

atacgatggc cccatattac catatgaaga aaaaattaat acagaattta tttcaagagg 31680

ttctttttct ggaagatata taaatttttt aagaaatttt tcattactac caactagtga 31740

attcatttca tttttattat taacatctat accaatatat aatattcttt tttggtttaa 31800

aaatactcaa tttgatatta aaaaacatac actttttagc gaagtttata ccaataacga 31860

taaacatata gaactaggaa aatattttag acaaaccggt gattataagc ctttatttag 31920

caggctaaag gataataata tatatacttt gccgtttcca atcaaccaaa gtaacattaa 31980

caacccaaca ttacctattg gcttttccaa atcagattat gaaacattat ctaatttaag 32040

cgcaatactt tatttaacta attatgatcc tgttttgatg ttcttagtgt tttatgtgcc 32100

taaaatatct ataacgacaa aaataacacc agctgttaaa tatttaatgg ataaactaaa 32160

tatgaatgaa acggatataa cattagttta attataaaaa ataggtctag ttccgaacat 32220

tttttcaaaa aatgatatgg ataatacttt gttatctaac aaattcacta tttcagtggc 32280

aagcctttta aagtatactt cgtaaaatat tcgttcagta gattttaatt taaaactcct 32340

atctataatt ctttcatacg tttttatatt tactaatttt ttttgccatg gttctttttt 32400

attgcatatg tatgcaaagt aatatcgttc acctatttca ataatttcat cattattttt 32460

attatattca tttactaagt acatatttgg attatcactt gatttataat tacaatgatg 32520

tgttctactc aatagaaaca tatccaatgg agctgttttt gtttcaaatt ctatttgtaa 32580

atcactttct aacgatgata aaatttcaac acatacttgt acagatgaca tatttccacc 32640

tgataacatt tgcaaaagcc ttgttttgta tattcttatc atatatttgt gaaacttaga 32700

tacgtctctc ctcgtctcac tagttccctt attgatacgt tcgggaattg attcattggt 32760

gaaagat 32767

<210> 4
<211> 32767
<212> DNA
<213> Artificial sequence

<220>
<223> nLSDV deleted in SOD (no marker) full genome sequence

<400> 4
   aagccaatta aacctaatta agtggaagcc aattaagtgg aagccaatta aacctaatta 60
   agtggaagcc aattaaacct aattaagtgg aagccaatta agtggaagcc aattaaacct 120
   gtaaatggat acttttttca ttcaatcttt taagtctaat cattagtgct gatttcacta 180
   gcgaaatatc gttgtcggaa acgaggtttc gaagcaatac caacactttc acagaagaac 240
   aagttggaga tgattcacca ccccaatatt ctgctgctct tgctaaaatg ccaatcactg 300
   cacatgattc cctaatgtaa ttttcttttt tcaacatgga attaatcata atttttgatt 360
   gttcaaatcc aattttagaa tccaaaaaca tgtttttgac aaaagctgtt agatcatttc 420
   caaatacaag tgaggcatcc tttttgaaag attcaaaaac taagaacacc tttccagcaa 480
   cctccctgga ggaaaatgcc aatgatgacc aaaacaaata atcagagatg gcggttgtga 540
   tatcatcatc atctgaaaag ttgtttcggt agacatagtt gccggaagac atcttgtaaa 600
   atgaacggga aaaaagtctc ttttaacccg aacttgttat ttcctgtgct tatttttcaa 660
   aattattcaa ccatattcat catcctttca actaatcctt ttctaggtgg ttcctttatt 720
   gtgttttgat tacagtagta ttcgtagtgt tttttaaacg gattaggaat tttaataaaa 780
   tcaaaaactc cattatcatt tctaatattt atatcgcttg tatctttttt atctaataca 840
   gttaatggca gatcattatt tatttcttta ctttctatag gtttgtgatt agtgtttata 900
   ttgtcttgga ttttttcatc cttatccaag acagaatcga acggatttag gtttccaaac 960
   atgaaggaga taagcttttg cattggaaac attaataatg aatacaaact atatataatt 1020
   aaattacata atctagctat ataaaaaata cacaacatac aaactttagc taagctatta 1080
   atgagtagtt gacaaaatcc ttttattgca tatatcacaa atcctaaaat ctttagtata 1140
   agaactaatg tccctcttac acatctccat atactaaaga ttattttatg aattaatgca 1200
   aaaatccacc agactaaaaa aaacggaaaa ctcctgtttc ttattcataa tttatatatg 1260
   tgaaaaagaa caaaataaat attatatcgt cttttgtata acaatcctat agttcattat 1320
   gatgaaatat ttttttaata taattatgct gatgttcata atattttttt ctatcaaatt 1380
   taccaacact gcacgttttt aaataattta cgtgttcgct aaattttaca gctgagcaat 1440
   ctattttatt attaaaccca aaaatctttt ttcctgtaga tatatatttt ttttcacatt 1500
   catttctaac tataacactt gaacttacat gttttacaca actaatatct gttgttataa 1560
   caaattctaa atcataatct ttaaaactta cactctcaac gtatgatttc atttctgtag 1620
   ttacttgatc acaagaaatt gacaatgata ttctcttagc acactgtggc agcgaataca 1680
   gagttgttat attactggtt gatatttcca tatatacata tttacccaat gaatagactg 1740
   cttcgttaac agtatctaat ataaagttgt tttgtgtttc ttctttggaa caatcatcat 1800
   gtgcgaaaat tttaaatcca tttccctctg ttagaagtcc tccaggtcct tgaattttat 1860
   aacagccagt tttaatcgct ctaaaattct ctgtttggat acataatcct acttcgattt 1920
   cccatgtgtt attttcttct tctgtgcatc tctttattga tattccgctt acactactag 1980
   taaaaatgaa tagttgcaaa aagaaaatga acaaagttaa tgatgacata ttggttggtt 2040
   gttttgacgt attaccgaca caaaccacct cgatactact gaactttttt ttctattatt 2100
   aactaaaaaa acttgaaaac aaacctttct ttttttcctc tttcttaaaa ggatttatat 2160
   atgatagaaa actgaaactt tttttctttt gcctaaaggg gtttatatta aacgaaaaca 2220
   gattttttgt cgatgaggta atcatgttat aaggattcat taaccctatc attgcccaaa 2280
   aaaacttcaa aaataattat atagagtagt atttaccacc aacatgaaaa caaacacaaa 2340
   aataatacta ttctgttatg ttatttttct tagtttgtat gtatttagtt gtgtagtagc 2400
   atcggctaaa aaatgtgacg acgttagctt tgattacata ttaaaagatt tacgttcaga 2460
   atttagcaaa ataaaaagtt ttgttcaaga caatgatcaa gaaaatatga tgttattaag 2520
   tcaatcaatg ctggacaagt tgacgagtcg cataggatgt aaatctttat cggatatgat 2580
   aaaattttat ttaaatgatg ttttaccaaa tgcagaaaaa atagaacaca tgaaaaataa 2640
   aataacttca ataggagaaa aattaaaatc gttaaaagaa aaactcatat cgtgtgattt 2700
   tttacattgt gaaaatcatg acgaaataaa aacagttaaa acaattttta acaaattaaa 2760
   agataaaggt atttataagg ctatgggaga gtttgatatt ttcattaatt acttagaaaa 2820
   gtacatagtt aaaaagtaat caaatctata cttataaaac tacaacatta tagtttcttt 2880
   ttatgtcata gttaatatta tctttgttta ttcttaagtt acatgtataa ataccactat 2940
   catcatgtgt tgtgtttttt ataaccaact taagttcgtt actttgtatc ctatcatcgt 3000
   atagcacttt gttattattt ttataccaat ctagtgtctt atttttaaaa taactactaa 3060
   taaggtcaat atccatacaa tgtactgtcg aattaagtcc ttcattgaat ctgtaagtaa 3120
   ttgtctcttc tttatccaaa aaagttaatt ttatagacat ttcttcacaa atattatgtg 3180
   aacgaaatct acaaatataa attccgtcat cgctatttat aacagggtac aaccataagt 3240
   tgtttcctat aagttgtacc cttgcgtttt cattatttac ttctattttt ttaaaatttt 3300
   tgtaatcgtt ataatttctt acaaacgata gagagttagt ttgaaaccat gttatgtcat 3360
   ataatgttat atttttttta aaaaaaatag attttttata attatccaat gggcatttta 3420
   aaaatgccgg atctttgaaa tttacattca ctggagttag aataccgcct ctatatttac 3480
   aatattctga taaaaatgcg ctttttaaac aacatattaa tatgataaaa tttataactt 3540
   tcattttgtt gttgtgttat ttattttaaa tagaaaaata gcatcggact acgttaaaat 3600
   ttatataatg tatttcagaa ttacagttcc cagttaatac ataataattt attttatctt 3660
   ttaaattatt atttacgtac ttaactaagt cttttaaaaa atcaaaatcg ttttttgatt 3720
   ttggatacca tatttcatca catgacgaat ctaaggcttt ccatgctata ttttttccat 3780
   cttcgttttt taagtaatca aaaataatat cattataact aaatgatatt tccatacaca 3840
   tatcgtcaaa attttcaaat attaattttt ccgttattat atcatcatac ttgtttacca 3900
   aaacacattt acctgatcta tttaaaatta atccaaatgt atctacttct gtggaataat 3960
   tatttacggt aacaagacag taacaaaact tcctatttcc ttcgtcttcg taaaagttta 4020
   tatgattatt ttccgtcaaa tagggtaatt taaatatggt ttcgtcatta tttgaattat 4080
   attttataac tacatttatt aatgctatat ttttttctcc atcagttata atatcagaac 4140
   tgtgatttaa tgttttatca aataggacat cgtttttagt agaaaatata gtattgggtt 4200
   ttatatgtat atttgtatta cctcctttgc tcgtattatt taaatacagt actaaatgat 4260
   aacaataaca gttttttgat ttgaaatgta taaaatctct gtgtgtgttg aaaaaatctc 4320
   ctttttcgta ctttataagc gtaattgtat tatctaatat aatatcgcta attttttgtg 4380
   gaaattcaaa agataataac agtttttttc ttataacttt aaaaatatca tctaaacctc 4440
   ttattatata ctgtttacat tttctttcat tagtattaat aacttctata cctttttcat 4500
   gatcaaatac tttagactct tcccatatta tgtttttgta tttaatcatg tccataatta 4560
   tttttttaac gttattaaaa taatcatcat taaaggtttt tacttttatt ataccatcac 4620
   taatcgtttc catcttcaaa aaatattatt tatatttaat attttttaaa aactaatgtt 4680
   aaggggtttt tttttatgtt ttataaaaat agtatgttaa ttcaattcat tgacgatata 4740
   ctcaaaaagc gataacacaa gattgtaaat tcctacaatt ttattaaatg atttttctcc 4800
   tttgaattta tttttagtaa ctttctgaat tcgccgttta agcaaactta aatctgtttt 4860
   ttgttttaca agaaatgtat caactttcct aggcgttaat tcgtcaatac atgctttact 4920
   tgtttcattt gtccttattt gataataatc agtttcgtaa tttccaaaaa ccgtaacaca 4980
   aactttttct tgagaaccaa attcaatagc acatcccttt ttatcacaaa attgttcatc 5040
   aacttcatat ttaattgtat ttgaatcacc aaacgtaata cttaaaatat atttaaacac 5100
   aatattacaa tacttatcgt tattatatat aggagaattt ttattattat ccttggctag 5160
   tgggtgatta atcgttagta cagtattaac atcttgtcgt ttaataaaca cagatggagt 5220
   agtaataaca acactttcac aaattggttt aaacgtaaaa acgttcgatt ctgtattatc 5280
   gactgtaata aattttatcc aaaaatcgtc agtattatca tctataaaag gtgaaacatt 5340
   acaatatttt tgaatagtat aattacatgc ttgtttccat tctccactgc tataagtcat 5400
   aatcattacc ctatagttac tatcgctgac atcgttagtc cactcgattt tagaattaaa 5460
   atcattagat gttagtttta tatctttagg aaaagaataa catagtttaa agagcaaaaa 5520
   agcaaaaaat actactgcgc ccatttttta gtaagtattt agttatattt tatatttttt 5580
   atggaaatct atcacagata tatttttcaa attcttttct cttaaaatga cttatttcgt 5640
   caaaaaatat gtaaaagctt ggatacttac atgtatcatg tgaagaaatt attgatatta 5700
   aacctattat atatttcatt gtggttatgt caatcacatt attttcattt ttttcataaa 5760
   gcgaaaaaca gtaacgtgtt ttacttaaac gcaattttga tattatttta ttaatatatt 5820
   caacaccgta gtgattaacg gccatagcat catatgttaa aaactcagca tatattttac 5880
   ctattttttt tgtatttttc atttttcgta ccataaaata attttccatc atatcgttaa 5940
   aatttacttt tttcatggaa ttaatatgct taaaactttt agattttata aatggataaa 6000
   aatcagcata gtattcacca tcaattttta attcttttat aactttttta taatcatttt 6060
   cattaagtac atattctgat ataactttta tcatagtaaa cgtaaattta tttgaatttt 6120
   ttaatacaat cttcgttatg tcatccttcg tataatcata atttgaaaga taaaaaactt 6180
   tatttaaaaa atattttacc tttttttcat tgatagaaaa aagaaaggtt atatacttag 6240
   taaagatttt aaatttattt tttgtataca taatgcataa cgattaacta tttttaaaaa 6300
   gtgtaatatt ttttttcatt tttatatata ctcataaacc tgatatgttg tgttatcact 6360
   cttcgcagca agataataat cgtacaatgc tattagtata tgtactacag ttataaatcc 6420
   cacaacacaa ggaagggaat atgccatcgc ggatattagt gcaataagat cacgattttc 6480
   aatattttct aacatacttt tcaaattaaa cttaactaaa agtatggtta gtggtaaaag 6540
   tagtaaacat attaaagaaa cgcaaatagc tggcacgtta caatacataa acgaacattt 6600
   ccatcttaaa caactctttt tatttttttt aatattgtat tttccattac atattttaca 6660
   ttttgtatta tgtgaaaaat taatccattc ttctaaacag tttttatgaa ctatcttaaa 6720
   ttcattcttg caattacaaa agtttgtact tacattatat tcatctttac aaatccaaca 6780
   atgagtgttg gtattatcac tcccttccat ttttataaaa tatcattatt tgttgttatt 6840
   atttttttat tttttatcca atgctaatac taccagcact actagtgcta cgcaatcgta 6900
   aaagcttttt agtaaattct ctactacaaa acgcataaat tagtggatta ataaaacaat 6960
   gacatagaga cacaatttca gctacatgaa ctgcaaggtt gacaaatcgt aatgccgtac 7020
   atccactaaa aacatttaac aaatacaacg atgaaacaaa tacagttaca ctaaatggga 7080
   gtaaaaacaa tactgaacag ataacaatca aaaacaccat ctttatggct ttcttattct 7140
   ttgtttgcga ggtttttaaa gtatttaaga ttttatagta acaatatagc aaaataatta 7200
   gcggtataat cattccaaat atgtttattt caaaatttat aaataatttc caaatttttg 7260
   cattatcgtt ataaaataca tgacaatacg ttattccata tacttttttt gtttcataaa 7320
   ataacattat tggaaaggat tcaatagttg aaacaatcca aaccaccata ctaagtacaa 7380
   ttccatatcg ttttgtcctt atcggcattg attttactgg gtgaactaca gctaggtatc 7440
   tatcgatact catcaatgtt ataaatgaca tactattgta aaaaccaaca aagtaaaaca 7500
   tagctttgaa tttacacaaa caatctccta aactccattg tttagcgata ctatcgtata 7560
   aattaaaagg aaacaccaac acgaaaatta aatcagacaa tgtcagatta agcaaaaata 7620
   tatcctgtat tgtttttatc ttatatttac gaagaacagt taacacaatt atatttccaa 7680
   ataatccaag aaagaatata gtcgaatata aagtaatcag tccaaaactt gtagtatcca 7740
   caccatcatc acaatgtggg atatcgacta tattcacttc ataatcatca taatcatcgc 7800
   tataataagt tgtattatat gcggttgtat aattagatat tgttgtcgta ttttcataag 7860
   ttgaaggcgt tgtaacatta ttttgatttg ttgaaattgt actgagaatt gtgctaataa 7920
   ttgtagtagc tatagtggta atattactgc tactattata catggtggcg ctactaactg 7980
   tgctaagagt ataattcatt tttgttggag ttgtgcttta cttaatcata cacttttatt 8040
   ttattttttt tttttaataa aaacacagta ttattgtatc atcatttatt tttttaaaaa 8100
   aactttttat tgtttgtcaa aaaaggtgag atttctaaat ttaattaaat ccttacaatt 8160
   gtaacctgac gtattagttt catcaatatc aataccaaga ctagttagat aatcaaatat 8220
   ctttttatca gaatgaaaca aaatataact atacaatata ttattattat caaaatcttt 8280
   ttgcaaaaaa ttaactccac tatctattag taactttatt acatttatac gaacattaaa 8340
   attacacatg tacatgtgta acaaattttt cccattttca tcttcttctg ttacagatga 8400
   tccagaatca attaatattt taactatttc aggttcgaca ttcttgttaa aagaaagata 8460
   atgagctagt gctgataaat tattatctct agttttaaaa tttatatcgg atccattttc 8520
   tatgagaaat tttaatattt caacattaac tttatctttt tcgacacacg aaaacaatgg 8580
   cgtttcatat aagtcgttac aatcattaac aaattttatc catttcttta ctccatttat 8640
   atcatcttgt tcaacgtaat aaaataatgg attacaatat ctataaaata agtaatctgt 8700
   aaagtcatta tcataatcac tacataattt ttccttttcc atagtaataa ttgatgatgg 8760
   caaactagct tattaataaa aagttatatt tcatttttgt aaagtgtttt taaatttaca 8820
   tatttaattt tttttgtgag atagttgtat tctaagttat gtaagttaca tgtgaaatta 8880
   gcagatagat attttctttt aacttctttt ataattaatg caattttata agagtttcct 8940
   acgtacattt ctctacctaa gtaaatattt ttacttctat cgtaatcaag tacattaact 9000
   ccgttaatta accaaaatat tgaacttcta taaacacgtt gtatgttttt agattctcca 9060
   atacattcca tcaaaaagtt tttacctaaa gctattgtac tatttaaaaa atttactgtt 9120
   aaatttatat catctttaaa tcttttttta attacgttat tatgataata acgttttaat 9180
   gccaagtttt ttggattaca ccgtgtagca gtagttatgg aacataaata tactccttca 9240
   tcactcttag aaatatttcg tattagtaat ttttctttgc tttttttata tttaaaatta 9300
   ttgtttattt tttccctgtc tttaaaccaa tttacttcat aagaacaatt aaacgtattt 9360
   ataaagtttt ttgtatttat acacaacaac tcatagtttg taccaatgac agagtttata 9420
   gtctcttttt tataaatatc taacataaca gatgaagtaa aataatatgt tgagttatgt 9480
   acattacatg catatattcc agaatcattt tttgttatat tatttatcca caggctatgt 9540
   ccaacagaat atactctatc ggtatttttg aactcatgaa aagatttttt tacatcccct 9600
   gttttaacgt attcattgta attaactaaa aaccatttta ctgttgtatt ataatttaaa 9660
   gtatcaacat atggacattt tactatttct gagttatttt caaacgtttt catcacggat 9720
   tgtagataac caaagcttaa ttttgtaaaa gcaagaattt taacattgat aatcagtaaa 9780
   tagaaaataa atgtcgttac tttttccatc ttatccaata agattttaat cttacaagat 9840
   ttaaaaaaaa atattttttt ttttaatttt tagttgttat tataaacttt tacatcaata 9900
   tagcccttta ttttgttcat ccttataatt tgcacattaa ttaatttatt aactaatgtt 9960
   ttttttatat tattcatttg atccatgttt atattaacat aatctattac aagtgcttcc 10020
   aaaccatagt tgattagttt tacatataca atgttatctt ttataaacac aataccatct 10080
   gttacttcat taatgttagg taaaacgtaa caaaatgcca aatcgctatt cgatgacatt 10140
   tttgctctca ctttctgaaa cttttttttc agaaataccg tttttgtgct cgcatgtatt 10200
   atttaaacaa ttccatatat cgttcaatat taaagttttt ttaattggtt cctccaaatc 10260
   cattagaaca catgttaagt tagtattttt aatttcttca gttacgtttt taattatcaa 10320
   atcagatcta ggttctttgc cacattttaa aggttttttg ttaaaataca ttcgttcttt 10380
   ataaatgtcg ccgttcataa attcaacaaa cgaattatta tttcctatca gccaatacac 10440
   atatgcataa ttgctatgat gactatatcc tttacattgt agtaaaacgt tttcgccctc 10500
   tttatgagga aagtaaataa ctaaatccct tttttttgca catttactat aaaccacata 10560
   aaaggttgta aataatgtca gacaaatgac tactttcttg aaagagatca ttttttattt 10620
   tttagaacta tattttatat ttttttttaa aaataatgaa acaaacttct taaatgacat 10680
   ctaacgccct catatcctaa tttgcatctg caaaacatta gtggtttttt attgtaagat 10740
   aaaataaaag ttgttttata acatgtacca ccatttaaac aaaaatttat acttttatca 10800
   cacatcatta tatctttgtt taagatagtt atgtaatcgt tttttatttc tttagaaagc 10860
   attaatataa ttttatagaa taacaaaacg gtaaaaaata ttttttttac ttccatcgta 10920
   tcctaccaat tattttaagc aaataatatg taataataat acttcctatc attacgaaaa 10980
   agttaattat tgaaaatata aaatcatttt gttcatcgat ttttttattt ttttcaacaa 11040
   aacttataat gtctttttga taactagaaa atgtatttat tagctcattt agtgctaaat 11100
   catcattaac aacttcattg ttttcacttt ttttcttttt tatgataatg gataacaacg 11160
   atatagaagc taatattact gaaggtctaa aatcactatt tatagtttcg ataattatat 11220
   tttttacatc atttatatca taacaagatt taacgttatt gtgtaaaata aaattacata 11280
   tttcattaaa ctcagtttta taatcttttt ttattacctc acagcattcg cgtatcatta 11340
   ttaaaattgc caactcatta ttatttaatg attctattct taagtctcgc aagtacacat 11400
   tcaaaacttt ttctatgttg taattgctgg tatccatttt tacaaaaagt aacttatatt 11460
   ttcctttatt ataaaaaact ataatattcc acttgaccca aatccattat tatctctatt 11520
   tgtaccatct aaacatttaa cttctttaat ttttggatat tctacttttt caaaaataat 11580
   ttgcgccact cgatcgcctc ttttaatatt aaatgtattt ttaccattat taataaatat 11640
   aactccaatg ttccccctat aatcttcatc tataactcct cctcctatat ctatgcaata 11700
   atataacgaa agaccagacc ttggcgctat tcgtccataa catttttttg gaatagataa 11760
   actgatatcg gttttaacta aaattctttc ttttgattta acgatataat cataagcact 11820
   atataaatca tatcctgcag aaaaatctgt tgatttggta ggcagtttag caaaagcaga 11880
   caaacgtata cagttaacga cagacgaact atgatccatt ttgttatttt ttaaaattag 11940
   gatatttttt ttttgttttt ttaaaaaaca ctatctatgt ctaatgctcc tataacgttc 12000
   catccttttg ttttatcgtc aaatatttct atttcacgta tatatctttc ataatgaaaa 12060
   cctccaacga tcataataca atcatcaaaa atacatattg atgaattaaa tcttggctga 12120
   ttcagacacg actcaattct ccatttacaa gacataggat taaagctttc gaccatgtta 12180
   aaaactttta tattattgat atatgaaatt ccgccaacaa catatattaa accatgatgg 12240
   tatatagcgc atccgccata atgagaatat tttgtacact cacccaatga ccatttatta 12300
   tatttcaaat tatacacttc tactgattta tcatattcag atattcctcc aataacatag 12360
   atggtatcat tgacattaac aacacatgga ttgtatctag gatatagtaa atttggttct 12420
   tctctccaca cactttctcc aaatgaccaa ctttcaactt tattaactat acaattatta 12480
   tagattccac caataacata tattctatta ttaaaataag tggcacctgg gcattttctt 12540
   ggataaatta atgaaggtaa gttttctctt tcaaaagttt ttacattgat tgcgaataca 12600
   ctatttactg atcgtaagta tttatccaca ccccctaaaa agtaaatatt attaccaata 12660
   gcaattgaag tacaaaactt aaaattttca tctattatgt tgcttaattt acttaagtta 12720
   ctgggttgtt gaatactttc atttaatgtt ttgttttcat caatttcatt acaattgttt 12780
   tcatacaata gttcattatc ataaacactt tcatctatta tatttatatc cattatatta 12840
   tttgtaaaca gtatttttgt taagttatca ttataaacct taaacttaaa agactttgta 12900
   aatttgtcac tattaataaa ttctttattt ttatagttac atcgtggtat gtgtttatca 12960
   ctatttttgt taaaatcata aggttttttt ttaaatctgg acgaccacgc ttgtaacctt 13020
   ttttttcctt tttctgttaa aaaggtaaat cttacagcgt catttactaa cttcatcgta 13080
   ttaagcctat tagttttttt gacagttccc cacttaatta taaaattaag aacaaaatct 13140
   tcggcatcga caactaaatc actacttttt aatatgggcc ttaattcatt aactcttaat 13200
   ttagttaaat cgttgcttat atacataaac ctttttttaa tatatttatc tgcaacatcg 13260
   tacacatcgt aacaaccata catataagat cttctaagat atcaacacaa ttactatcat 13320
   caatgtaatt tatcataaaa tcagtacatg catccattat aaacttaatt tcgattacgt 13380
   tagaaattga aaaaatgtca acaacatttt tgctattcaa tgtaatataa ccagtattaa 13440
   tatactctaa cacatacgta aatggcttat atttaaaagg taacttaact tcgttactgt 13500
   attttttaat agctttttta aagtaattag atgatatctc gaaaatatct ttccttgttt 13560
   gtgatatacc tccaattgta aatattaaaa ttctatcata atcatttttt aataacatgt 13620
   actttagttc ttctacatat tctttattga tatacctttt taaagtcata ttggttattc 13680
   aagataatta acaagagttg ttatttattc aactttataa tattcaataa aaactaaaaa 13740
   tcaacatcta acgaaaaaac attatcgtct ttgttagaaa taacgcccat tttttgatat 13800
   tcactaactc ttttttcaaa gaagttagtt ttaccttcca gtgatatatt ttccataaaa 13860
   tcaaatggat ttgttgacat aaacacttta tcacaaccta gttctaatag taatctatca 13920
   gcaataaact caatatattg agacattaaa acacaattca tacctattag ttttacagga 13980
   attatattgg ttaaaaattc tttttctata gacacagcct cgtttataat aatcgtaata 14040
   atgtcattag aaggaggata ttttagatgt ttaaaaatta aacacgcaaa atcacaatga 14100
   aggccctcat ctctactaat aagttcgttt gaaaatgtta atcctggcat caatcctcgt 14160
   ttttttaacc aaaatattgc agcgaatgat ccggaaaaga atattccctc aactgcagca 14220
   aacgctatta atcgttctcc aaacgatgct tttttgtttg atatccattt tctagcccat 14280
   tcagattttt ttttaataaa atccattgtt tcaatagcat taaataaatg ttccttctct 14340
   ttagtacttg aaatgtatgt atcaataagt aagctataca tttcagagtg tatgttttcc 14400
   atagctatct gaaaaccata aaaacatcta gcttctgacc attgtacctc cgagtaaaat 14460
   ctttcggcca aattttcatt aactatacca tcactagccg caaaaaatgc caatatgttt 14520
   ttaataaagt attgctcttc attgttgagt tttttccaat cggctaaatc cttagaaaga 14580
   tctacttcat caactgtcca aaaactagcg acagcttttt tatacattac ccatatttct 14640
   ttatataata taggaaaaac tacaaatctt gaagatgttt ctttaagaat tggttccatt 14700
   ttattttata ccataaaaat tatatttcat tattttattt agtcaacatt gacgcccaca 14760
   cacttatcat cgatataata aacattttct aattcgtcaa aggatcttct tgtggttaat 14820
   ttcatcattc ttacttttat tttattaaac aaatcgaata agtaaaagca gttaaccagt 14880
   tgaaatagca aataaagact tattacgata acaattatta aaaccatcaa aattagtaca 14940
   gttcctatgc taaaggcgtt attgttaggc attgctgttg taagattcat ctgatgcatt 15000
   tgctaccact attgtaaaat ttatactttt ttttattttt tatagaaata catcatcatt 15060
   tgaaacactg tcgtcataat ttatgtaata cattacttta tgaggtgtct ttctttttgt 15120
   aaatttatac ttgtagttat aattatattt agtatcatta tcatcactac taacggtatc 15180
   actatcacta ttactattac tatcattagc accattacca tcaccatcac catctaacaa 15240
   tgtaacacgt tcaatagata ttgccgtttt catattgctt ttccttataa aactaacaca 15300
   tttaatagcc ttttctttta tacttttttt taataatctg taaccaaata ctataccaca 15360
   ataaattttt ttatccattt ttcctcactt actatcaata acgtataact tagtttattt 15420
   tttttgtttt ttaaccttaa ataatatatt aatttattta gataaaaata tttatttatt 15480
   tttttttgac gattttatac atccttcata gtataaaagt atatagttat agtagtatta 15540
   tgtacttttt gcagtgtaaa ctacttatgt gcaatcaaaa gttgaaagta aacataagag 15600
   tcacatttta tgtatcataa aacgaagtat ttgttgttat taccattgga taatcgttat 15660
   gcaggtaggt ttctttttta tcattgtgag atgatggttt tttaatattt aaacaatatc 15720
   catatttttt aatttcgtct ttagatgggg tagagttact actattgctg ttatatggct 15780
   ttccatttac aacaactttg ttaacttttt ttagtttttc gctcatgttt tatttaataa 15840
   gtatttattt atgttattag ctatgattta tgtttcattt ttaatgattt tttaaaaaaa 15900
   attatacata taaaaatgaa ccgtatttgt acttaatgtt tatttttcgt tttatagaac 15960
   ataaaccaag taaaaaaata ataaaaatta tcaatacaaa aaaaataaac caatgaacat 16020
   cgttgatgaa tggtatccta ttatcgatat aattattaat tgtatatcta ttactcattg 16080
   atttcattat tgtattcata acacaatttg cttcagaagt tccagaatta attatttgta 16140
   ataaaatgtt ttttccattt ggtgcgatac actctccaat atcgaaagtt tgaatatcaa 16200
   tcacattagt tacggcggcc gaagaagtac atttgcgtat tatttcgggt atataatttt 16260
   tatcttgatt atccaaatca atcccaactt cattcgctaa ttcatacttc tcgttttgag 16320
   atagagtatt agcaaactct ttaaatgttt ttatcaataa gtaaaaactt agttctttat 16380
   catcaataca taaatttatt atttttaatt tacacccttt aagcgttccc tttatttcac 16440
   taatgtgaat accacaagtt gtgttaactg gaacggagta taatgataaa ttttgtaaat 16500
   agcgctctat gaataaatta tacaatgtat caacttgaaa gtggttttcc atttatccac 16560
   gttgagaaaa tgtttttctt tgaaataatt ttttctaaaa aagaaatagg atgtaatatt 16620
   gatattttta acctaaattt atcacacgta tttttattac aacatataat aaattcttct 16680
   aatgtattat aaaattcttt atctgtttct atttctggat atgtttttaa aagtgtatga 16740
   atgaaaaaat gaaaatcgta gtaccaatta tgttcaactt ttatattatt ttttattttt 16800
   ttatttatta tgttcgcaac ctgtgaaaag tcaaaatcat ttaaacaagc ttttataggt 16860
   tcattaaaaa tataagtatt ttcatttaat gatattttta ttgattcgtc cgaatcaaaa 16920
   attaaaatat tatcaggttt taaatcaaca tgtaaaaagt tgttacagca tggtaattca 16980
   tatattttaa catataaaag agccatctgt aaaaaaataa attttatata ctcaactaat 17040
   gacttaaaac ctaattcaat agccattttt tcactaactt tatcagaaga gaattttgcc 17100
   aaaggaaata taataatatt tcctctatcg tataaataat tagaccgttt ttcgtgttca 17160
   aaaaaatgaa acatgtgtgt aaaatagttt attacattta cattactttg tataactata 17220
   gggtaaaaat atgataatag ttttacaaat tttacatctc cttttttctc attaaacgat 17280
   tttaaaaaat atttatgtga aaaattactt atacttaatt tttgatcatc cattgtttga 17340
   ataagtaata atatcatata tagtacccgc ttatataagt tatataaaaa tgtaagttta 17400
   aagtttacac ccattgccca cgcacacact attagttttt tttcatctcc ctttaagtta 17460
   ttaaacaaga actttggtat tgtatattct gcagttgttt caataggact atactgttta 17520
   ttaggttcat aaacaaattt tacaacatac ttatcaattt taaaaactat accatatcct 17580
   ccagttgaaa tatgataaaa gtcatcatta attggataaa atctcttatc tttttgctga 17640
   aaaaacgatg gattgatata ttcgttttca gcaattttat ttaatgtttc ttttgtaaac 17700
   tttttaaagt atcttattaa cctaacatta ggtgaccaac tttggtaaat atctaattga 17760
   gatattacat aatcaaaata aatatcatcg cctaatactg ttgtagattt tatatcttca 17820
   tcgttatccc attggcattc taatgagtta tcatctattt ccatgattac catttattgt 17880
   ataaaaagcg tttattataa tattatagtt aataataata ttttacttta tttattatat 17940
   agtcgttaat caattgataa tgttttatat tataatctaa aagtttgtct atttttaaaa 18000
   ataaatttgt gattttttcc caaagggttt catcatcgtc gatcttgtac ttttttttga 18060
   aactatacaa aaagttatta tttttattta aaatagtatc ataaataaca aagttgttag 18120
   gaatataaca attattttct ttttccatta ccttttttaa ctttataatt ttattaacta 18180
   attcgattat ttttaatctt ctaattgaaa taatatttga cgttatgttt aatgtttctt 18240
   cattattctt ttcataaaaa aatgcttttt ctgtaattga taattcttta ggagataaga 18300
   ttgtcactaa ataaattgat tcgccaccta tttgtggtaa aaaattaacc ccattaagaa 18360
   agccactcat aataaaatct ccttcttcta tatttatttt taaaaatgta gtatctggaa 18420
   taaataatcc agatattcct ttattcttaa cacaggccgt aatgacaacg ctattactaa 18480
   atgttgattc gtttaaaaaa aatgcatttc cgtgttttaa tttaaccaaa tacgatccta 18540
   taactgtaaa aaactcttca ttgagtaaaa gtttataaaa aatatttaca tttatttctt 18600
   tcaatgaata aaatcctact ttttttttac tcatatctaa gtcatttaaa aaaagattaa 18660
   aagaaaaata taactcttca cttttacttt ttaaaaaatt accaactgca acattcccac 18720
   cattaactga atataaatca tcataacatc tattatttaa aaagtacttc aaaatatatt 18780
   cttctttttt tttattactt aaacacataa tagacattat cgtatttata aatcatatat 18840
   catttcatat aatgcatttt tatctttttt aaaatcgtcc gatatatata tttctttata 18900
   attatacatt aacacatagt ctcctaaaaa atgactatca attttttgta ttaaagaatg 18960
   aatattgaat ctacagtaac taatagaaaa tgctttaaat aataataaat aattatttaa 19020
   tgtagtatta ttttcataat gctctagtaa ttcgttatta tttactatat agtctacgat 19080
   atcatcgctt aatatttctt ttatatgatc aatagttttc ataaaatcgt cgttatgaaa 19140
   tgtaatactg ttttcccatt ctaacacgcc taactccttt attttatatc cgcctatttt 19200
   tctcattaat tttatatcta ttgaggtcaa tggagtaaaa aatacagatg tttctaattc 19260
   atcatcgatt ataggaatct ttacgttatt atttttactc actttacata atgctatata 19320
   taaattgtta ttatttatat ctatacttct tttatagatg gggaaaccaa tagggtatcg 19380
   ccctaaactt attatatcta atatggattt aactttaaat ataacaaatt tttttttagg 19440
   agaataattg accgttttaa catttttaaa ccttattaac tgcataataa atcgtacagt 19500
   gtcgtgttct agtggataat acataaaatt gtttacttta gatagtgatg tttgtattat 19560
   tttagttaaa aaaatatctt ctattctatt aaaagaaaac atattaaaag gatatattaa 19620
   attatttaaa acatcatatt gtttgttgat ggaatcatat aataccaaaa ctgactctaa 19680
   tgccattttt tctaatgctt ttattttaca ttttgcttct tcttccgtga taaaatcttt 19740
   tggtataatt atcttttttt tattattcca ctgtttaaaa taatcactta tttccatttt 19800
   acacgaaaag ttagcgatat ctactaatat tagtttgaat ccgtcatctg atacaatata 19860
   atcatttcct ataaaattcc atcctatgtt ttttggccac gacgctttaa gcagtaaaaa 19920
   gtcaaaaacg ttgcttaaaa accctactaa aaatgctgtt ttaatattta ttagtattgt 19980
   ttcctttaac cattttaaaa aacataatat taactctttt gagttaaaat ttataaatga 20040
   tttaccaaaa cttgtcatca tataaattga tggacctaat ggagttatta ttgagtttat 20100
   aattataggt attattgtaa ctacttcatc atatatgtta ttttttgacc ataacctttt 20160
   cgatgcaata cttttaacat tataaggcaa tatgtctagt tccggtctat tatatttttt 20220
   tttttgttta acccatatat aatgttttcc gatgcaccct attatcattt catcaattaa 20280
   tgtataatca tcattacctt ttttactatt tttataatta acaaatctat aattagataa 20340
   tccattatcg cacatccaat aatttatctc actcaatgaa atatttttat tttcaaaaac 20400
   atgcaattta tgaacagatt tatttatttt atcgttttct aaagctcttc ttttgacaat 20460
   atctaccaaa ggagatatag atgttttagg tgaaaaaaat gtttctgtgt cgacttcgtc 20520
   aatgaataac ttgtgcatat gcatgtcaat aaaatatggt tctacatact ttgtaattcc 20580
   aactatttct aaatgagata gttttgttac agatcgcact aaatctaaat tatttaggta 20640
   tattataact ccctttccgc ttcctaatct cgctaatata atagagtgtt tatatgtttc 20700
   taatgcatta ataatattaa attcattgtt ttgtttttta aacatgtttg attacagcac 20760
   tgtatttttt ttgtctgacc aatctctttc aaatatattt gacaacatct taactaattc 20820
   attatcgttg gtattaaaac taacaaacgc atagtgtaaa taatgtgttc cgtcaaaatt 20880
   tgcaggagtt atatgtgcaa attcgttatc aataatcatc aacttggtat tattagtatc 20940
   atgaaaaatt ttaacagtta aatcattgtt actacacaaa gattgtaaac ttttaaccga 21000
   tgacataaca taaacgtcat ttttatccca agatccaacc aaaagcctaa cttttacacc 21060
   tcgattgata gttgcactga tgatagcatt gtaaatatct ggccaaaatg ttgtttttaa 21120
   atcttctctt ataacgggaa ccaacgataa caattccaaa tctattgatt tttttgctga 21180
   attaattttg ctcaacacaa catccgcatc taaagttcta gaatatccta atatatgatc 21240
   aggagaatct gacaaaaaca caccaccaat tggattatta atatggtatt ttgtactcac 21300
   tggcaaacaa catgttatat atattcctct aaaaatactg ctgttgttat ttaaagcttt 21360
   aaatgtttca aaccttcttt gtaaatctaa agctaatggc gcgtaagtag aatatatacc 21420
   taaagtcttt atattggaaa tggatccacc agttaaagag gtgttaccta tatagcattt 21480
   attataatca gaaacccaaa aacttcccaa taatactccc ggatcgttat ctcctcctat 21540
   ttttactttg acatactcaa ttttatttga tattaattcc tcttcatcct tgttaccgct 21600
   ttgatgatct actaaaattg taactttaat tccagatgct gcggcttctt ttaatttatt 21660
   taaaatattt tttccatgtt cgctagtgcg aagattacaa caaaatgatg ctatatttat 21720
   atttttttta gcttgtgaaa taatttcatc aaagcactca tgtgtgagca tatgttgtgt 21780
   tgttatccct gaggttttag gcaatgtttc aacgagtcta catcctgctc cagatggagg 21840
   ttttgaaaaa aataaggacc acatttaatg ctaaaaaaaa aatagttatt ttataacaat 21900
   gtttgttttt cttcaatatg tttgtagtta ttatctttat ttatttcttt aaacataata 21960
   actaaatcta aaattattga cactataccc actaatgcaa aaatagcacc aataaagacc 22020
   aaaaaaaatt caaacatcat ttaatagaca tgttatttat ttaaattatt ctatttgaaa 22080
   ctttgaagac caaaaatgtt ttattcttaa cccaattatt aaatatttta agtcaaaagt 22140
   tggtacaaat atatttatat ttaaactatt atcatcttta ttaatgatac aatatttaca 22200
   tattgttttg ttttttgttg cctcgtcaat acattttaaa acaaaacttg gtaataagta 22260
   ttgacattga ggttgttgaa aatgaaatgg tgaatatagt aactcagtta atgtaaacct 22320
   ataacctttt ataataacag aatctgatgt agctacggaa tttgtaggaa agtgttttac 22380
   tgtatccatc ttgacaaagc atctatttcc aagtaagcaa ttatcatctt tattgatttt 22440
   tattctgtcc atatttctaa atggacttaa tttaattatt tcatctaact tgttaacatt 22500
   catggttgaa aatgttaatg atgttagatt tatcttaaaa aaaacgaatg ctttttcatt 22560
   tttgatcgca tacacttcta cactgttata tttttaaagg atgtacatat atcgtttaca 22620
   taatggatgt tatctaaatc ttttatttta tttacaaatt tattataata ataatttttt 22680
   tcctttatat tttttttact ttttcttgga tgcaatgctt tccttaacca taacgaatca 22740
   caattaattg catccattaa tattttaaaa gtttcaaagt tatcgtaaat aaatttcttt 22800
   atgatatcat cactacatgt attcatatta attaaaacaa acaaatcttt gagctttttt 22860
   cttgtattaa ttttgctatc aagattaata tgtctaaatg atggggttat cccatcaaac 22920
   aataaattat ctttaacata atatatcgtt aaggatttat ttctttttct cattaaaaag 22980
   ttatataaat gatcatttga aacagatagt tgttttttct ggtaaaatac caatatatca 23040
   attttattat catgagatat acactcccat ctttttgaag atataacttt ttcatctata 23100
   taccctacct cttttagctc aatttttata gatgaattta ttaaattgca atagtttcta 23160
   catattttta tttgataatc tatagatgta tcaaataacg atattaaact tgttactata 23220
   gctgcatttt ttaacatttt ttaaaaaaca aaggtgattt tttatacttt tttataatag 23280
   tttttcattt atgttatagt aatataaatg gcagcatata attcacaata ttatgcaaac 23340
   acaccatttt atataacgac aaaagaagga aaatatcttg tattaaaagc aattaaagtt 23400
   tgcgatataa gaacagttga atgtgatggc gataaagcat catgtatttt aaaagtggaa 23460
   aaatcaaatc aaacttgtga caggccatct tccccatgtg aacgagctgc aagagcttca 23520
   tccccaaata gagggaataa ctctgtacca tttatgcgca caaatatgtt agaggacctt 23580
   caagcgaaaa atcgtagcgt tatgtctaga atacttggtt aaattttaat gcgttataat 23640
   atctttttct atatcaatta taccatgttt accacttttc ttatctctag gtataatatc 23700
   ataaattggg tttttttcta ccccaattaa tgagtttacg atatctgtaa gtggttttat 23760
   tatatctcct ttagttaaaa tttgataaat taatatatgt gcgcttaatc ctctggaact 23820
   tatctcatga atatcggttt ttgatttcat tagtattgta tttgaaaaat aagtatataa 23880
   aatattatta tttatcaaga atgcagaatt tgaaacattt tcaaaatcaa tagcatcgtc 23940
   agcatttaaa tctaatatat cactagataa tgaactttca tctaaataaa ctaaacattt 24000
   tttgaatggg aaatcgtaat tgctagtatc gaccgtgatg atacgtttat caatgtctat 24060
   aactgaatac ataggcatgt tatttttttt tgtttggtta ctgataaagt ttataccata 24120
   gttaactcta acatactcta ataatgtagc taatctaact gttagttttt caggattttt 24180
   agctaaatct tctaatctgt ctatttgtga aaacattttt aacattgaca taagttgcaa 24240
   tgttggatct actatatata tattatcaat taaaactttt ggtattgatt gcatggtatc 24300
   ttgtcgtata ttaaaactat ctattatatg gctatcgttt tgatccttta acaccatata 24360
   gttttttaag tatggtactt ttaataaaac aacatttaag ccagtaataa acttgattaa 24420
   aaacgcaaga tctataagaa aagttctaga gtttgtttgt aatatatcta tatctccata 24480
   tttaacatcc ggatttaata aatgtagaga atatgatcca taacagatac aatttttatt 24540
   atgtcgtcgc aaatactctt ccattaaaga gtttacatta tcaactaacg atgaaacatt 24600
   atgtctaccc atggttttat ccgtcatctg tgcaacgttc ataaaattta aagcatccct 24660
   agctaaagat tgcatcgatg atgcattata gtaaatatta gaagtaaata aatcaggccc 24720
   tttagttgtc aatatcccta atacatcagt atatgttgta actaatatac tttgtaattc 24780
   tattatggta tataacttac caattgagta tgtgttctct tgtttagaaa agtaagtaag 24840
   aattgatctt tttatatcag atggagaaga cttgatatca gaaaaaaatt tctttttatt 24900
   cttttttatc aaagatataa atatttcttt attaaaaatg tttactcttt gtatgttttt 24960
   atattgtgac ttcaacatgt gaaactcggt taaagatgga tctcttttta aatacaattt 25020
   taaaacatct acaacaattt tatcattcat ttttgtttat tcaatatgat gaagatacaa 25080
   actttttcac aaaaaagtag tgtatttttt aaataatttt tttttattat tacaaatcca 25140
   ctatcgcata tatatttttt aaaaagattt aattctttac aaacgctaga tatgtcttca 25200
   gtaagtgttt catttatttt cattcctgta aagaaattac ttataagtaa agatataaaa 25260
   acattagttc ctctactaga atacgttgac cctactacta agtataaaaa aagaatttga 25320
   ataaatgaat ttattataga gtggaaattg gatatgggtt catttagtga ataaatatta 25380
   gttatataat tgcctaaaaa tttatattcg ataaaatcta caggcgatat cttaaaaata 25440
   acgccgtgct ctatctttga agggtgtgaa tatttagatc cttttattat atcaattata 25500
   ggaacccagg aaggaaaata tctatatggt aaatatacca aaccatatgt cgttaactta 25560
   gcaattgtaa atacttgatt taaaatatct tcttctgttt ttaaaataat atcattttct 25620
   ttatttattt gtatttttat tccagtgcta tcttcaatat aagacatatt tttaaaaacg 25680
   gtcatatcga tttctggtat attactatat aatattaatg attttatttt attttgatca 25740
   tatataaaca tactattttt atttggcaat tttttaaatg gtattaagtt ttttatatca 25800
   ctaacctttt taaggataaa aataacatta ttttcgctgt gtgtgcagga tagataaaat 25860
   aacgacggtt gaattttata gctaaaatat ttatttatat tattagatga aaatatattt 25920
   agtaaataca tcgtagatgt tttatttaat ggataatcaa acattaactt gttcatttta 25980
   tgagagcaaa atccatattt ttggacaaaa atatctatta gcttattatt tttattatac 26040
   caatcagtac taaatgtgta atccgaaacc aagtttgcgt ttttttctaa taatacattt 26100
   atatcaattg tatctagtag ctcaactaaa tcttcgtatc ccatttcaat acacactatc 26160
   ataactaacg aactatgtat tggaatatct ttgattttaa ctttaatatt tgattcacaa 26220
   actattttta tacataattc ttctgttaat atatttaatg gtattttatc tataatagta 26280
   gaaagcaaat tgatataatt tttttctatt aagtaattga taatatacat tttattgtta 26340
   taatcataat tatcaatata tttacagcat atttttaaaa aatcttttgt caattcatat 26400
   tttgtaaaat ctattaatgg atctttaaaa aaaaaactat aatataatcc taattcatct 26460
   tttttatcgt ataaaaattc tatagcatag gttttatatt gatcaacttc tccatttttt 26520
   attttagtta tgataaattt ttttattctg tcatctttta attgttcaga cgttaacata 26580
   tctaaaaatg ttaaaatatc tttatgaaat tttatagatt gtaaaacatc caagataagt 26640
   tcaatagaaa ccacgcttaa aattccatca ttgtttggta ttattccata tttatataac 26700
   atatccatta aaaatttact ttcaaaggat tgatgttgat ataaactatc acataaacta 26760
   aagtcattaa taaaaatttt ttctgccaat gactcactaa acaaatagtt tatattttta 26820
   atactttccg aatttatttt agttagtata ttttctactt cgatgttaga aaatctgtgt 26880
   attagacaaa aaatatcatc atctgtaata accatatact gaatacattt cagtattatt 26940
   cttttactac attttttaat aatatctttt ttatgaaaat ttatatgttc tttgtataac 27000
   tcaatatttt tttgattata aactaaagct atcaaatcaa caaatggtac agtttttggt 27060
   ttgaataaat atattttact aggaaattct tttcttatcc taaaatacca tttttgtggc 27120
   aaattattgg ggttaattcc attactgagc attttcatag catcatctat atttactttt 27180
   ttatatgatt ctgttaaatt atcacaatta tataaaataa atgcttcctt taacggaatt 27240
   aaaaaattaa acatattatt gtaaatttaa aatttgataa tagatgtatt gattagaaaa 27300
   tcaatagttc gtttcgtcga tttttgttta gcttcttttt ttgtattacc aatttctgga 27360
   aaaaatttta taccgcttat aataacagag gccaaaaaca ttggttttct accatttcca 27420
   caactagata tatttatgta ccaatctcta gatgtaaact gacaatattc gtttatcgca 27480
   gaacaaggat ttttttcttt ccataaaaca attttatagt acggaattgt atcagaaaat 27540
   gatttattta gttttttcgt atcgttattc tcattgtcat cgttatcgat gccatttttt 27600
   ttaaaccatt taggtggatt cgatcttatc ataaaaataa aaccgtcatt atgtagttta 27660
   tacagctgtt tgtttacatt tgatttttca atatttagtt ttctagatat ttctattgct 27720
   gttataaact cactttctga tagattattt accatttttt tcacaagttc atacgaatct 27780
   acttcatcac aagaatacat ttttattttt gttgtattta aaactaaaaa attttttttt 27840
   tttcattttt atagtttatg ttatttttct acagctctaa actttggagg tttaaaattt 27900
   ttcttgcagt ctctacaaga atgcataact aatggtggtt catctgctgc tcttgtttgt 27960
   atcatgacgg gaatagtgtt tttacttttg cactcagggc atgggaggtt atacttttta 28020
   tctaaaacat taaaatattc ttcgtggttt atatctctaa tattgtctat attgaattct 28080
   acattttttt gaacacattt tattccaaaa agtatatatc ttaaaatttc tttctctgtt 28140
   ccatttgtcg tacgtataag atcacatatg tcagaataat ctttatttgt ctctattaaa 28200
   ggcttgtttc tatacgataa tttatttttt gaatcttttg agtattcaat acctatattg 28260
   tttcttggtt caaattttgt ttcttctata tttgactttg tattaataat atttcttata 28320
   taaaacttgg aagctttgtc tgttgcccat tttaatattt cttctacctt tgatttttct 28380
   tttacatatt tataaattat atcttctatg tcttgatatg tgggggtagt attatcacta 28440
   tatgaattag tattatcatc atccatattt gtactgaagt tgtttaattt caatatttac 28500
   aaaaatgaat ttttagttct ttatttagat ttttccatta atggcaaaaa tggaaaatat 28560
   tacaccacct aatcactttg aaaaagcttc tgtttatgca tgtaggttat ttaaacagtt 28620
   ttataacgaa aaaaaatatg atttagatga atgtttttta aaagttagaa atagcatgtc 28680
   tcgatactat tctatggttg gtgatgattt agtttggaac agagaaatat taagttactt 28740
   atatgaatac atagcatata aaaataatgc aaataatcca aattttgttc tatcttcaaa 28800
   aattgttaat ttaactaaga aaaatacaaa aaagataaaa aatgaaataa attctgttgt 28860
   tcaaacttac aaattggata aacatataaa aaaagtaaat ataacacact atgattatat 28920
   aaaaaataaa aaaacgtatg atacattcaa aaaaaatgtt gatattaaat caaatgatat 28980
   atctgtaaca ctattaaaaa tttataaaca actaagctta attagtgatg tttcgataaa 29040
   tacacgttct atgacaaaat ctttaatgta ttacatgttt cctgatctct ttaaaaagga 29100
   taaacgacat gtattttaca gagtagacgc taaaaaagga tattgtgtat tatcatctaa 29160
   aaagataaac ttaattaggg ttttattaga atataaattt gatataaaat acaaagagtg 29220
   gggattaata aaaagatata ttaatactat ttgtaataaa ggtagtaaaa gtagcaaaat 29280
   ctgtattgga aaatatccat atttaaagtt aaaacctgtt tctaaaatta aatttaatta 29340
   tagatctaaa tacgtaagtg attttaagag aatagtgagt tctagttttt cattggttga 29400
   tttgacagaa aaaataacga aaaaaacttt tagtaatatt tttagaaatg aaattagtca 29460
   tctgtataag tttaatgcag aaaaaaacca tctcgcgcta gatgaataca aaaaaatgag 29520
   aatgtgtaat aaaataatat ctgctataga ttatccaaat aaagatcaca tttatagtgc 29580
   gataatagaa actgttaaca attacttaga taatcctccg aaaaagctag gatacaatat 29640
   tgatacttgt ttgactatag caaaaaacaa ttaacaacta atcatggatt ttatacgaag 29700
   aaagtattta atatatacaa tagaaaacaa agttgatttt ttaaaagatg aagtactaac 29760
   aaagatatca aactttacat taaatcatat attagccatt aaatatttaa ttcttaactt 29820
   tcctagagat gttatatcaa aagatgtatt atcgaaccca aatttttatg tttttttaca 29880
   tatggtaaag tgtgatgaca tttatgagac tgttttaaaa cattcgttcg atattcctac 29940
   gttatatata aaagcactta taaaaaatta tccgttattt aataacacaa tagaaagata 30000
   taaaacttta gttagtgaac tttttttaga cgataaattt atcgaaatag taaaatattc 30060
   gtctgaattt gataatatca tagctgttaa ttacgatctt ttactaaatc ctttattcca 30120
   taataatgaa ccgattaaaa atatggaaat tatttatagt aaacttttta aaaaatctaa 30180
   ttttaggaag gtaaaaaaaa tggaggttat cagattaatg atatgggcgt acctaagtaa 30240
   acaagatact ggattagatt ttactgattt tgactctcaa gatatataca cactgtttca 30300
   aaaaagtgat aaagttatta ttcatagtga tatgacagaa aagtttaaag agtatatttt 30360
   cgggtcggaa aaaactagtt attggttatg gttaaatgaa tcaatattta atgataatgt 30420
   ctacaatgaa gggtttgcag aattaatgta cgataaaatt cttagtttta tctattcaga 30480
   gattaaacaa ggaagagtaa ataagaatat gctgaaatta gtctatatat ttgaaaatga 30540
   tgaatatata cagtctattt tattacaaat aatttacggg atacctggtg atatcttatc 30600
   aattatagat gtaaaggatg ataactggaa aaaatatttt ataggttttt ataaagaaaa 30660
   ttttatcgat ggaaaaactt ttataagtgc aaaaacattc aactatgatt tatttaaagt 30720
   agtggctaaa ataaatcctg agtactttga cccagacaaa ataatatcca tatttgatca 30780
   taaacctgaa aaagttaaat attttgatac aatagatata aataatacat ttatatcaaa 30840
   tataatttat gaaacgaatg aattaaatct ttcaacgatt gaagaattac aatcatgcca 30900
   aatatataat gaagaaacag aatatttcat taaagaatat aatacttatc tttatttaaa 30960
   agagaaggat ccatatattt tgtataatgg catgttaaca aatatatcaa aagttccatc 31020
   aaataaaaag ttttcattgt ttagtaaaaa catattaaaa tattatatag atggaaaatt 31080
   ggcaaatata ggtcttgtgt taccaaatta taaaggtgat atattggtaa aaatattatc 31140
   acatttgaaa tgtgtagagg acgttactgt tttcattaaa ttttcagtat gtaaaaactc 31200
   tagcatacta ccatcaataa taagaacaat tttagcaaac tttaatattt caataataat 31260
   attatttcaa aagtttttaa gggaaaactt attttacgtg gaatcttttt tagaaaaaac 31320
   aaatcattta actaataacg ataaaaagta tatactagaa ataataactc gtggaagatc 31380
   ttaatttaaa atgatgggag acatttattc agagtcacaa aaaagaaacg acgatcaaaa 31440
   aacatacttt acaagaaatt taagtccttt aatgaaaaat acatatttat accataatta 31500
   ttcatatgga tggataccgg aaacagctat ttggagtagt agatttgcaa atttagacgt 31560
   aacagagttt tacccaatta caataaattt aataaaaaaa tttgaattta tgctttcatt 31620
   atacgatggc cccatattac catatgaaga aaaaattaat acagaattta tttcaagagg 31680
   ttctttttct ggaagatata taaatttttt aagaaatttt tcattactac caactagtga 31740
   attcatttca tttttattat taacatctat accaatatat aatattcttt tttggtttaa 31800
   aaatactcaa tttgatatta aaaaacatac actttttagc gaagtttata ccaataacga 31860
   taaacatata gaactaggaa aatattttag acaaaccggt gattataagc ctttatttag 31920
   caggctaaag gataataata tatatacttt gccgtttcca atcaaccaaa gtaacattaa 31980
   caacccaaca ttacctattg gcttttccaa atcagattat gaaacattat ctaatttaag 32040
   cgcaatactt tatttaacta attatgatcc tgttttgatg ttcttagtgt tttatgtgcc 32100
   taaaatatct ataacgacaa aaataacacc agctgttaaa tatttaatgg ataaactaaa 32160
   tatgaatgaa acggatataa cattagttta attataaaaa ataggtctag ttccgaacat 32220
   tttttcaaaa aatgatatgg ataatacttt gttatctaac aaattcacta tttcagtggc 32280
   aagcctttta aagtatactt cgtaaaatat tcgttcagta gattttaatt taaaactcct 32340
   atctataatt ctttcatacg tttttatatt tactaatttt ttttgccatg gttctttttt 32400
   attgcatatg tatgcaaagt aatatcgttc acctatttca ataatttcat cattattttt 32460
   attatattca tttactaagt acatatttgg attatcactt gatttataat tacaatgatg 32520
   tgttctactc aatagaaaca tatccaatgg agctgttttt gtttcaaatt ctatttgtaa 32580
   atcactttct aacgatgata aaatttcaac acatacttgt acagatgaca tatttccacc 32640
   tgataacatt tgcaaaagcc ttgttttgta tattcttatc atatatttgt gaaacttaga 32700
   tacgtctctc ctcgtctcac tagttccctt attgatacgt tcgggaattg attcattggt 32760
   gaaagat 32767

<210> 5
<211> 32767
<212> DNA
<213> Artificial sequence

<220>
<223> nLSDVSODis-UCT (no marker) full genome sequence

<400> 5
   aagccaatta aacctaatta agtggaagcc aattaagtgg aagccaatta aacctaatta 60
   agtggaagcc aattaaacct aattaagtgg aagccaatta agtggaagcc aattaaacct 120
   gtaaatggat acttttttca ttcaatcttt taagtctaat cattagtgct gatttcacta 180
   gcgaaatatc gttgtcggaa acgaggtttc gaagcaatac caacactttc acagaagaac 240
   aagttggaga tgattcacca ccccaatatt ctgctgctct tgctaaaatg ccaatcactg 300
   cacatgattc cctaatgtaa ttttcttttt tcaacatgga attaatcata atttttgatt 360
   gttcaaatcc aattttagaa tccaaaaaca tgtttttgac aaaagctgtt agatcatttc 420
   caaatacaag tgaggcatcc tttttgaaag attcaaaaac taagaacacc tttccagcaa 480
   cctccctgga ggaaaatgcc aatgatgacc aaaacaaata atcagagatg gcggttgtga 540
   tatcatcatc atctgaaaag ttgtttcggt agacatagtt gccggaagac atcttgtaaa 600
   atgaacggga aaaaagtctc ttttaacccg aacttgttat ttcctgtgct tatttttcaa 660
   aattattcaa ccatattcat catcctttca actaatcctt ttctaggtgg ttcctttatt 720
   gtgttttgat tacagtagta ttcgtagtgt tttttaaacg gattaggaat tttaataaaa 780
   tcaaaaactc cattatcatt tctaatattt atatcgcttg tatctttttt atctaataca 840
   gttaatggca gatcattatt tatttcttta ctttctatag gtttgtgatt agtgtttata 900
   ttgtcttgga ttttttcatc cttatccaag acagaatcga acggatttag gtttccaaac 960
   atgaaggaga taagcttttg cattggaaac attaataatg aatacaaact atatataatt 1020
   aaattacata atctagctat ataaaaaata cacaacatac aaactttagc taagctatta 1080
   atgagtagtt gacaaaatcc ttttattgca tatatcacaa atcctaaaat ctttagtata 1140
   agaactaatg tccctcttac acatctccat atactaaaga ttattttatg aattaatgca 1200
   aaaatccacc agactaaaaa aaacggaaaa ctcctgtttc ttattcataa tttatatatg 1260
   tgaaaaagaa caaaataaat attatatcgt cttttgtata acaatcctat agttcattat 1320
   gatgaaatat ttttttaata taattatgct gatgttcata atattttttt ctatcaaatt 1380
   taccaacact gcacgttttt aaataattta cgtgttcgct aaattttaca gctgagcaat 1440
   ctattttatt attaaaccca aaaatctttt ttcctgtaga tatatatttt ttttcacatt 1500
   catttctaac tataacactt gaacttacat gttttacaca actaatatct gttgttataa 1560
   caaattctaa atcataatct ttaaaactta cactctcaac gtatgatttc atttctgtag 1620
   ttacttgatc acaagaaatt gacaatgata ttctcttagc acactgtggc agcgaataca 1680
   gagttgttat attactggtt gatatttcca tatatacata tttacccaat gaatagactg 1740
   cttcgttaac agtatctaat ataaagttgt tttgtgtttc ttctttggaa caatcatcat 1800
   gtgcgaaaat tttaaatcca tttccctctg ttagaagtcc tccaggtcct tgaattttat 1860
   aacagccagt tttaatcgct ctaaaattct ctgtttggat acataatcct acttcgattt 1920
   cccatgtgtt attttcttct tctgtgcatc tctttattga tattccgctt acactactag 1980
   taaaaatgaa tagttgcaaa aagaaaatga acaaagttaa tgatgacata ttggttggtt 2040
   gttttgacgt attaccgaca caaaccacct cgatactact gaactttttt ttctattatt 2100
   aactaaaaaa acttgaaaac aaacctttct ttttttcctc tttcttaaaa ggatttatat 2160
   atgatagaaa actgaaactt tttttctttt gcctaaaggg gtttatatta aacgaaaaca 2220
   gattttttgt cgatgaggta atcatgttat aaggattcat taaccctatc attgcccaaa 2280
   aaaacttcaa aaataattat atagagtagt atttaccacc aacatgaaaa caaacacaaa 2340
   aataatacta ttctgttatg ttatttttct tagtttgtat gtatttagtt gtgtagtagc 2400
   atcggctaaa aaatgtgacg acgttagctt tgattacata ttaaaagatt tacgttcaga 2460
   atttagcaaa ataaaaagtt ttgttcaaga caatgatcaa gaaaatatga tgttattaag 2520
   tcaatcaatg ctggacaagt tgacgagtcg cataggatgt aaatctttat cggatatgat 2580
   aaaattttat ttaaatgatg ttttaccaaa tgcagaaaaa atagaacaca tgaaaaataa 2640
   aataacttca ataggagaaa aattaaaatc gttaaaagaa aaactcatat cgtgtgattt 2700
   tttacattgt gaaaatcatg acgaaataaa aacagttaaa acaattttta acaaattaaa 2760
   agataaaggt atttataagg ctatgggaga gtttgatatt ttcattaatt acttagaaaa 2820
   gtacatagtt aaaaagtaat caaatctata cttataaaac tacaacatta tagtttcttt 2880
   ttatgtcata gttaatatta tctttgttta ttcttaagtt acatgtataa ataccactat 2940
   catcatgtgt tgtgtttttt ataaccaact taagttcgtt actttgtatc ctatcatcgt 3000
   atagcacttt gttattattt ttataccaat ctagtgtctt atttttaaaa taactactaa 3060
   taaggtcaat atccatacaa tgtactgtcg aattaagtcc ttcattgaat ctgtaagtaa 3120
   ttgtctcttc tttatccaaa aaagttaatt ttatagacat ttcttcacaa atattatgtg 3180
   aacgaaatct acaaatataa attccgtcat cgctatttat aacagggtac aaccataagt 3240
   tgtttcctat aagttgtacc cttgcgtttt cattatttac ttctattttt ttaaaatttt 3300
   tgtaatcgtt ataatttctt acaaacgata gagagttagt ttgaaaccat gttatgtcat 3360
   ataatgttat atttttttta aaaaaaatag attttttata attatccaat gggcatttta 3420
   aaaatgccgg atctttgaaa tttacattca ctggagttag aataccgcct ctatatttac 3480
   aatattctga taaaaatgcg ctttttaaac aacatattaa tatgataaaa tttataactt 3540
   tcattttgtt gttgtgttat ttattttaaa tagaaaaata gcatcggact acgttaaaat 3600
   ttatataatg tatttcagaa ttacagttcc cagttaatac ataataattt attttatctt 3660
   ttaaattatt atttacgtac ttaactaagt cttttaaaaa atcaaaatcg ttttttgatt 3720
   ttggatacca tatttcatca catgacgaat ctaaggcttt ccatgctata ttttttccat 3780
   cttcgttttt taagtaatca aaaataatat cattataact aaatgatatt tccatacaca 3840
   tatcgtcaaa attttcaaat attaattttt ccgttattat atcatcatac ttgtttacca 3900
   aaacacattt acctgatcta tttaaaatta atccaaatgt atctacttct gtggaataat 3960
   tatttacggt aacaagacag taacaaaact tcctatttcc ttcgtcttcg taaaagttta 4020
   tatgattatt ttccgtcaaa tagggtaatt taaatatggt ttcgtcatta tttgaattat 4080
   attttataac tacatttatt aatgctatat ttttttctcc atcagttata atatcagaac 4140
   tgtgatttaa tgttttatca aataggacat cgtttttagt agaaaatata gtattgggtt 4200
   ttatatgtat atttgtatta cctcctttgc tcgtattatt taaatacagt actaaatgat 4260
   aacaataaca gttttttgat ttgaaatgta taaaatctct gtgtgtgttg aaaaaatctc 4320
   ctttttcgta ctttataagc gtaattgtat tatctaatat aatatcgcta attttttgtg 4380
   gaaattcaaa agataataac agtttttttc ttataacttt aaaaatatca tctaaacctc 4440
   ttattatata ctgtttacat tttctttcat tagtattaat aacttctata cctttttcat 4500
   gatcaaatac tttagactct tcccatatta tgtttttgta tttaatcatg tccataatta 4560
   tttttttaac gttattaaaa taatcatcat taaaggtttt tacttttatt ataccatcac 4620
   taatcgtttc catcttcaaa aaatattatt tatatttaat attttttaaa aactaatgtt 4680
   aaggggtttt tttttatgtt ttataaaaat agtatgttaa ttcaattcat tgacgatata 4740
   ctcaaaaagc gataacacaa gattgtaaat tcctacaatt ttattaaatg atttttctcc 4800
   tttgaattta tttttagtaa ctttctgaat tcgccgttta agcaaactta aatctgtttt 4860
   ttgttttaca agaaatgtat caactttcct aggcgttaat tcgtcaatac atgctttact 4920
   tgtttcattt gtccttattt gataataatc agtttcgtaa tttccaaaaa ccgtaacaca 4980
   aactttttct tgagaaccaa attcaatagc acatcccttt ttatcacaaa attgttcatc 5040
   aacttcatat ttaattgtat ttgaatcacc aaacgtaata cttaaaatat atttaaacac 5100
   aatattacaa tacttatcgt tattatatat aggagaattt ttattattat ccttggctag 5160
   tgggtgatta atcgttagta cagtattaac atcttgtcgt ttaataaaca cagatggagt 5220
   agtaataaca acactttcac aaattggttt aaacgtaaaa acgttcgatt ctgtattatc 5280
   gactgtaata aattttatcc aaaaatcgtc agtattatca tctataaaag gtgaaacatt 5340
   acaatatttt tgaatagtat aattacatgc ttgtttccat tctccactgc tataagtcat 5400
   aatcattacc ctatagttac tatcgctgac atcgttagtc cactcgattt tagaattaaa 5460
   atcattagat gttagtttta tatctttagg aaaagaataa catagtttaa agagcaaaaa 5520
   agcaaaaaat actactgcgc ccatttttta gtaagtattt agttatattt tatatttttt 5580
   atggaaatct atcacagata tatttttcaa attcttttct cttaaaatga cttatttcgt 5640
   caaaaaatat gtaaaagctt ggatacttac atgtatcatg tgaagaaatt attgatatta 5700
   aacctattat atatttcatt gtggttatgt caatcacatt attttcattt ttttcataaa 5760
   gcgaaaaaca gtaacgtgtt ttacttaaac gcaattttga tattatttta ttaatatatt 5820
   caacaccgta gtgattaacg gccatagcat catatgttaa aaactcagca tatattttac 5880
   ctattttttt tgtatttttc atttttcgta ccataaaata attttccatc atatcgttaa 5940
   aatttacttt tttcatggaa ttaatatgct taaaactttt agattttata aatggataaa 6000
   aatcagcata gtattcacca tcaattttta attcttttat aactttttta taatcatttt 6060
   cattaagtac atattctgat ataactttta tcatagtaaa cgtaaattta tttgaatttt 6120
   ttaatacaat cttcgttatg tcatccttcg tataatcata atttgaaaga taaaaaactt 6180
   tatttaaaaa atattttacc tttttttcat tgatagaaaa aagaaaggtt atatacttag 6240
   taaagatttt aaatttattt tttgtataca taatgcataa cgattaacta tttttaaaaa 6300
   gtgtaatatt ttttttcatt tttatatata ctcataaacc tgatatgttg tgttatcact 6360
   cttcgcagca agataataat cgtacaatgc tattagtata tgtactacag ttataaatcc 6420
   cacaacacaa ggaagggaat atgccatcgc ggatattagt gcaataagat cacgattttc 6480
   aatattttct aacatacttt tcaaattaaa cttaactaaa agtatggtta gtggtaaaag 6540
   tagtaaacat attaaagaaa cgcaaatagc tggcacgtta caatacataa acgaacattt 6600
   ccatcttaaa caactctttt tatttttttt aatattgtat tttccattac atattttaca 6660
   ttttgtatta tgtgaaaaat taatccattc ttctaaacag tttttatgaa ctatcttaaa 6720
   ttcattcttg caattacaaa agtttgtact tacattatat tcatctttac aaatccaaca 6780
   atgagtgttg gtattatcac tcccttccat ttttataaaa tatcattatt tgttgttatt 6840
   atttttttat tttttatcca atgctaatac taccagcact actagtgcta cgcaatcgta 6900
   aaagcttttt agtaaattct ctactacaaa acgcataaat tagtggatta ataaaacaat 6960
   gacatagaga cacaatttca gctacatgaa ctgcaaggtt gacaaatcgt aatgccgtac 7020
   atccactaaa aacatttaac aaatacaacg atgaaacaaa tacagttaca ctaaatggga 7080
   gtaaaaacaa tactgaacag ataacaatca aaaacaccat ctttatggct ttcttattct 7140
   ttgtttgcga ggtttttaaa gtatttaaga ttttatagta acaatatagc aaaataatta 7200
   gcggtataat cattccaaat atgtttattt caaaatttat aaataatttc caaatttttg 7260
   cattatcgtt ataaaataca tgacaatacg ttattccata tacttttttt gtttcataaa 7320
   ataacattat tggaaaggat tcaatagttg aaacaatcca aaccaccata ctaagtacaa 7380
   ttccatatcg ttttgtcctt atcggcattg attttactgg gtgaactaca gctaggtatc 7440
   tatcgatact catcaatgtt ataaatgaca tactattgta aaaaccaaca aagtaaaaca 7500
   tagctttgaa tttacacaaa caatctccta aactccattg tttagcgata ctatcgtata 7560
   aattaaaagg aaacaccaac acgaaaatta aatcagacaa tgtcagatta agcaaaaata 7620
   tatcctgtat tgtttttatc ttatatttac gaagaacagt taacacaatt atatttccaa 7680
   ataatccaag aaagaatata gtcgaatata aagtaatcag tccaaaactt gtagtatcca 7740
   caccatcatc acaatgtggg atatcgacta tattcacttc ataatcatca taatcatcgc 7800
   tataataagt tgtattatat gcggttgtat aattagatat tgttgtcgta ttttcataag 7860
   ttgaaggcgt tgtaacatta ttttgatttg ttgaaattgt actgagaatt gtgctaataa 7920
   ttgtagtagc tatagtggta atattactgc tactattata catggtggcg ctactaactg 7980
   tgctaagagt ataattcatt tttgttggag ttgtgcttta cttaatcata cacttttatt 8040
   ttattttttt tttttaataa aaacacagta ttattgtatc atcatttatt tttttaaaaa 8100
   aactttttat tgtttgtcaa aaaaggtgag atttctaaat ttaattaaat ccttacaatt 8160
   gtaacctgac gtattagttt catcaatatc aataccaaga ctagttagat aatcaaatat 8220
   ctttttatca gaatgaaaca aaatataact atacaatata ttattattat caaaatcttt 8280
   ttgcaaaaaa ttaactccac tatctattag taactttatt acatttatac gaacattaaa 8340
   attacacatg tacatgtgta acaaattttt cccattttca tcttcttctg ttacagatga 8400
   tccagaatca attaatattt taactatttc aggttcgaca ttcttgttaa aagaaagata 8460
   atgagctagt gctgataaat tattatctct agttttaaaa tttatatcgg atccattttc 8520
   tatgagaaat tttaatattt caacattaac tttatctttt tcgacacacg aaaacaatgg 8580
   cgtttcatat aagtcgttac aatcattaac aaattttatc catttcttta ctccatttat 8640
   atcatcttgt tcaacgtaat aaaataatgg attacaatat ctataaaata agtaatctgt 8700
   aaagtcatta tcataatcac tacataattt ttccttttcc atagtaataa ttgatgatgg 8760
   caaactagct tattaataaa aagttatatt tcatttttgt aaagtgtttt taaatttaca 8820
   tatttaattt tttttgtgag atagttgtat tctaagttat gtaagttaca tgtgaaatta 8880
   gcagatagat attttctttt aacttctttt ataattaatg caattttata agagtttcct 8940
   acgtacattt ctctacctaa gtaaatattt ttacttctat cgtaatcaag tacattaact 9000
   ccgttaatta accaaaatat tgaacttcta taaacacgtt gtatgttttt agattctcca 9060
   atacattcca tcaaaaagtt tttacctaaa gctattgtac tatttaaaaa atttactgtt 9120
   aaatttatat catctttaaa tcttttttta attacgttat tatgataata acgttttaat 9180
   gccaagtttt ttggattaca ccgtgtagca gtagttatgg aacataaata tactccttca 9240
   tcactcttag aaatatttcg tattagtaat ttttctttgc tttttttata tttaaaatta 9300
   ttgtttattt tttccctgtc tttaaaccaa tttacttcat aagaacaatt aaacgtattt 9360
   ataaagtttt ttgtatttat acacaacaac tcatagtttg taccaatgac agagtttata 9420
   gtctcttttt tataaatatc taacataaca gatgaagtaa aataatatgt tgagttatgt 9480
   acattacatg catatattcc agaatcattt tttgttatat tatttatcca caggctatgt 9540
   ccaacagaat atactctatc ggtatttttg aactcatgaa aagatttttt tacatcccct 9600
   gttttaacgt attcattgta attaactaaa aaccatttta ctgttgtatt ataatttaaa 9660
   gtatcaacat atggacattt tactatttct gagttatttt caaacgtttt catcacggat 9720
   tgtagataac caaagcttaa ttttgtaaaa gcaagaattt taacattgat aatcagtaaa 9780
   tagaaaataa atgtcgttac tttttccatc ttatccaata agattttaat cttacaagat 9840
   ttaaaaaaaa atattttttt ttttaatttt tagttgttat tataaacttt tacatcaata 9900
   tagcccttta ttttgttcat ccttataatt tgcacattaa ttaatttatt aactaatgtt 9960
   ttttttatat tattcatttg atccatgttt atattaacat aatctattac aagtgcttcc 10020
   aaaccatagt tgattagttt tacatataca atgttatctt ttataaacac aataccatct 10080
   gttacttcat taatgttagg taaaacgtaa caaaatgcca aatcgctatt cgatgacatt 10140
   tttgctctca ctttctgaaa cttttttttc agaaataccg tttttgtgct cgcatgtatt 10200
   atttaaacaa ttccatatat cgttcaatat taaagttttt ttaattggtt cctccaaatc 10260
   cattagaaca catgttaagt tagtattttt aatttcttca gttacgtttt taattatcaa 10320
   atcagatcta ggttctttgc cacattttaa aggttttttg ttaaaataca ttcgttcttt 10380
   ataaatgtcg ccgttcataa attcaacaaa cgaattatta tttcctatca gccaatacac 10440
   atatgcataa ttgctatgat gactatatcc tttacattgt agtaaaacgt tttcgccctc 10500
   tttatgagga aagtaaataa ctaaatccct tttttttgca catttactat aaaccacata 10560
   aaaggttgta aataatgtca gacaaatgac tactttcttg aaagagatca ttttttattt 10620
   tttagaacta tattttatat ttttttttaa aaataatgaa acaaacttct taaatgacat 10680
   ctaacgccct catatcctaa tttgcatctg caaaacatta gtggtttttt attgtaagat 10740
   aaaataaaag ttgttttata acatgtacca ccatttaaac aaaaatttat acttttatca 10800
   cacatcatta tatctttgtt taagatagtt atgtaatcgt ttttattttc tttagaaagc 10860
   attaatataa ttttatagaa taacaaaacg gtaaaaaata ttttttttac ttccatcgta 10920
   tcctaccaat tattttaagc aaataatatg taataataat acttcctatc attacgaaaa 10980
   agttaattat tgaaaatata aaatcatttt gttcatcgat ttttttattt ttttcaacaa 11040
   aacttataat gtctttttga taactagaaa atgtatttat tagctcattt agtgctaaat 11100
   catcattaac aacttcattg ttttcacttt ttttcttttt tatgataatg gataacaacg 11160
   atatagaagc taatattact gaaggtctaa aatcactatt tatagtttcg ataattatat 11220
   tttttacatc atttatatca taacaagatt taacgttatt gtgtaaaata aaattacata 11280
   tttcattaaa ctcagtttta taatcttttt ttattacctc acagcattcg cgtatcatta 11340
   ttaaaattgc caactcatta ttatttaatg attctattct taagtctcgc aagtacacat 11400
   tcaaaacttt ttctatgttg taattgctgg tatccatttt tacaaaaagt aacttatatt 11460
   ttcctttatt ataaaaaact ataatattcc acttgaccca aatccattat tatctctatt 11520
   tgtaccatct aaacatttaa cttctttaat ttttggatat tctacttttt caaaaataat 11580
   ttgcgccact cgatcgcctc ttttaatatt aaatgtattt ttaccattat taataaatat 11640
   aactccaatg ttccccctat aatcttcatc tataactcct cctcctatat ctatgcaata 11700
   atataacgaa agaccagacc ttggcgctat tcgtccataa catttttttg gaatagataa 11760
   actgatatcg gttttaacta aaattctttc ttttgattta acgatataat cataagcact 11820
   atataaatca tatcctgcag aaaaatctgt tgatttggta ggcagtttag caaaagcaga 11880
   caaacgtata cagttaacga cagacgaact atgatccatt ttgttatttt ttaaaattag 11940
   gatatttttt ttttgttttt ttaaaaaaca ctatctatgt ctaatgctcc tataacgttc 12000
   catccttttg ttttatcgtc aaatatttct atttcacgta tatatctttc ataatgaaaa 12060
   cctccaacga tcataataca atcatcaaaa atacatattg atgaattaaa tcttggctga 12120
   ttcagacacg actcaattct ccatttacaa gacataggat taaagctttc gaccatgtta 12180
   aaaactttta tattattgat atatgaaatt ccgccaacaa catatattaa accatgatgg 12240
   tatatagcgc atccgccata atgagaatat tttgtacact cacccaatga ccatttatta 12300
   tatttcaaat tatacacttc tactgattta tcatattcag atattcctcc aataacatag 12360
   atggtatcat tgacattaac aacacatgga ttgtatctag gatatagtaa atttggttct 12420
   tctctccaca cactttctcc aaatgaccaa ctttcaactt tattaactat acaattatta 12480
   tagattccac caataacata tattctatta ttaaaataag tggcacctgg gcattttctt 12540
   ggataaatta atgaaggtaa gttttctctt tcaaaagttt ttacattgat tgcgaataca 12600
   ctatttactg atcgtaagta tttatccaca ccccctaaaa agtaaatatt attaccaata 12660
   gcaattgaag tacaaaactt aaaattttca tctattatgt tgcttaattt acttaagtta 12720
   ctgggttgtt gaatactttc atttaatgtt ttgttttcat caatttcatt acaattgttt 12780
   tcatacaata gttcattatc ataaacactt tcatctatta tatttatatc cattatatta 12840
   tttgtaaaca gtatttttgt taagttatca ttataaacct taaacttaaa agactttgta 12900
   aatttgtcac tattaataaa ttctttattt ttatagttac atcgtggtat gtgtttatca 12960
   ctatttttgt taaaatcata aggttttttt ttaaatctgg acgaccacgc ttgtaacctt 13020
   ttttttcctt tttctgttaa aaaggtaaat cttacagcgt catttactaa cttcatcgta 13080
   ttaagcctat tagttttttt gacagttccc cacttaatta taaaattaag aacaaaatct 13140
   tcggcatcga caactaaatc actacttttt aatatgggcc ttaattcatt aactcttaat 13200
   ttagttaaat cgttgcttat atacataaac ctttttttaa tatatttatc tgcaacatcg 13260
   tacacatcgt aacaaccata catataagat cttctaagat atcaacacaa ttactatcat 13320
   caatgtaatt tatcataaaa tcagtacatg catccattat aaacttaatt tcgattacgt 13380
   tagaaattga aaaaatgtca acaacatttt tgctattcaa tgtaatataa ccagtattaa 13440
   tatactctaa cacatacgta aatggcttat atttaaaagg taacttaact tcgttactgt 13500
   attttttaat agctttttta aagtaattag atgatatctc gaaaatatct ttccttgttt 13560
   gtgatatacc tccaattgta aatattaaaa ttctatcata atcatttttt aataacatgt 13620
   actttagttc ttctacatat tctttattga tatacctttt taaagtcata ttggttattc 13680
   aagataatta acaagagttg ttatttattc aactttataa tattcaataa aaactaaaaa 13740
   tcaacatcta acgaaaaaac attatcgtct ttgttagaaa taacgcccat tttttgatat 13800
   tcactaactc ttttttcaaa gaagttagtt ttaccttcca gtgatatatt ttccataaaa 13860
   tcaaatggat ttgttgacat aaacacttta tcacaaccta gttctaatag taatctatca 13920
   gcaataaact caatatattg agacattaaa acacaattca tacctattag ttttacagga 13980
   attatattgg ttaaaaattc tttttctata gacacagcct cgtttataat aatcgtaata 14040
   atgtcattag aaggaggata ttttagatgt ttaaaaatta aacacgcaaa atcacaatga 14100
   aggccctcat ctctactaat aagttcgttt gaaaatgtta atcctggcat caatcctcgt 14160
   ttttttaacc aaaatattgc agcgaatgat ccggaaaaga atattccctc aactgcagca 14220
   aacgctatta atcgttctcc aaacgatgct tttttgtttg atatccattt tctagcccat 14280
   tcagattttt ttttaataaa atccattgtt tcaatagcat taaataaatg ttccttctct 14340
   ttagtacttg aaatgtatgt atcaataagt aagctataca tttcagagtg tatgttttcc 14400
   atagctatct gaaaaccata aaaacatcta gcttctgacc attgtacctc cgagtaaaat 14460
   ctttcggcca aattttcatt aactatacca tcactagccg caaaaaatgc caatatgttt 14520
   ttaataaagt attgctcttc attgttgagt tttttccaat cggctaaatc cttagaaaga 14580
   tctacttcat caactgtcca aaaactagcg acagcttttt tatacattac ccatatttct 14640
   ttatataata taggaaaaac tacaaatctt gaagatgttt ctttaagaat tggttccatt 14700
   ttattttata ccataaaaat tatatttcat tattttattt agtcaacatt gacgcccaca 14760
   cacttatcat cgatataata aacattttct aattcgtcaa aggatcttct tgtggttaat 14820
   ttcatcattc ttacttttat tttattaaac aaatcgaata agtaaaagca gttaaccagt 14880
   tgaaatagca aataaagact tattacgata acaattatta aaaccatcaa aattagtaca 14940
   gttcctatgc taaaggcgtt attgttaggc attgctgttg taagattcat ctgatgcatt 15000
   tgctaccact attgtaaaat ttatactttt ttttattttt tatagaaata catcatcatt 15060
   tgaaacactg tcgtcataat ttatgtaata cattacttta tgaggtgtct ttctttttgt 15120
   aaatttatac ttgtagttat aattatattt agtatcatta tcatcactac taacggtatc 15180
   actatcacta ttactattac tatcattagc accattacca tcaccatcac catctaacaa 15240
   tgtaacacgt tcaatagata ttgccgtttt catattgctt ttccttataa aactaacaca 15300
   tttaatagcc ttttctttta tacttttttt taataatctg taaccaaata ctataccaca 15360
   ataaattttt ttatccattt ttcctcactt actatcaata acgtataact tagtttattt 15420
   tttttgtttt ttaaccttaa ataatatatt aatttattta gataaaaata tttatttatt 15480
   tttttttgac gattttatac atccttcata gtataaaagt atatagttat agtagtatta 15540
   tgtacttttt gcagtgtaaa ctacttatgt gcaatcaaaa gttgaaagta aacataagag 15600
   tcacatttta tgtatcataa aacgaagtat ttgttgttat taccattgga taatcgttat 15660
   gcaggtaggt ttctttttta tcattgtgag atgatggttt tttaatattt aaacaatatc 15720
   catatttttt aatttcgtct ttagatgggg tagagttact actattgctg ttatatggct 15780
   ttccatttac aacaactttg ttaacttttt ttagtttttc gctcatgttt tatttaataa 15840
   gtatttattt atgttattag ctatgattta tgtttcattt ttaatgattt ttttaaaaaa 15900
   attatacata taaaaatgaa ccgtatttgt acttaatgtt tatttttcgt tttatagaac 15960
   ataaaccaag taaaaaaata ataaaaatta tcaatacaaa aaaaataaac caatgaacat 16020
   cgttgatgaa tggtatccta ttatcgatat aattattaat tgtatatcta ttactcattg 16080
   atttcattat tgtattcata acacaatttg cttcagaagt tccagaatta attatttgta 16140
   ataaaatgtt ttttccattt ggtgcgatac actctccaat atcgaaagtt tgaatatcaa 16200
   tcacattagt tacggcggcc gaagaagtac atttgcgtat tatttcgggt atataatttt 16260
   tatcttgatt atccaaatca atcccaactt cattcgctaa ttcatacttc tcgttttgag 16320
   atagagtatt agcaaactct ttaaatgttt ttatcaataa gtaaaaactt agttctttat 16380
   catcaataca taaatttatt atttttaatt tacacccttt aagcgttccc tttatttcac 16440
   taatgtgaat accacaagtt gtgttaactg gaacggagta taatgataaa ttttgtaaat 16500
   agcgctctat gaataaatta tacaatgtat caacttgaaa gtggttttcc atttatccac 16560
   gttgagaaaa tgtttttctt tgaaataatt ttttctaaaa aagaaatagg atgtaatatt 16620
   gatattttta acctaaattt atcacacgta tttttattac aacatataat aaattcttct 16680
   aatgtattat aaaattcttt atctgtttct atttctggat atgtttttaa aagtgtatga 16740
   atgaaaaaat gaaaatcgta gtaccaatta tgttcaactt ttatattatt ttttattttt 16800
   ttatttatta tgttcgcaac ctgtgaaaag tcaaaatcat ttaaacaagc ttttataggt 16860
   tcattaaaaa tataagtatt ttcatttaat gatattttta ttgattcgtc cgaatcaaaa 16920
   attaaaatat tatcaggttt taaatcaaca tgtaaaaagt tgttacagca tggtaattca 16980
   tatattttaa catataaaag agccatctgt aaaaaaataa attttatata ctcaactaat 17040
   gacttaaaac ctaattcaat agccattttt tcactaactt tatcagaaga gaattttgcc 17100
   aaaggaaata taataatatt tcctctatcg tataaataat tagaccgttt ttcgtgttca 17160
   aaaaaatgaa acatgtgtgt aaaatagttt attacattta cattactttg tataactata 17220
   gggtaaaaat atgataatag ttttacaaat tttacatctc cttttttctc attaaacgat 17280
   tttaaaaaat atttatgtga aaaattactt atacttaatt tttgatcatc cattgtttga 17340
   ataagtaata atatcatata tagtacccgc ttatataagt tatataaaaa tgtaagttta 17400
   aagtttacac ccattgccca cgcacacact attagttttt tttcatctcc ctttaagtta 17460
   ttaaacaaga actttggtat tgtatattct gcagttgttt caataggact atactgttta 17520
   ttaggttcat aaacaaattt tacaacatac ttatcaattt taaaaactat accatatcct 17580
   ccagttgaaa tatgataaaa gtcatcatta attggataaa atctcttatc tttttgctga 17640
   aaaaacgatg gattgatata ttcgttttca gcaattttat ttaatgtttc ttttgtaaac 17700
   tttttaaagt atcttattaa cctaacatta ggtgaccaac tttggtaaat atctaattga 17760
   gatattacat aatcaaaata aatatcatcg cctaatactg ttgtagattt tatatcttca 17820
   tcgttatccc attggcattc taatgagtta tcatctattt ccatgattac catttattgt 17880
   ataaaaagcg tttattataa tattatagtt aataataata ttttacttta tttattatat 17940
   agtcgttaat caattgataa tgttttatat tataatctaa aagtttgtct atttttaaaa 18000
   ataaatttgt gattttttcc caaagggttt catcatcgtc gatcttgtac ttttttttga 18060
   aactatacaa aaagttatta tttttattta aaatagtatc ataaataaca aagttgttag 18120
   gaatataaca attattttct ttttccatta ccttttttaa ctttataatt ttattaacta 18180
   attcgattat ttttaatctt ctaattgaaa taatatttga cgttatgttt aatgtttctt 18240
   cattattctt ttcataaaaa aatgcttttt ctgtaattga taattcttta ggagataaga 18300
   ttgtcactaa ataaattgat tcgccaccta tttgtggtaa aaaattaacc ccattaagaa 18360
   agccactcat aataaaatct ccttcttcta tatttatttt taaaaatgta gtatctggaa 18420
   taaataatcc agatattcct ttattcttaa cacaggccgt aatgacaacg ctattactaa 18480
   atgttgattc gtttaaaaaa aatgcatttc cgtgttttaa tttaaccaaa tacgatccta 18540
   taactgtaaa aaactcttca ttgagtaaaa gtttataaaa aatatttaca tttatttctt 18600
   tcaatgaata aaatcctact ttttttttac tcatatctaa gtcatttaaa aaaagattaa 18660
   aagaaaaata taactcttca cttttacttt ttaaaaaatt accaactgca acattcccac 18720
   cattaactga atataaatca tcataacatc tattatttaa aaagtacttc aaaatatatt 18780
   cttctttttt tttattactt aaacacataa tagacattat cgtatttata aatcatatat 18840
   catttcatat aatgcatttt tatctttttt aaaatcgtcc gatatatata tttctttata 18900
   attatacatt aacacatagt ctcctaaaaa atgactatca attttttgta ttaaagaatg 18960
   aatattgaat ctacagtaac taatagaaaa tgctttaaat aataataaat aattatttaa 19020
   tgtagtatta ttttcataat gctctagtaa ttcgttatta tttactatat agtctacgat 19080
   atcatcgctt aatatttctt ttatatgatc aatagttttc ataaaatcgt cgttatgaaa 19140
   tgtaatactg ttttcccatt ctaacacgcc taactccttt attttatatc cgcctatttt 19200
   tctcattaat tttatatcta ttgaggtcaa tggagtaaaa aatacagatg tttctaattc 19260
   atcatcgatt ataggaatct ttacgttatt atttttactc actttacata atgctatata 19320
   taaattgtta ttatttatat ctatacttct tttatagatg gggaaaccaa tagggtatcg 19380
   ccctaaactt attatatcta atatggattt aactttaaat ataacaaatt tttttttagg 19440
   agaataattg accgttttaa catttttaaa ccttattaac tgcataataa atcgtacagt 19500
   gtcgtgttct agtggataat acataaaatt gtttacttta gatagtgatg tttgtattat 19560
   tttagttaaa aaaatatctt ctattctatt aaaagaaaac atattaaaag gatatattaa 19620
   attatttaaa acatcatatt gtttgttgat ggaatcatat aataccaaaa ctgactctaa 19680
   tgccattttt tctaatgctt ttattttaca ttttgcttct tcttccgtga taaaatcttt 19740
   tggtataatt atcttttttt tattattcca ctgtttaaaa taatcactta tttccatttt 19800
   acacgaaaag ttagcgatat ctactaatat tagtttgaat ccgtcatctg atacaatata 19860
   atcatttcct ataaaattcc atcctatgtt ttttggccac gacgctttaa gcagtaaaaa 19920
   gtcaaaaacg ttgcttaaaa accctactaa aaatgctgtt ttaatattta ttagtattgt 19980
   ttcctttaac cattttaaaa aacataatat taactctttt gagttaaaat ttataaatga 20040
   tttaccaaaa cttgtcatca tataaattga tggacctaat ggagttatta ttgagtttat 20100
   aattataggt attattgtaa ctacttcatc atatatgtta ttttttgacc ataacctttt 20160
   cgatgcaata cttttaacat tataaggcaa tatgtctagt tccggtctat tatatttttt 20220
   tttttgttta acccatatat aatgttttcc gatgcaccct attatcattt catcaattaa 20280
   tgtataatca tcattacctt ttttactatt tttataatta acaaatctat aattagataa 20340
   tccattatcg cacatccaat aatttatctc actcaatgaa atatttttat tttcaaaaac 20400
   atgcaattta tgaacagatt tatttatttt atcgttttct aaagctcttc ttttgacaat 20460
   atctaccaaa ggagatatag atgttttagg tgaaaaaaat gtttctgtgt cgacttcgtc 20520
   aatgaataac ttgtgcatat gcatgtcaat aaaatatggt tctacatact ttgtaattcc 20580
   aactatttct aaatgagata gttttgttac agatcgcact aaatctaaat tatttaggta 20640
   tattataact ccctttccgc ttcctaatct cgctaatata atagagtgtt tatatgtttc 20700
   taatgcatta ataatattaa attcattgtt ttgtttttta aacatgtttg attacagcac 20760
   tgtatttttt ttgtctgacc aatctctttc aaatatattt gacaacatct taactaattc 20820
   attatcgttg gtattaaaac taacaaacgc atagtgtaaa taatgtgttc cgtcaaaatt 20880
   tgcaggagtt atatgtgcaa attcgttatc aataatcatc aacttggtat tattagtatc 20940
   atgaaaaatt ttaacagtta aatcattgtt actacacaaa gattgtaaac ttttaaccga 21000
   tgacataaca taaacgtcat ttttatccca agatccaacc aaaagcctaa cttttacacc 21060
   tcgattgata gttgcactga tgatagcatt gtaaatatct ggccaaaatg ttgtttttaa 21120
   atcttctctt ataacggaaa ccaacgataa caattccaaa tctattgatt tttttgctga 21180
   attaattttg ctcaacacaa catccgcatc taaagttcta gaatatccta atatatgatc 21240
   aggagaatct gacaaaaaca caccaccaat tggattatta atatggtatt ttgtactcac 21300
   tggcaaacaa catgttatat atattcctct aaaaatactg ctgttgttat ttaaagcttt 21360
   aaatgtttca aaccttcttt gtaaatctaa agctaatggc gcgtaagtag aatatatacc 21420
   taaagtcttt atattggaaa tggatccacc agttaaagag gtgttaccta tatagcattt 21480
   attataatca gaaacccaaa aacttcccaa taatactccc ggatcgttat ctcctcctat 21540
   ttttactttg acatactcaa ttttatttga tattaattcc tcttcatcct tgttaccgct 21600
   ttgatgatct actaaaattg taactttaat tccagatgct gcggcttctt ttaatttatt 21660
   taaaatattt tttccatgtt cgctagtgcg aagattacaa caaaatgatg ctatatttat 21720
   atttttttta gcttgtgaaa taatttcatc aaagcactca tgtgtgagca tatgttgtgt 21780
   tgttatccct gaggttttag gcaatgtttc aacgagtcta catcctgctc cagatggagg 21840
   ttttgaaaaa aataaggacc acatttaatg ctaaaaaaaa aatagttatt ttataacaat 21900
   gtttgttttt cttcaatatg tttgtagtta ttatctttat ttatttcttt aaacataata 21960
   actaaatcta aaattattga cactataccc actaatgcaa aaatagcacc aataaagacc 22020
   aaaaaaaatt caaacatcat ttaatagaca tgttatttat ttaaattatt ctatttgaaa 22080
   ctttgaagac caaaaatgtt ttattcttaa cccaattatt aaatatttta agtcaaaagt 22140
   tggtacaaat atatttatat ttaaactatt atcatcttta ttaatgatac aatatttaca 22200
   tattgttttg ttttttgttg cctcgtcaat acattttaaa acaaaacttg gtaataagta 22260
   ttgacattga ggttgttgaa aatgaaatgg tgaatatagt aactcagtta atgtaaacct 22320
   ataacctttt ataataacag aatctgatgt agctacggaa tttgtaggaa agtgttttac 22380
   tgtatccatc ttgacaaagc atctatttcc aagtaagcaa ttatcatctt tattgatttt 22440
   tattctgtcc atatttctaa atggacttaa tttaattatt tcatctaact tgttaacatt 22500
   catggttgaa aatgttaatg atgttagatt tatcttaaaa aaaacgaatg ctttttcatt 22560
   tttgatcgca tacacttcta cactgttata tttttaaagg atgtacatat atcgtttaca 22620
   taatggatgt tatctaaatc ttttatttta tttacaaatt tattataata ataatttttt 22680
   tcctttatat tttttttact ttttcttgga tgcaatgctt tccttaacca taacgaatca 22740
   caattaattg catccattaa tattttaaaa gtttcaaagt tatcgtaaat aaatttcttt 22800
   atgatatcat cactacatgt attcatatta attaaaacaa acaaatcttt gagctttttt 22860
   cttgtattaa ttttgctatc aagattaata tgtctaaatg atggggttat cccatcaaac 22920
   aataaattat ctttaacata atatatcgtt aaggatttat ttctttttct cattaaaaag 22980
   ttatataaat gatcatttga aacagatagt tgttttttct ggtaaaatac caatatatca 23040
   attttattat catgagatat acactcccat ctttttgaag atataacttt ttcatctata 23100
   taccctacct cttttagctc aatttttata gatgaattta ttaaattgca atagtttcta 23160
   catattttta tttgataatc tatagatgta tcaaataacg atattaaact tgttactata 23220
   gctgcatttt ttaacatttt ttaaaaaaca aaggtgattt tttatacttt tttataatag 23280
   tttttcattt atgttatagt aatataaatg gcagcatata attcacaata ttatgcaaac 23340
   acaccatttt atataacgac aaaagaagga aaatatcttg tattaaaagc aattaaagtt 23400
   tgcgatataa gaacagttga atgtgatggc gataaagcat catgtatttt aaaagtggaa 23460
   aaatcaaatc aaacttgtga caggccatct tccccatgtg aacgagctgc aagagcttca 23520
   tccccaaata gagggaataa ctctgtacca tttatgcgca caaatatgtt agaggacctt 23580
   caagcgaaaa atcgtagcgt tatgtctaga atacttggtt aaattttaat gcgttataat 23640
   atctttttct atatcaatta taccatgttt accacttttc ttatctctag gtataatatc 23700
   ataaattggg tttttttcta ccccaattaa tgagtttacg atatctgtaa gtggttttat 23760
   tatatctcct ttagttaaaa tttgataaat taatatatgt gcgcttaatc ctctggaact 23820
   tatctcatga atatcggttt ttgatttcat tagtattgta tttgaaaaat aagtatataa 23880
   aatattatta tttatcaaga atgcagaatt tgaaacattt tcaaaatcaa tagcatcgtc 23940
   agcatttaaa tctaatatat cactagataa tgaactttca tctaaataaa ctaaacattt 24000
   tttgaatggg aaatcgtaat tgctagtatc gaccgtgatg atacgtttat caatgtctat 24060
   aactgaatac ataggcatgt tatttttttt tgtttggtta ctgataaagt ttataccata 24120
   gttaactcta acatactcta ataatgtagc taatctaact gttagttttt caggattttt 24180
   agctaaatct tctaatctgt ctatttgtga aaacattttt aacattgaca taagttgcaa 24240
   tgttggatct actatatata tattatcaat taaaactttt ggtattgatt gcatggtatc 24300
   ttgtcgtata ttaaaactat ctattatatg gctatcgttt tgatccttta acaccatata 24360
   gttttttaag tatggtactt ttaataaaac aacatttaag ccagtaataa acttgattaa 24420
   aaacgcaaga tctataagaa aagttctaga gtttgtttgt aatatatcta tatctccata 24480
   tttaacatcc ggatttaata aatgtagaga atatgatcca taacagatac aatttttatt 24540
   atgtcgtcgc aaatactctt ccattaaaga gtttacatta tcaactaacg atgaaacatt 24600
   atgtctaccc atggttttat ccgtcatctg tgcaacgttc ataaaattta aagcatccct 24660
   agctaaagat tgcatcgatg atgcattata gtaaatatta gaagtaaata aatcaggccc 24720
   tttagttgtc aatatcccta atacatcagt atatgttgta actaatatac tttgtaattc 24780
   tattatggta tataacttac caattgagta tgtgttctct tgtttagaaa agtaagtaag 24840
   aattgatctt tttatatcag atggagaaga cttgatatca gaaaaaaatt tctttttatt 24900
   cttttttatc aaagatataa atatttcttt attaaaaatg tttactcttt gtatgttttt 24960
   atattgtgac ttcaacatgt gaaactcggt taaagatgga tctcttttta aatacaattt 25020
   taaaacatct acaacaattt tatcattcat ttttgtttat tcaatatgat gaagatacaa 25080
   actttttcac aaaaaagtag tgtatttttt aaataatttt tttttattat tacaaatcca 25140
   ctatcgcata tatatttttt aaaaagattt aattctttac aaacgctaga tatgtcttca 25200
   gtaagtgttt catttatttt cattcctgta aagaaattac ttataagtaa agatataaaa 25260
   acattagttc ctctactaga atacgttgac cctactacta agtataaaaa aagaatttga 25320
   ataaatgaat ttattataga gtggaaattg gatatgggtt catttagtga ataaatatta 25380
   gttatataat tgcctaaaaa tttatattcg ataaaatcta caggcgatat cttaaaaata 25440
   acgccgtgct ctatctttga agggtgtgaa tatttagatc cttttattat atcaattata 25500
   ggaacccagg aaggaaaata tctatatggt aaatatacca aaccatatgt cgttaactta 25560
   gcaattgtaa atacttgatt taaaatatct tcttctgttt ttaaaataat atcattttct 25620
   ttatttattt gtatttttat tccagtgcta tcttcaatat aagacatatt tttaaaaacg 25680
   gtcatatcga tttctggtat attactatat aatattaatg attttatttt attttgatca 25740
   tatataaaca tactattttt atttggcaat tttttaaatg gtattaagtt ttttatatca 25800
   ctaacctttt taaggataaa aataacatta ttttcgctgt gtgtgcagga tagataaaat 25860
   aacgacggtt gaattttata gctaaaatat ttatttatat tattagatga aaatatattt 25920
   agtaaataca tcgtagatgt tttatttaat ggataatcaa acattaactt gttcatttta 25980
   tgagagcaaa atccatattt ttggacaaaa atatctatta gcttattatt tttattatac 26040
   caatcagtac taaatgtgta atccgaaacc aagtttgcgt ttttttctaa taatacattt 26100
   atatcaattg tatctagtag ctcaactaaa tcttcgtatc ccatttcaat acacactatc 26160
   ataactaacg aactatgtat tggaatatct ttgattttaa ctttaatatt tgattcacaa 26220
   actattttta tacataattc ttctgttaat atatttaatg gtattttatc tataatagta 26280
   gaaagcaaat tgatataatt tttttctatt aagtaattga taatatacat tttattgtta 26340
   taatcataat tatcaatata tttacagcat atttttaaaa aatcttttgt caattcatat 26400
   tttgtaaaat ctattaatgg atctttaaaa aaaaaactat aatataatcc taattcatct 26460
   tttttatcgt ataaaaattc tatagcatag gttttatatt gatcaacttc tccatttttt 26520
   attttagtta tgataaattt ttttattctg tcatctttta attgttcaga cgttaacata 26580
   tctaaaaatg ttaaaatatc tttatgaaat tttatagatt gtaaaacatc caagataagt 26640
   tcaatagaaa ccacgcttaa aattccatca ttgtttggta ttattccata tttatataac 26700
   atatccatta aaaatttact ttcaaaggat tgatgttgat ataaactatc acataaacta 26760
   aagtcattaa taaaaatttt ttctgccaat gactcactaa acaaatagtt tatattttta 26820
   atactttccg aatttatttt agttagtata ttttctactt cgatgttaga aaatctgtgt 26880
   attagacaaa aaatatcatc atctgtaata accatatact gaatacattt cagtattatt 26940
   cttttactac attttttaat aatatctttt ttatgaaaat ttatatgttc tttgtataac 27000
   tcaatatttt tttgattata aactaaagct atcaaatcaa caaatggtac agtttttggt 27060
   ttgaataaat atattttact aggaaattct tttcttatcc taaaatacca tttttgtggc 27120
   aaattattgg ggttaattcc attactgagc attttcatag catcatctat atttactttt 27180
   ttatatgatt ctgttaaatt atcacaatta tataaaataa atgcttcctt taacggaatt 27240
   aaaaaattaa acatattatt gtaaatttaa aatttgataa tagatgtatt gattagaaaa 27300
   tcaatagttc gtttcgtcga tttttgttta gcttcttttt ttgtattacc aatttctgga 27360
   aaaaatttta taccgcttat aataacagag gccaaaaaca ttggttttct accatttcca 27420
   caactagata tatttatgta ccaatctcta gatgtaaact gacaatattc gtttatcgca 27480
   gaacaaggat ttttttcttt ccataaaaca attttatagt acggaattgt atcagaaaat 27540
   gatttattta gttttttcgt atcgttattc tcattgtcat cgttatcgat gccatttttt 27600
   ttaaaccatt taggtggatt cgatcttatc ataaaaataa aaccgtcatt atgtagttta 27660
   tacagctgtt tgtttacatt tgatttttca atatttagtt ttctagatat ttctattgct 27720
   gttataaact cactttctga tagattattt accatttttt tcacaagttc atacgaatct 27780
   acttcatcac aagaatacat ttttattttt gttgtattta aaactaaaaa attttttttt 27840
   tttcattttt atagtttatg ttatttttct acagctctaa actttggagg tttaaaattt 27900
   ttcttgcagt ctctacaaga atgcataact aatggtggtt catctgctgc tcttgtttgt 27960
   atcatgacgg gaatagtgtt tttacttttg cactcagggc atgggaggtt atacttttta 28020
   tctaaaacat taaaatattc ttcgtggttt atatctctaa tattgtctat attgaattct 28080
   acattttttt gaacacattt tattccaaaa agtatatatc ttaaaatttc tttctctgtt 28140
   ccatttgtcg tacgtataag atcacatatg tcagaataat ctttatttgt ctctattaaa 28200
   ggcttgtttc tatacgataa tttatttttt gaatcttttg agtattcaat acctatattg 28260
   tttcttggtt caaattttgt ttcttctata tttgactttg tattaataat atttcttata 28320
   taaaacttgg aagctttgtc tgttgcccat tttaatattt cttctacctt tgatttttct 28380
   tttacatatt tataaattat atcttctatg tcttgatatg tgggggtagt attatcacta 28440
   tatgaattag tattatcatc atccatattt gtactgaagt tgtttaattt caatatttac 28500
   aaaaatgaat ttttagttct ttatttagat ttttccatta atggcaaaaa tggaaaatat 28560
   tacaccacct aatcactttg aaaaagcttc tgtttatgca tgtaggttat ttaaacagtt 28620
   ttataacgaa aaaaaatatg atttagatga atgtttttta aaagttagaa atagcatgtc 28680
   tcgatactat tctatggttg gtgatgattt agtttggaac agagaaatat taagttactt 28740
   atatgaatac atagcatata aaaataatgc aaataatcca aattttgttc tatcttcaaa 28800
   aattgttaat ttaactaaga aaaatacaaa aaagataaaa aatgaaataa attctgttgt 28860
   tcaaacttac aaattggata aacatataaa aaaagtaaat ataacacact atgattatat 28920
   aaaaataaaa aaaacgtatg atacattcaa aaaaaatgtt gatattaaat caaatgatat 28980
   atctgtaaca ctattaaaaa tttataaaca actaagctta attagtgatg tttcgataaa 29040
   tacacgttct atgacaaaat ctttaatgta ttacatgttt cctgatctct ttaaaaagga 29100
   taaacgacat gtattttaca gagtagacgc taaaaaagga tattgtgtat tatcatctaa 29160
   aaagataaac ttaattaggg ttttattaga atataaattt gatataaaat acaaagagtg 29220
   gggattaata aaaagatata ttaatactat ttgtaataaa ggtagtaaaa gtagcaaaat 29280
   ctgtattgga aaatatccat atttaaagtt aaaacctgtt tctaaaatta aatttaatta 29340
   tagatctaaa tacgtaagtg attttaagag aatagtgagt tctagttttt cattggttga 29400
   tttgacagaa aaaataacga aaaaaacttt tagtaatatt tttagaaatg aaattagtca 29460
   tctgtataag tttaatgcag aaaaaaacca tctcgcgcta gatgaataca aaaaaatgag 29520
   aatgtgtaat aaaataatat ctgctataga ttatccaaat aaagatcaca tttatagtgc 29580
   gataatagaa actgttaaca attacttaga taatcctccg aaaaagctag gatacaatat 29640
   tgatacttgt ttgactatag caaaaaacaa ttaacaacta atcatggatt ttatacgaag 29700
   aaagtattta atatatacaa tagaaaacaa agttgatttt ttaaaagatg aagtactaac 29760
   aaagatatca aactttacat taaatcatat attagccatt aaatatttaa ttcttaactt 29820
   tcctagagat gttatatcaa aagatgtatt atcgaaccca aatttttatg tttttttaca 29880
   tatggtaaag tgtgatgaca tttatgagac tgttttaaaa cattcgttcg atattcctac 29940
   gttatatata aaagcactta taaaaaatta tccgttattt aataacacaa tagaaagata 30000
   taaaacttta gttagtgaac tttttttaga cgataaattt atcgaaatag taaaatattc 30060
   gtctgaattt gataatatca tagctgttaa ttacgatctt ttactaaatc ctttattcca 30120
   taataatgaa ccgattaaaa atatggaaat tatttatagt aaacttttta aaaaatctaa 30180
   ttttaggaag gtaaaaaaaa tggaggttat cagattaatg atatgggcgt acctaagtaa 30240
   acaagatact ggattagatt ttactgattt tgactctcaa gatatataca cactgtttca 30300
   aaaaagtgat aaagttatta ttcatagtga tatgacagaa aagtttaaag agtatatttt 30360
   cgggtcggaa aaaactagtt attggttatg gttaaatgaa tcaatattta atgataatgt 30420
   ctacaatgaa gggtttgcag aattaatgta cgataaaatt cttagtttta tctattcaga 30480
   gattaaacaa ggaagagtaa ataagaatat gctgaaatta gtctatatat ttgaaaatga 30540
   tgaatatata cagtctattt tattacaaat aatttacggg atacctggtg atatcttatc 30600
   aattatagat gtaaaggatg ataactggaa aaaatatttt ataggttttt ataaagaaaa 30660
   ttttatcgat ggaaaaactt ttataagtgc aaaaacattc aactatgatt tatttaaagt 30720
   agtggctaaa ataaatcctg agtactttga cccagacaaa ataatatcca tatttgatca 30780
   taaacctgaa aaagttaaat attttgatac aatagatata aataatacat ttatatcaaa 30840
   tataatttat gaaacgaatg aattaaatct ttcaacgatt gaagaattac aatcatgcca 30900
   aatatataat gaagaaacag aatatttcat taaagaatat aatacttatc tttatttaaa 30960
   agagaaggat ccatatattt tgtataatgg catgttaaca aatatatcaa aagttccatc 31020
   aaataaaaag ttttcattgt ttagtaaaaa catattaaaa tattatatag atggaaaatt 31080
   ggcaaatata ggtcttgtgt taccaattta taaaggtgat atattggtaa aaatattatc 31140
   acatttgaaa tgtgtagagg acgttactgt tttcattaaa ttttcagtat gtaaaaactc 31200
   tagcatacta ccatcaataa taagaacaat tttagcaaac tttaatattt caataataat 31260
   attatttcaa aagtttttaa gggaaaactt attttacgtg gaatcttttt tagaaaaaac 31320
   aaatcattta actaataacg ataaaaagta tatactagaa ataataactc gtggaagatc 31380
   ttaatttaaa atgatgggag acatttattc agagtcacaa aaaagaaacg acgatcaaaa 31440
   aacatacttt acaagaaatt taagtccttt aatgaaaaat acatatttat accataatta 31500
   ttcatatgga tggataccgg aaacagctat ttggagtagt agatttgcaa atttagacgt 31560
   aacagagttt tacccaatta caataaattt aataaaaaaa tttgaattta tgctttcatt 31620
   atacgatggc cccatattac catatgaaga aaaaattaat acagaattta tttcaagagg 31680
   ttctttttct ggaagatata taaatttttt aagaaatttt tcattactac caactagtga 31740
   attcatttca tttttattat taacatctat accaatatat aatattcttt tttggtttaa 31800
   aaatactcaa tttgatatta aaaaacatac actttttagc gaagtttata ccaataacga 31860
   taaacatata gaactaggaa aatattttag acaaaccggt gattataagc ctttatttag 31920
   caggctaaag gataataata tatatacttt gccgtttcca atcaaccaaa gtaacattaa 31980
   caacccaaca ttacctattg gcttttccaa atcagattat gaaacattat ctaatttaag 32040
   cgcaatactt tatttaacta attatgatcc tgttttgatg ttcttagtgt tttatgtgcc 32100
   taaaatatct ataacgacaa aaataacacc agctgttaaa tatttaatgg ataaactaaa 32160
   tatgaatgaa acggatataa cattagttta attataaaaa ataggtctag ttccgaacat 32220
   tttttcaaaa aatgatatgg ataatacttt gttatctaac aaattcacta tttcagtggc 32280
   aagcctttta aagtatactt cgtaaaatat tcgttcagta gattttaatt taaaactcct 32340
   atctataatt ctttcatacg tttttatatt tactaatttt ttttgccatg gttctttttt 32400
   attgcatatg tatgcaaagt aatatcgttc acctatttca ataatttcat cattattttt 32460
   attatattca tttactaagt acatatttgg attatcactt gatttataat tacaatgatg 32520
   tgttctactc aatagaaaca tatccaatgg agctgttttt gtttcaaatt ctatttgtaa 32580
   atcactttct aacgatgata aaatttcaac acatacttgt acagatgaca tatttccacc 32640
   tgataacatt tgcaaaagcc ttgttttgta tattcttatc atatatttgt gaaacttaga 32700
   tacgtctctc ctcgtctcac tagttccctt attgatacgt tcgggaattg attcattggt 32760
   gaaagat                                                                32767

<210>  6
<211>  34
<212>  DNA
<213>  Artificial sequence

<220>
<223>  LSDV 130 forward primer sequence

<400>  6
ggtaccggag acaacattga acaaaccgat aatg                                  34

<210>  7
<211>  59
<212>  DNA
<213>  Artificial sequence

<220>
<223>  LSDV 130 reverse primer sequence

<400>  7
ggaattagtg atcactcgag gcatgcatcg atctatagtt tgttattatc agttgcatg       59

<210>  8
<211>  59
<212>  DNA
<213>  Artificial sequence

<220>
<223>  pRO-2-GPT forward primer sequence

<400>  8
catgcaactg ataataacaa actatagatc gatgcatgcc tcgagtgatc actaattcc       59

<210>  9
<211>  59
<212>  DNA
<213>  Artificial sequence

<220>
<223>  pRO-2-eGFP reverse primer sequence

<400>  9
gcaattccga taattccata tgcaacgaat tccatccgat ccaatgtcga cgtatacag       59

<210>  10
<211>  57
<212>  DNA
<213>  Artificial sequence

<220>
<223>  LSDV 132 forward primer sequence

<400>  10
gtatacgtcg acattggatc ggatggaatt cgttgcatat ggaattatcg gaattgc         57

<210> 11
<211> 34
<212> DNA
<213> Artificial sequence

<220>
<223> LSDV 132 reverse primer sequence

<400> 11
actagtgata catcacacaa tgtaacaagt tcag 34


<210> 12
<211> 2251
<212> DNA
<213> Artificial sequence

<220>
<223> Sequence of pHM2v3 between the sites of insertion (PvuI and NotI)

<400> 12
cgatcgtaag ccgtggcggt ctggttccgg gtgcgttact ggcgcgtgaa ctgggtattc 60

gtcatgtcga taccgtttgt atttccagct acgatcacga caaccagcgc gagcttaaag 120

tgctgaaacg cgcagaaggc gatggcgaag gcttcatcgt tattgatgac ctggtggata 180

ccggtggtac tgcggttgcg attcgtgaaa tgtatccaaa agcgcacttt gtcaccatct 240

tcgcaaaacc ggctggtcgt ccgctggttg atgactatgt tgttgatatc ccgcaagata 300

cctggattga acagccgtgg gatatgggcg tcgtattcgt cccgccaatc tccggtcgct 360

aatttttcta tcgatggatc ctagttagtt tggtttttaa ataacaaata ttgacaatca 420

ctgttttaat catattaaat gattaggaaa tattatgaat ttaataaaac agtttcaaaa 480

gcggtctgtg ttttgaaagg atataaactt catggtgtta ttaattttga ccagttacaa 540

aatggaatag taattatatc aggcgttgta cttggattac cggaaggtaa tcatggatta 600

catgtacacg agttcggtga tgaaactaat gggtttttga gtatgggaaa tcattataat 660

cctgaaaata aaaaacatgg tagcccttta ataatgagag gcatattgga gatctgggaa 720

atatttattc aaacaaatat ggaatatctt atctacatac tcgatggtaa aatctctctt 780

gttggagatt attctattat aggaagatcg ttagttatta gtgagaaaaa tgatgattta 840

ggcaaaggat ataactttaa aagctttata gatggaaact ctggaaacgg tgtcgcgtat 900

ggaattatcg gaattgcata atctattttt ttttaaatat ataaattata aaaatgacaa 960

acacttccct ccttttaagc tactttttct ggatccggcg cgcctctaga aaaaattgaa 1020

aataaataca aaggttcttg agggttgtgt taaattgaaa gcgagaaata atcataaata 1080

gtcgacatgg tgagcaaggg cgaggaggat aacatggcca tcatcaagga gttcatgcgc 1140

ttcaaggtgc acatggaggg ctccgtgaac ggccacgagt tcgagatcga gggcgagggc 1200

gagggccgcc cctacgaggg cacccagacc gccaagctga aggtgaccaa gggtggcccc 1260

ctgcccttcg cctgggacat cctgtcccct cagttcatgt acggctccaa ggcctacgtg 1320

aagcaccccg ccgacatccc cgactacttg aagctgtcct tccccgaggg cttcaagtgg 1380

gagcgcgtga tgaacttcga ggacggcggc gtggtgaccg tgacccagga ctcctccctg 1440

caggacggcg agttcatcta caaggtgaag ctgcgcggca ccaacttccc ctccgacggc 1500

cccgtaatgc agaagaagac catgggctgg gaggcctcct ccgagcggat gtaccccgag 1560

gacggcgccc tgaagggcga gatcaagcag aggctgaagc tgaaggacgg cggccactac 1620

gacgctgagg tcaagaccac ctacaaggcc aagaagcccg tgcagctgcc cggcgcctac 1680

aacgtcaaca tcaagttgga catcacctcc cacaacgagg actacaccat cgtggaacag 1740

tacgaacgcg ccgagggccg ccactccacc ggcggcatgg acgagctgta caagtagttt 1800

ttctccgcgg cccggggtat actgatcaat tggatcggat ggaattcgtt gcatatggaa 1860

ttatcggaat tgcataatta ttttcttttt aaaaatatat aaattataaa aatgacaaat 1920

acttccctcc ttttaagcta ctttttctta tttttgtacg gaaacgtgtt tgtcaaatct 1980

gactataact atttaatgta taagataaaa tttttgtacg gaaacgtgtt tgtcaaatct 2040

gactataact atttaatgta taagataaat gtttttaaaa acaatgaaag tactatcaag 2100

tgctatcata ataatgatat tattttttta tcagatgatt gcgtaagctt taactctaca 2160

tataatacaa ctgttttaaa caatgatgat gttaaaactg aacttgttac attgtgtgat 2220

gtatcactag tggtaccaac gttgcggccg c 2251

<210> 13
<211> 486
<212> DNA
<213> Lumpy skin disease virus

<400> 13
atgattagga aatattatga atttaataaa actgtttcaa aagcggtctg tgttttgaaa 60

ggatataaac ttcatggtgt tattaatttt gatcagttac aaaatggaat agtaattata 120

tcaggcgttg tgctcggatt accggaaggt aatcatggat tacatgtaca cgagttcggt 180

gatgaaacta atgggttttt gagtatgggg aatcattata atcctgaaaa taaaaaacat 240

ggtagccctt ttaataatga gaggcatatt ggagatctgg gaaatattta ttcaaacaaa 300

tatggaatat cttatatata tatattagat ggtaaaatct ctcttgttgg agattattct 360

attataggaa gatcgttagt tattagtgag aaaaatgatg atttaggcaa aggatataac 420

tttaaaagtt tcatagatgg aaactctgga aacggtgttg catatggaat tatcggaatt 480

gcataa 486

<210> 14
<211> 485
<212> DNA

<213>  Lumpy skin disease virus

<400>  14
atgattagga aatattatga atttaataaa acagtttcaa aagcggtctg tgttttgaaa      60

ggatataaac ttcatggtgt tattaatttt gaccagttac aaaatggaat agtaattata     120

tcaggcgttg tacttggatt accggaaggt aatcatggat tacatgtaca cgagttcggt     180

gatgaaacta atgggttttt gagtatggga aatcattata atcctgaaaa taaaaaacat     240

ggtagccctt taataatgag aggcatattg gagatctggg aaatatttat tcaaacaaat     300

atggaatatc ttatatatat atattagatg gtaaaatctc tcttgttgga gattattcta     360

ttataggaag atcgttagtt attagtgaga aaaatgatga tttaggcaaa ggatataact     420

ttaaaagctt tatagatgga aactctggaa acggtgtcgc gtatggaatt atcggaattg     480

cataa                                                                 485


<210>  15
<211>  327
<212>  DNA
<213>  Lumpy skin disease virus

<400>  15
atgattagga aatattatga atttaataaa acagtttcaa aagcggtctg tgttttgaaa      60

ggatataaac ttcatggtgt tattaatttt gaccagttac aaaatggaat agtaattata     120

tcaggcgttg tacttggatt accggaaggt aatcatggat tacatgtaca cgagttcggt     180

gatgaaacta atgggttttt gagtatggga aatcattata atcctgaaaa taaaaaacat     240

ggtagccctt taataatgag aggcatattg gagatctggg aaatatttat tcaaacaaat     300

atggaatatc ttatatatat atattag                                         327


<210>  16
<211>  483
<212>  DNA
<213>  Lumpy skin disease virus

<400>  16
atgattagga aatattatga atttaataaa acagtttcaa aagcggtctg tgttttgaaa      60

ggatataaac ttcatggtgt tattaatttt gaccagttac aaaatggaat agtaattata     120

tcaggcgttg tacttggatt accggaaggt aatcatggat tacatgtaca cgagttcggt     180

gatgaaacta atgggttttt gagtatggga aatcattata atcctgaaaa taaaaaacat     240

ggtagccctt taataatgag aggcatattg gagatctggg aaatatttat tcaaacaaat     300

atggaatatc ttatatatat attagatggt aaaatctctc ttgttggaga ttattctatt     360

ataggaagat cgttagttat tagtgagaaa aatgatgatt taggcaaagg atataacttt     420

aaaagcttta tagatggaaa ctctggaaac ggtgtcgcgt atggaattat cggaattgca     480

taa                                                                    483

<210>  17
<211>  161
<212>  PRT
<213>  Lumpy skin disease virus

<400>  17

Met Ile Arg Lys Tyr Tyr Glu Phe Asn Lys Thr Val Ser Lys Ala Val
1               5                   10                  15

Cys Val Leu Lys Gly Tyr Lys Leu His Gly Val Ile Asn Phe Asp Gln
            20                  25                  30

Leu Gln Asn Gly Ile Val Ile Ile Ser Gly Val Val Leu Gly Leu Pro
        35                  40                  45

Glu Gly Asn His Gly Leu His Val His Glu Phe Gly Asp Glu Thr Asn
    50                  55                  60

Gly Phe Leu Ser Met Gly Asn His Tyr Asn Pro Glu Asn Lys Lys His
65                  70                  75                  80

Gly Ser Pro Phe Asn Asn Glu Arg His Ile Gly Asp Leu Gly Asn Ile
                85                  90                  95

Tyr Ser Asn Lys Tyr Gly Ile Ser Tyr Ile Tyr Ile Leu Asp Gly Lys
            100                 105                 110

Ile Ser Leu Val Gly Asp Tyr Ser Ile Ile Gly Arg Ser Leu Val Ile
        115                 120                 125

Ser Glu Lys Asn Asp Asp Leu Gly Lys Gly Tyr Asn Phe Lys Ser Phe
    130                 135                 140

Ile Asp Gly Asn Ser Gly Asn Gly Val Ala Tyr Gly Ile Ile Gly Ile
145                 150                 155                 160

Ala

<210>  18
<211>  108
<212>  PRT
<213>  Lumpy skin disease virus

<400>  18

Met Ile Arg Lys Tyr Tyr Glu Phe Asn Lys Thr Val Ser Lys Ala Val
1               5                   10                  15

Cys Val Leu Lys Gly Tyr Lys Leu His Gly Val Ile Asn Phe Asp Gln
            20                  25                  30

Leu Gln Asn Gly Ile Val Ile Ile Ser Gly Val Val Leu Gly Leu Pro
        35                  40                  45

Glu Gly Asn His Gly Leu His Val His Glu Phe Gly Asp Glu Thr Asn
    50                  55                  60

Gly Phe Leu Ser Met Gly Asn His Tyr Asn Pro Glu Asn Lys Lys His
65                  70                  75                  80

Gly Ser Pro Leu Ile Met Arg Gly Ile Leu Glu Ile Trp Glu Ile Phe
                85                  90                  95

Ile Gln Thr Asn Met Glu Tyr Leu Ile Tyr Ile Tyr
            100                 105

<210>  19
<211>  160
<212>  PRT
<213>  Lumpy skin disease virus

<400>  19

Met Ile Arg Lys Tyr Tyr Glu Phe Asn Lys Thr Val Ser Lys Ala Val
1               5                   10                  15

Cys Val Leu Lys Gly Tyr Lys Leu His Gly Val Ile Asn Phe Asp Gln
            20                  25                  30

Leu Gln Asn Gly Ile Val Ile Ile Ser Gly Val Val Leu Gly Leu Pro
        35                  40                  45

Glu Gly Asn His Gly Leu His Val His Glu Phe Gly Asp Glu Thr Asn
    50                  55                  60

Gly Phe Leu Ser Met Gly Asn His Tyr Asn Pro Glu Asn Lys Lys His
65                  70                  75                  80

Gly Ser Pro Leu Ile Met Arg Gly Ile Leu Glu Ile Trp Glu Ile Phe
                85                  90                  95

Ile Gln Thr Asn Met Glu Tyr Leu Ile Tyr Ile Leu Asp Gly Lys Ile
            100                 105                 110

Ser Leu Val Gly Asp Tyr Ser Ile Ile Gly Arg Ser Leu Val Ile Ser
        115                 120                 125

Glu Lys Asn Asp Asp Leu Gly Lys Gly Tyr Asn Phe Lys Ser Phe Ile
    130                 135                 140

Asp Gly Asn Ser Gly Asn Gly Val Ala Tyr Gly Ile Ile Gly Ile Ala
145                 150                 155                 160

<210>  20
<211>  2186
<212>  DNA
<213>  Artificial sequence

<220>
<223>  pHM1 insertion cassette used to make nLSDVdSOD-M

<400>  20
   ggagacaaca ttgaacaaac cgataatgaa aaacaactat taagtgattt tagtgatatt      60
   ctaaacaatg atgaaaaaaa tcacgataaa gataaaaaaa aggttgtagc gtaaattaag     120
   caaaatattg tatactttat gtatacgttt taatattgaa ttaatatata gagtttataa     180
   ttatggaaga acttgaatgt atgtcgataa aaataacttc cgaaaatggg agcaccattt     240
   ccatatactg tgaatccaaa actaaaatat cattagatat aacaacaaaa aaaaataaca     300
   taaaacctat tacaacaaaa aaatctatta aaaaaataga aaaaaatgat gatgatgatt     360
   tattcacaga ccaagaaaat gcaatggaaa tagatgggga gcatgcaact gataataaca     420
   aactatagat cgatgcatgc ctcgagtgat cactaattcc aaacccaccc gctttttata     480
   gtaagttttt cacccataaa taataaatac aataattaat ttctcgtaaa agtagaaaat     540
   atattctaat ttattgcacg gtaaggaagt agaatcataa agaacagtga cggatccatg     600
   agcgaaaaat acatcgtcac ctgggacatg ttgcagatcc atgcacgtaa actcgcaagc     660
   cgactgatgc cttctgaaca atggaaaggc attattgccg taagccgtgg cggtctggtt     720
   ccgggtgcgt tactggcgcg tgaactgggt attcgtcatg tcgataccgt ttgtatttcc     780
   agctacgatc acgacaacca gcgcgagctt aaagtgctga aacgcgcaga aggcgatggc     840
   gaaggcttca tcgttattga tgacctggtg gataccggtg gtactgcggt tgcgattcgt     900
   gaaatgtatc caaaagcgca ctttgtcacc atcttcgcaa aaccggctgg tcgtccgctg     960
   gttgatgact atgttgttga tatcccgcaa gatacctgga ttgaacagcc gtgggatatg    1020
   ggcgtcgtat tcgtcccgcc aatctccggt cgctaatttt tctaagatct aaaattgaaa    1080
   ttttattttt tttttttgga atataaataa tggtgagcaa gggcgaggag ctgttcaccg    1140
   gggtggtgcc catcctggtc gagctggacg gcgacgtaaa cggccacaag ttcagcgtgt    1200
   ccggcgaggg cgagggcgat gccacctacg gcaagctgac cctgaagttc atctgcacca    1260
   ccggcaagct gcccgtgccc tggcccaccc tcgtgaccac cctgacctac ggcgtgcagt    1320
   gcttcagccg ctaccccgac cacatgaagc agcacgactt cttcaagtcc gccatgcccg 1380
   aaggctacgt ccaggagcgc accatcttct tcaaggacga cggcaactac aagacccgcg 1440
   ccgaggtgaa gttcgagggc gacaccctgg tgaaccgcat cgagctgaag ggcatcgact 1500
   tcaaggagga cggcaacatc ctggggcaca agctggagta caactacaac agccacaacg 1560
   tctatatcat ggccgacaag cagaagaacg gcatcaaggt gaacttcaag atccgccaca 1620
   acatcgagga cggcagcgtg cagctcgccg accactacca gcagaacacc cccatcggcg 1680
   acggccccgt gctgctgccc gacaaccact acctgagcac ccagtccgcc ctgagcaaag 1740
   accccaacga gaagcgcgat cacatggtcc tgctggagtt cgtgaccgcc gccgggatca 1800
   ctctcggcat ggacgagctg tacaagtaat ttttctgtat acgtcgacat tggatcggat 1860
   ggaattcgtt gcatatggaa ttatcggaat tgcataatct attttttttt aaatatataa 1920
   attataaaaa tgacaaacac ttccctcctt ttaagctact ttttcttatt tttgtacgga 1980
   aacttgtttg tcaaatctga ctataactat ttaatgtata agataaatgt ttttaaaaac 2040
   aatgaaagta ctatcaagtg ttatcataat aatgatatta tttttttatc agatgattgt 2100
   gtaagcttta actctacaac atataataca actgttttta acaatgatga tgttaaaact 2160
   gaactcgtta cattatgtga tgtatc 2186

<210> 21
<211> 2011
<212> DNA
<213> Artificial sequence

<220>
<223> pHM 2V3 used to make nLSDVSODis-M

<400> 21
atgccgatcg taagccgtgg cggtctggtt ccgggtgcgt tactggcgcg tgaactgggt 60

attcgtcatg tcgataccgt ttgtatttcc agctacgatc acgacaacca gcgcgagctt 120

aaagtgctga aacgcgcaga aggcgatggc gaaggcttca tcgttattga tgacctggtg 180

gataccggtg gtactgcggt tgcgattcgt gaaatgtatc caaaagcgca ctttgtcacc 240

atcttcgcaa aaccggctgg tcgtccgctg gttgatgact atgttgttga tatcccgcaa 300

gatacctgga ttgaacagcc gtgggatatg ggcgtcgtat tcgtcccgcc aatctccggt 360

cgctaatttt tctatcgatg gatcctagtt agtttggttt ttaaataaca aatattgaca 420

atcactgttt taatcatatt aaatgattag gaaatattat gaatttaata aaacagtttc 480

aaaagcggtc tgtgttttga aaggatataa acttcatggt gttattaatt ttgaccagtt 540

acaaaatgga atagtaatta tatcaggcgt tgtacttgga ttaccggaag gtaatcatgg 600

attacatgta cacgagttcg gtgatgaaac taatgggttt ttgagtatgg gaaatcatta 660

taatcctgaa aataaaaaac atggtagccc tttaataatg agaggcatat tggagatctg 720

ggaaatattt attcaaacaa atatggaata tcttatctac atactcgatg gtaaaatctc 780

tcttgttgga gattattcta ttataggaag atcgttagtt attagtgaga aaaatgatga 840

tttaggcaaa ggatataact ttaaaagctt tatagatgga aactctggaa acggtgtcgc 900

gtatggaatt atcggaattg cataatctat ttttttttaa atatataaat tataaaaatg 960

acaaacactt ccctcctttt aagctacttt ttctggatcc ggcgcgcctc tagaaaaaat 1020

tgaaaataaa tacaaaggtt cttgagggtt gtgttaaatt gaaagcgaga aataatcata 1080

aatagtcgac atggtgagca agggcgagga ggataacatg gccatcatca aggagttcat 1140

gcgcttcaag gtgcacatgg agggctccgt gaacggccac gagttcgaga tcgagggcga 1200

gggcgagggc cgcccctacg agggcaccca gaccgccaag ctgaaggtga ccaagggtgg 1260

ccccctgccc ttcgcctggg acatcctgtc ccctcagttc atgtacggct ccaaggccta 1320

cgtgaagcac cccgccgaca tccccgacta cttgaagctg tccttccccg agggcttcaa 1380

gtgggagcgc gtgatgaact tcgaggacgg cggcgtggtg accgtgaccc aggactcctc 1440

cctgcaggac ggcgagttca tctacaaggt gaagctgcgc ggcaccaact tcccctccga 1500

cggccccgta atgcagaaga agaccatggg ctgggaggcc tcctccgagc ggatgtaccc 1560

cgaggacggc gccctgaagg gcgagatcaa gcagaggctg aagctgaagg acggcggcca 1620

ctacgacgct gaggtcaaga ccacctacaa ggccaagaag cccgtgcagc tgcccggcgc 1680

ctacaacgtc aacatcaagt tggacatcac ctcccacaac gaggactaca ccatcgtgga 1740

acagtacgaa cgcgccgagg gccgccactc caccggcggc atggacgagc tgtacaagta 1800

gtttttctcc gcggcccggg gtatactgat caattggatc ggatggaatt cgttgcatat 1860

ggaattatcg gaattgcata attattttct ttttaaaaat atataaatta taaaaatgac 1920

aaatacttcc ctccttttaa gctacttttt cttatttttg tacggaaacg tgtttgtcaa 1980

atctgactat aactatttaa tgtataagat a 2011

<210> 22
<211> 743
<212> DNA
<213> Artificial sequence

<220>
<223> pHM1dSOD used to make nLSDVdSOD-UCT

<400> 22
acattgaaca aaccgataat gaaaaacaac tattaagtga ttttagtgat attctaaaca 60

atgatgaaaa aaatcacgat aaagataaaa aaaaggttgt agcgtaaatt aagcaaaata 120

ttgtatactt tatgtatacg ttttaatatt gaattaatat ataagtttat aattatggaa 180

gaacttgaat gtatgtcgat aaaaataact tccgaaaatg ggagcaccat ttccatatac 240

tgtgaatcca aaactaaaat atcattagat ataacaacaa aaaaaaataa cataaaacct 300

attacaacaa aaaaatctat taaaaaaata gaaaaaaatg atgatgatga tttatgcgca 360

gaccaagaaa atgcaatgga aatagatggg gagcatgcaa ctgataataa caaactataa 420

attcgttgca tatggaatta tcggaattgc ataatctatt tttttttaaa tatataaatt 480

ataaaaatga caaacacttc cctcctttta agctactttt tcttattttt gtacggaaac 540

ttgtttgtca aatctgacta taactattta atgtataaga taaatgtttt taaaaacaat 600

gaaagtacta tcaagtgtta tcataataat gatattattt ttttatcaga tgattgtgta 660

agctttaact ctacaacata taatacaact gtttttaaca atgatgatgt taaaactgaa 720

cttgttacat tgtgtgatgt atc 743

<210> 23
<211> 1321
<212> DNA
<213> Artificial sequence

<220>
<223> pHM1-SODis used to make nLSDVSODis-UCT

<400> 23
acattgaaca aaccgataat gaaaaacaac tattaagtga ttttagtgat attctaaaca 60

atgatgaaaa aaatcacgat aaagataaaa aaaaggttgt agcgtaaatt aagcaaaata 120

ttgtatactt tatgtatacg ttttaatatt gaattaatat ataagtttat aattatggaa 180

gaacttgaat gtatgtcgat aaaaataact tccgaaaatg ggagcaccat ttccatatac 240

tgtgaatcca aaactaaaat atcattagat ataacaacaa aaaaaaataa cataaaacct 300

attacaacaa aaaaatctat taaaaaaata gaaaaaaatg atgatgatga tttatgcgca 360

gaccaagaaa atgcaatgga aatagatggg gagcatgcaa ctgataataa caaactataa 420

attcgttgca tatgccggtc gctaattttt ctatcgatgg atcctagtta gtttggtttt 480

taaataacaa atattgacaa tcactgtttt aatcatatta aatgattagg aaatattatg 540

aatttaataa aacagtttca aaagcggtct gtgttttgaa aggatataaa cttcatggtg 600

ttattaattt tgaccagtta caaaatggaa tagtaattat atcaggcgtt gtacttggat 660

taccggaagg taatcatgga ttacatgtac acgagttcgg tgatgaaact aatgggtttt 720

tgagtatggg aaatcattat aatcctgaaa ataaaaaaca tggtagccct ttaataatga 780

gaggcatatt ggagatctgg gaaatattta ttcaaacaaa tatggaatat cttatctaca 840

tactcgatgg taaaatctct cttgttggag attattctat tataggaaga tcgttagtta 900

ttagtgagaa aaatgatgat ttaggcaaag gatataactt taaaagcttt atagatggaa 960

actctggaaa cggtgtcgcg tatggaatta tcggaattgc ataatccata tggaattatc 1020

ggaattgcat aatctatttt tttttaaata tataaattat aaaaatgaca aacacttccc 1080

tccttttaag ctactttttc ttatttttgt acggaaactt gtttgtcaaa tctgactata     1140

actatttaat gtataagata aatgttttta aaaacaatga aagtactatc aagtgttatc     1200

ataataatga tattattttt ttatcagatg attgtgtaag ctttaactct acaacatata     1260

atacaactgt ttttaacaat gatgatgtta aaactgaact tgttacattg tgtgatgtat     1320

c                                                                     1321


<210>  24
<211>  20
<212>  DNA
<213>  Artificial sequence

<220>
<223>  LSDV 130 forward primer for confirmation of recombinant virus
       nLSDVdSOD-UCT

<400>  24
ggagcaccat ttccatatac                                                   20


<210>  25
<211>  21
<212>  DNA
<213>  Artificial sequence

<220>
<223>  LSDV 132 reverse primer for confirmation of recombinant virus
       nLSDVdSOD-UCT

<400>  25
agtagcttaa aaggagggaa g                                                 21


<210>  26
<211>  20
<212>  DNA
<213>  Artificial sequence

<220>
<223>  LSDV 129 forward primer for confirmation of recombinant virus
       nLSDVSODis-UCT

<400>  26
gagccctgta attcactttt                                                   20


<210>  27
<211>  18
<212>  DNA
<213>  Artificial sequence

<220>
<223>  LSDV 132 reverse primer for confirmation of recombinant virus
       nLSDVSODis-UCT

<400>  27
atcgatggaa aagatccg                                                     18